# EUROPEAN PATENT APPLICATION

(11) **EP 3 112 465 A2**
(43) Date of publication of application: **04.01.2017**
(21) Application number: 16156909.0
(22) Date of filing: 20.07.2012
(51) Int. Cl.: C12N 15/113, C12N 15/82, C12N 5/04, A01H 5/00

(54) **METHOD FOR IDENTIFICATION AND ISOLATION OF TERMINATOR SEQUENCES CAUSING ENHANCED TRANSCRIPTION**

(30) Priority: 01.08.2011 US 201161513682 P
(62) Divisional of application: 12819746.4
(71) Applicant: BASF Plant Science Company GmbH, 67056 Ludwigshafen (DE)
(72) Inventor: Hartig, Julia Verena, 67547 Worms (DE); Burgmeier, Alrun, Chapel Hill, NC 27517 (US); Kuhn, Josef Martin, 67117 Limburgerhof (DE); Loyall, Linda Patricia, 67117 Limburgerhof (DE); Duwenig, Elke, 67117 Limburgerhof (DE)
(74) Representative: BASF IP Association

(57) **Abstract**

FIELD OF THE INVENTION

The invention relates to efficient, high-throughput methods, systems, and DNA constructs for identification and isolation of terminator sequences causing enhanced transcription. The invention further relates to terminator sequences isolated with such methods and their use for enhancing gene expression.

## Description

### FIELD OF THE INVENTION

The invention relates to efficient, high-throughput methods, systems, and DNA constructs for identification and isolation of terminator sequences causing enhanced transcription. The invention further relates to terminator sequences isolated with such methods and their use for enhancing gene expression.

### BACKGROUND OF THE INVENTION

The aim of plant biotechnology is the generation of plants with advantageous novel properties, such as pest and disease resistance, resistance to environmental stress (e.g., water-logging, drought, heat, cold, light-intensity, day-length, chemicals, *etc.*)*,* improved qualities (*e.g.,* high yield of fruit, extended shelf-life, uniform fruit shape and color, higher sugar content, higher vitamins C and A content, lower acidity, *etc.*)*,* or for the production of certain chemicals or pharmaceuticals (Dunwell, 2000). Furthermore resistance against abiotic stress (drought, salt) and/or biotic stress (insects, fungi, nematode infections) can be increased. Crop yield enhancement and yield stability can be achieved by developing genetically engineered plants with desired phenotypes.

For all fields of biotechnology, beside promoter sequences, terminator sequences positioned in the 3'UTR of genes are a basic prerequisite for the recombinant expression of specific genes. In animal systems, a machinery of transcription termination has been well defined (Zhao *et al*., 1999; Proudfoot, 1986; Kim *et al.,* 2003; Yonaha and Proudfoot, 2000; Cramer *et al.,* 2001; Kuerstem and Goodwin, 2003).
However, terminators may have more effects than simple termination of transcription. For example, by Narsai et al. it has been reported that terminators house mRNA stabilizing or destabilizing elements (Narsai et al. 2007). Among these may be AU-rich elements or miRNA binding sites. Furthermore, efficient termination may serve to free the transcribing polymerase to recycle back to the transcriptional start site. This may cause higher levels of transcriptional initiation (Nagaya et al. 2010.;Mapendano et al. 2010). 3' end processing of an mRNA can also be involved in nuclear export and subsequent translation. Therefore, terminator sequences can influence the expression level of a transgene positively or negatively.
Mutagenesis experiments of plant terminators have identified three major elements: far up-stream elements (FUEs), near upstream elements (NUEs; AAUAAA-like motifs) and a cleavage/polyadenylation site (CS). The NUE region is an A-rich element located within 30 nt of the poly(A) site (Hunt, 1994). The FUE region is a U- or UG-richsequence that enhances processing efficiency at the CS (Mogen et al. 1990 , Rothnie, 1996), which is itself a YA (CA or UA) dinucleotide within a U-rich region at which polyadenylation occurs (Bassett, 2007).

In plant biotech transgenes need often be expressed at high levels to cause a desired effect. For most applications the strength of the promoter is considered to primarily decide over the expression levels of a transgene. Promoter identification and characterization is costly and time-consuming as every promoter has its own specific expression pattern and strength. Furthermore, the set of strong suitable promoters is limited. With modern plant biotechnology proceeding towards stacking multiple expression cassettes in one construct, doubling of identical strong promoter elements for optimal gene expression bears the risk of homologous recombination or transcriptional gene silencing. However, using a weaker promoter as an alternative to circumvent this problem may limit the expression of one gene in the construct. This in turn may impair generating the desired phenotype in the transgenic plant.

Instead of strong promoters, terminator sequences may be used to achieve enhanced expression levels of the gene they are functionally linked to. This allows use of alternative weaker promoters in a multigene-construct without compromising overall expression levels. A further advantage may be that promoter specificity and expression patterns are not changed if only the terminator is modified.

It is therefore an objective of the present invention, to provide a method to identify, isolate and test terminator sequences that enhance gene expression in plants. It is also an objective of the invention to provide such terminators that enhance gene expression. A further objective of the invention is to provide terminators efficiently terminating transcription. These objectives are achieved by this invention.

### Detailed description of the Invention

A first embodiment of the invention are terminator molecules comprising at least one of the Enhancing Terminator elements (ET) as defined in table 1 or combinations thereof as defined in table 2, or the complement or reverse complement of any of these ETs or combinations of these ETs, wherein the terminator molecules comprising said ETs or combinations thereof cause enhanced expression of heterologous nucleic acid molecules to be expressed to which the terminator molecules are functionally linked.
Enhancing terminator elements are sequence motives defined by a highly conserved core sequence of approximately 4 to 6 nucleotides surrounded by a conserved matrix sequence of in total up to 26 nucleotides within the plus or minus strand of the ET, which is able of interacting with DNA binding proteins or DNA binding nucleic acid molecules. The conserved matrix sequence allows some variability in the sequence without loosing its ability to be bound by the DNA binding proteins or nucleic acids.
One way to describe DNA binding protein or nucleic acid binding sites is by nucleotide or position weight matrices (NWM or PWM) (for review see Stormo, 2000). A weight matrix pattern definition is superior to a simple IUPAC consensus sequence as it represents the complete nucleotide distribution for each single position. It also allows the quantification of the similarity between the weight matrix and a potential DNA binding protein or nucleic acid binding site detected in the sequence (Cartharius et al. 2005).

The "core sequence" of a matrix is defined as the 4, 5 or 6 consecutive highest conserved positions of the matrix.
The core similarity is calculated as described here and in the papers related to Matlnspector (Cartharius K, et al. (2005) Bioinformatics 21; Cartharius K (2005), DNA Press; Quandt K, et al (1995) Nucleic Acids Res. 23).

The maximum core similarity of 1.0 is only reached when the highest conserved bases of a matrix match exactly in the sequence. More important than the core similarity is the matrix similarity which takes into account all bases over the whole matrix length. The matrix similarity is calculated as described here and in the Matlnspector paper (Quandt K, et al (1995) Nucleic Acids Res. 23). A perfect match to the matrix gets a score of 1.00 (each sequence position corresponds to the highest conserved nucleotide at that position in the matrix), a "good" match to the matrix has a similarity of >0.80.

Mismatches in highly conserved positions of the matrix decrease the matrix similarity more than mismatches in less conserved regions.

In one embodiment the ETs have a sequence as defined in table 1. In another embodiment, the sequence of the ETs is defined as described in table 10, wherein core and matrix similarity of a matching sequence are calculated as described in Quandt et al (1995, NAR 23 (23) 4878 - 4884) in equation 2 and 3 on page 4879 right column, wherein the matrix similarity is at least 0.8, preferably the matrix similarity is at least 0.85, more preferably the matrix similarity is at least 0.9, even more preferably the matrix similarity is at least 0.95. In a most preferred embodiment the matrix similarity is at least 1.0. In one embodiment the core similarity is at least 0.75, preferably the core similarity is at least 0.8 for example 0.85, more preferably the core similarity is at least 0.9, even more preferably the core similarity is at least 0.95. In a most preferred embodiment the core similarity is at least 1.0,. Reference to a SEQ ID of any of the ETs of the invention are to be understood as the sequences as defined in table 1 or as described in table 10.

Preferably the terminator molecules are comprising at least one of the Enhancing Terminator elements (ET) defined by SEQ ID NO5 or SEQ ID NO6, or the combination of SEQ ID NO5 and SEQ ID NO6.
Preferably all ET elements of one group (i.e. one line in table 2) are present in a terminator defined as expression enhancing. Each combination of at least two, preferably at least three, most preferably all ET elements in one group (i.e. one line in table 2) are present in a gene expression enhancing terminator sequence.
In a preferred embodiment, these terminator molecules are functionally linked to another nucleic acid molecule heterologous to the terminator molecule of the invention. Such nucleic acid molecule may for example be any regulatory nucleic acid molecule such as a promoter, a NEENA or an intron. The nucleic acid molecule may also be any nucleic acid to be expressed such as a gene of interest, a coding region or a noncoding region, for example a 5' or 3' UTR, a microRNA, RNAi, antisense RNA and the like.

Another embodiment of the invention are terminator molecules comprising a sequence selected from the group consisting of
a) the nucleic acid molecules having a sequence as defined in SEQ ID NO: 1, 2, 3, 4, 57, 58, 59, 60, 61, 62, 63 and 64, or
b) a nucleic acid molecule having a sequence with an identity of at least 60% or more to any of the sequences as defined by SEQ ID NO: 1, 2, 3, 4, 57, 58, 59, 60, 61, 62, 63 and 64, preferably 70% or more to any of the sequences as defined by SEQ ID NO: 1, 2, 3, 4, 57, 58, 59, 60, 61, 62, 63 and 64, for example 80% or more to any of the sequences as defined by SEQ ID NO: 1, 2, 3, 4, 57, 58, 59, 60, 61, 62, 63 and 64, more preferably, the identity is 85% or more, more preferably the identity is 90% or more, even more preferably, the identity is 95% or more, 96% or more, 97% or more, 98% or more, in the most preferred embodiment, the identity is 99% or more to any of the sequences as defined by SEQ ID NO: 1, 2, 3, 4, 57, 58, 59, 60, 61, 62, 63 and 64., or
c) a fragment of 100 or more consecutive bases, preferably 150 or more consecutive bases, more, preferably 200 consecutive bases or more even more preferably 250 or more consecutive bases of a nucleic acid molecule of a) or b) which has an expressing enhancing activity, for example 65% or more, preferably 70% or more, more preferably 75% or more, even more preferably 80% or more, 85% or more or 90% or more, in a most preferred embodiment it has 95% or more of the expression enhancing activity as the corresponding nucleic acid molecule having the sequence of any of the sequences as defined by SEQ ID NO: 1, 2, 3, 4, 57, 58, 59, 60, 61, 62, 63 and 64, or
d) a nucleic acid molecule which is the complement or reverse complement of any of the previously mentioned nucleic acid molecules under a) or b), or
e) a nucleic acid molecule which is obtainable by PCR with genomic DNA, preferably plant genomic DNA, more preferably monocotyledonous plant genomic DNA using oligonucleotide primers described by SEQ ID NO: 67 to 96 as shown in Table 5 or
f) a nucleic acid molecule of 100 nucleotides or more, 150 nucleotides or more, 200 nucleotides or more or 250 nucleotides or more, hybridizing under conditions equivalent to hybridization in 7% sodium dodecyl sulfate (SDS), 0.5 M NaPO4, 1 mM EDTA at 50°C with washing in 2 X SSC, 0.1 % SDS at 50°C or 65°C, preferably 65°C to a nucleic acid molecule comprising at least 50, preferably at least 100, more preferably at least 150, even more preferably at least 200, most preferably at least 250 consecutive nucleotides of a transcription enhancing terminator described by SEQ ID NO: 1, 2, 3, 4, 57, 58, 59, 60, 61, 62, 63 and 64 or the complement thereof. Preferably, said nucleic acid molecule is hybridizing under conditions equivalent to hybridization in 7% sodium dodecyl sulfate (SDS), 0.5 M NaPO₄, 1 mM EDTA at 50°C with washing in 1 X SSC, 0.1 % SDS at 50°C or 65°C, preferably 65°C to a nucleic acid molecule comprising at least 50, preferably at least 100, more preferably at least 150, even more preferably at least 200, most preferably at least 250 consecutive nucleotides of a transcription enhancing terminator described by SEQ ID NO: 1, 2, 3, 4, 57, 58, 59, 60, 61, 62, 63 and 64 or the complement thereof, more preferably, said nucleic acid molecule is hybridizing under conditions equivalent to hybridization in 7% sodium dodecyl sulfate (SDS), 0.5 M NaPO₄, 1 mM EDTA at 50°C with washing in 0.1 X SSC, 0.1% SDS at 50°C or 65°C, preferably 65°C to a nucleic acid molecule comprising at least 50, preferably at least 100, more preferably at least 150, even more preferably at least 200, most preferably at least 250 consecutive nucleotides of a transcription enhancing terminator described by any of the sequences as defined by SEQ ID NO: 1, 2, 3, 4, 57, 58, 59, 60, 61, 62, 63 and 64,
wherein the terminator molecules as defined under a) to f) cause enhanced expression of heterologous nucleic acid molecules to which they are functionally linked.
Preferably the nucleic acid molecules as defined above under b) to f) are functional enhancing terminator molecules, hence are terminating transcription and are having at least 50% of the expression enhancing effect as the respective molecule as defined above under a). More preferably the nucleic acid molecules as defined above under b) to f) are comprising the Enhancing Terminator elements (ET) comprised in the nucleic acid molecules as defined above under a) and as defined in table 1 or as defined intable 10 or combinations thereof as defined in table 2 and are functional enhancing terminator molecules, hence are terminating transcription and are having at least 50% of the expression enhancing effect as the molecules as defined above under a). For example, the nucleic acid molecules as defined above under b) to f) comprise functional polyadenylation signals and an AT content deviating not more than 50%, preferably 40%, more preferably 30%, even more preferably 20%, especially preferably 10% from the AT content of the molecules as defined above under a). Most preferably they have an identical AT content as the molecules as defined above under a).

A further embodiment of the invention is a method for the identification and isolation of terminator molecules causing enhanced expression of heterologous nucleic acid molecules to which the terminator molecules are functionally linked comprising the steps of identification of nucleic acid molecules comprising a sequence comprising in not more than 500 bp, preferably 400 bp, more preferable 300 bp most preferable 250 bp at least one, preferably at least 2, more preferably at least 4 ET as defined in table 1 or as defined in table 10. Most preferably they comprise all ET elements listed in one line of table 2. In another step of the invention at hand, the respective nucleic acid molecules are isolated from their natural background such as genomic DNA with methods known to the person skilled in the art or synthesized.
A further embodiment of the invention is a method for the identification and isolation of terminator molecules causing enhanced expression of heterologous nucleic acid molecules to which the terminator molecules are functionally linked comprising the steps of
A) identification of 3'UTR and/or a transcribed region such as a coding region and
B) identification of corresponding genomic DNA
C) identification of molecules in the group identified in B) that contain any of the ETs or combinations thereof as defined in table 1 or table 10 and 2 using any computational ET detection or IUPAC string matching sequence analysis and
D) isolation of at least 250 bp of genomic DNA comprising said ETs.

The 3'UTRs and the corresponding genomic DNA may be identified by any method known to a person skilled in the art, for example sequence determination of full-length cDNAs, computational predictions for example based on the prediction of coding sequences in genomic DNA sequences or the use of annotated databases for e.g. cDNAs or genomic DNA.
The term " corresponding genomic DNA" is to be understood as genomic DNA, preferably plant genomic DNA, more preferably monocotyledonous plant genomic DNA comprising the sequence identified as 3'UTR or transcribed region in step A). The corresponding genomic DNA may comprise an identical sequence or a sequence having an identity of at least 60% or, preferably 70% or more, for example 80% or more, more preferably, the identity is 85% or more, more preferably the identity is 90% or more, even more preferably, the identity is 95% or more, 96% or more, 97% or more, 98% or more, in the most preferred embodiment, the identity is 99% or more to any of the sequences of the 3'UTR or transcribed region identified in step A).
Identification of molecules in the group identified in B) that contain any of the ETs or combinations thereof may for example be done with tools known to the skilled person, such as Matln-spector of Genomatix Software GmbH, The MEME suite of the University of Queensland and Univeristy of Washington, or comparable tools. The isolation of the at least 250 bp, for example 300 bp, preferably at least 350 bp, for example 400 bp, more preferably at least 450 bp, for example 500 bp may be done with recombinant methods known in the art such as PCR, restriction cloning or gene synthesis. The isolated expression enhancing terminator molecules may in subsequent steps be functionally linked to promoters and / or nucleic acid molecules to be expressed. A skilled person is aware of various methods for functionally linking two or more nucleic acid molecules. Such methods may encompass restriction/ligation, ligase independent cloning, recombineering, recombination or synthesis. Other methods may be employed to functionally link two or more nucleic acid molecules.

A further embodiment of the invention is a method for producing a plant or part thereof with, compared to a respective control plant or part thereof, enhanced expression of one or more nucleic acid molecule comprising the steps of
a) introducing the one or more expression enhancing terminator molecules as defined above into a plant or part thereof and
b) integrating said one or more expression enhancing terminator molecule into the genome of said plant cell, plant or part thereof whereby said one or more expression enhancing terminator molecule is functionally linked to an endogenous nucleic acid heterologous to said one or more expression enhancing terminator molecule and optionally
c) regenerating a plant or part thereof comprising said one or more expression enhancing terminator from said transformed plant cell, plant or part thereof.
The one or more expression enhancing terminator molecule may be introduced into the plant or part thereof by means of particle bombardment, protoplast electroporation, virus infection, *Agrobacterium* mediated transformation or any other approach known in the art. The expression enhancing terminator molecule may be introduced integrated for example into a plasmid or viral DNA or viral RNA. The expression enhancing terminator moleculemay also be comprised on a BAC, YAC or artificial chromosome prior to introduction into the plant or part of the plant. It may be also introduced as a linear nucleic acid molecule comprising the expression enhancing terminator sequence wherein additional sequences may be present adjacent to the expression enhancing terminator sequence on the nucleic acid molecule. These sequences neighboring the expression enhancing terminator sequence may be from about 20 bp, for example 20 bp to several hundred base pairs, for example 100 bp or more and may facilitate integration into the genome for example by homologous recombination. Any other method for genome integration may be employed, for example targeted integration approaches, such as homologous recombination or random integration approaches, such as illegitimate recombination.
The endogenous nucleic acid to which the expression enhancing terminator molecule may be functionally linked may be any nucleic acid molecule. The nucleic acid molecule may be a protein coding nucleic acid molecule or a non-coding molecule such as antisense RNA, rRNA, tRNA, miRNA, ta-siRNA, siRNA, dsRNA, snRNA, snoRNA or any other non-coding RNA known in the art.

Another embodiment of the invention is a method for producing a plant or part thereof with, compared to a respective control plant or part thereof, enhanced expression of one or more nucleic acid molecule comprising the steps of
1. functionally linking one or more expression enhancing terminator molecule to a promoter and/or to a nucleic acid molecule being under the control of said promoter and
2. introducing into the genome of plant or part thereof said one or more expression enhancing terminator molecules comprising an ET or combinations thereof as defined in table 1 or 10 or 2 or a terminator molecule as defined above under a) to f) functionally linked to said heterologous promoter and/or said heterologous nucleic acid to be expressed and
3. regenerating a plant or part thereof comprising said one or more expression enhancing terminator molecule from said transformed plant or part thereof.

The expression enhancing terminator molecule may be heterologous to the nucleic acid molecule which is under the control of said promoter to which the expression enhancing terminator is functionally linked or it may be heterologous to both the promoter and the nucleic acid molecule under the control of said promoter.

The one or more expression enhancing terminator molecule may be introduced into the plant or part thereof by means of particle bombardment, protoplast electroporation, virus infection, *Agrobacterium* mediated transformation or any other approach known in the art. The expression enhancing terminator molecule may be introduced integrated for example into a plasmid or viral DNA or viral RNA. The expression enhancing terminator moleculemay also be comprised on a BAC, YAC or artificial chromosome prior to introduction into the plant or part of the plant. It may be also introduced as a linear nucleic acid molecule comprising the expression enhancing terminator sequence wherein additional sequences may be present adjacent to the expression enhancing terminator sequence on the nucleic acid molecule. These sequences neighboring the expression enhancing terminator sequence may be from about 20 bp, for example 20 bp to several hundred base pairs, for example 100 bp or more and may facilitate integration into the genome for example by homologous recombination. Any other method for genome integration may be employed, for example targeted integration approaches, such as homologous recombination or random integration approaches, such as illegitimate recombination.

In one embodiment of the methods of the invention as defined above, the method comprises the setps of
i) providing an expression construct comprising one or more expression enhancing terminator molecule comprising an ET or combinations thereof as defined in table 1 or 10 or 2 or a terminator molecule as defined above under a) to f) functionally linked to a promoter and to one or more nucleic acid molecule the latter being heterologous to said one or more expression enhancing terminator molecule and which is under the control of said promoter and
ii) integrating said expression construct comprising said one or more expression enhancing terminator molecule into the genome of said plant or part thereof and optionally
iii) regenerating a plant or part thereof comprising said one or more expression construct from said transformed plant or part thereof.

The methods of the invention may be applied to any plant, for example gymnosperm or angiosperm, preferably angiosperm, for example dicotyledonous or monocotyledonous plants. Preferred monocotyledonous plants are for example corn, wheat, rice, barley, sorghum, musa, sugarcane, miscanthus and brachypodium, especially preferred monocotyledonous plants are corn, wheat and rice. Preferred dicotyledonous plants are for example soy, rape seed, canola, linseed, cotton, potato, sugar beet, tagetes and Arabidopsis, especially preferred dicotyledonous plants are soy, rape seed, canola and potato.
A plant exhibiting enhanced expression of a nucleic acid molecule as meant herein means a plant having a higher, preferably statistically significant higher expression of a nucleic acid molecule compared to a control plant grown under the same conditions without the respective expression enhancing terminator functionally linked to the respective nucleic acid molecule. Such control plant may be a wild-type plant or a transgenic plant comprising the same promoter controlling the same gene as in the plant of the invention wherein the promoter and/ or the nucleic acid to be expressed is not linked to an expression enhancing terminator of the invention.

A recombinant expression construct comprising one or more expression enhancing terminator molecule comprising an ET or combination thereof as defined in table 1 or 10 or 2 or an expression enhancing terminator molecule as defined above under a) to f) is a further embodiment of the invention. The recombinant expression construct may further comprise one or more promoter and optionally one or more expressed nucleic acid molecule to both of them the one or more expression enhancing terminator molecule is functionally linked, at least the latter being heterologous to said one or more expression enhancing terminator molecule.
The expression enhancing terminator molecule may be heterologous to the nucleic acid molecule which is under the control of said promoter to which the expression enhancing terminator molecule is functionally linked or it may be heterologous to both the promoter and the nucleic acid molecule under the control of said promoter.

The expression construct may comprise one ore more, for example two or more, for example 5 or more, such as 10 or more combinations of promoters functionally linked to a expression enhancing terminator molecule and a nucleic acid molecule to be expressed which is heterologous to the respective expression enhancing terminator molecule. The expression construct may also comprise further expression constructs not comprising an expression enhancing terminator molecule.

A recombinant expression vector comprising one or more recombinant expression construct as defined above is another embodiment of the invention. A multitude of expression vectors that may be used in the present invention are known to a skilled person. Methods for introducing such a vector comprising such an expression construct comprising for example a promoter functionally linked to a expression enhancing terminator and optionally other elements such as a promoters, UTRs, NEENAs and the like into the genome of a plant and for recovering transgenic plants from a transformed cell are also well known in the art. Depending on the method used for the transformation of a plant or part thereof the entire vector might be integrated into the genome of said plant or part thereof or certain components of the vector might be integrated into the genome, such as, for example a T-DNA.

A transgenic cell or transgenic plant or part thereof comprising a recombinant expression vector as defined above or a recombinant expression construct as defined above is a further embodiment of the invention. The transgenic cell, transgenic plant or part thereof may be selected from the group consisting of bacteria, fungi, yeasts, or plant, insect or mammalian cells or plants. Preferred transgenic cells are bacteria, fungi, yeasts and plant cells. Preferred bacteria are Enterobacteria such as *E. coli* and bacteria of the genus Agrobacteria, for example *Agrobacterium tumefaciens* and *Agrobacterium rhizogenes.* Preferred plants are monocotyledonous or dicotyledonous plants for example monocotyledonous or dicotyledonous crop plants such as corn, soy, canola, cotton, potato, sugar beet, rice, wheat, sorghum, barley, musa, sugarcane, miscanthus and the like. Preferred crop plants are corn, rice, wheat, soy, canola, cotton or potato. Especially preferred dicotyledonous crop plants are soy, canola, cotton or potato.
Especially preferred monocotyledonous crop plants are corn, wheat and rice.

A transgenic cell culture, transgenic seed, parts or propagation material derived from a transgenic cell or plant or part thereof as defined above comprising said heterologous expression enhancing terminator molecule comprising an ET or combination thereof as defined in table 1 or 10 or 2 or an expression enhancing terminator molecule as defined above under a) to f) or said recombinant expression construct or said recombinant vector as defined above are other embodiments of the invention.
Transgenic parts or propagation material as meant herein comprise all tissues and organs, for example leaf, stem and fruit as well as material that is useful for propagation and/or regeneration of plants such as cuttings, scions, layers, branches or shoots comprising the respective expression enhancing terminator molecule, recombinant expression construct or recombinant vector.

A further embodiment of the invention is the use of the expression enhancing terminator molecule comprising an ET or combination thereof as defined in table 1 or 10 or 2 or an expression enhancing terminator molecule as defined above under a) to f) or the recombinant construct or recombinant vector as defined above for enhancing expression in plants or parts thereof.

A further embodiment of the invention is a method for the production of an agricultural product by introducing an expression enhancing terminator molecule comprising an ET or combination thereof as defined in table 1 or 10 or 2 or an expression enhancing terminator molecule as defined above under a) to f) or the recombinant construct or recombinant vector as defined above into a plant, growing the plant, harvesting and processing the plant or parts thereof.

A further embodiment of the invention is a method for producing a recombinant terminator molecule which is enhancing the expression of a heterologous nucleic acid molecules to which the terminator molecule is functionally linked, the method comprising the steps of introducing into a terminator molecule lacking the expression enhancing property, at least one ET element as defined in table 1 or table 10 or combinations thereof as defined in table 2, or the complement or reverse complement thereof.
In one embodiment the ETs have a sequence as defined in table 1. In another embodiment, the sequence of the ETs is defined as described in table 10, wherein core and matrix similarity of a matching sequence are calculated as described in Quandt et al (1995, NAR 23 (23) 4878 - 4884) in equation 2 and 3 on page 4879 right column, wherein the matrix similarity is at least 0.8, preferably the matrix similarity is at least 0.85, more preferably the matrix similarity is at least 0.9, even more preferably the matrix similarity is at least 0.95. In a most preferred embodiment the matrix similarity is at least 1.0. In one embodiment the core similarity is at least 0.75, preferably the core similarity is at least 0.8 for example 0.85, more preferably the core similarity is at least 0.9, even more preferably the core similarity is at least 0.95. In a most preferred embodiment the core similarity is at least 1.0,.

In the method of the invention, preferably at least one of the Enhancing Terminator elements (ET) defined by SEQ ID NO5 or SEO ID NO6, or the combination of SEQ ID NO5 and SEQ ID NO6 is introduced into the recombinant terminator molecules of the invention.
In a preferred embodiment of the methd of the invention all ET elements of one group (i.e. one line in table 2) are introduced into a recombinant terminator of the invention. Each combination of at least two, preferably at least three, most preferably all ET elements in one group (i.e. one line in table 2) are present in a gene expression enhancing terminator sequence that is produced according to the method of the invention.
The skilled person is aware of methods to produce such recombinant terminator molecules. A terminator molecule lacking the ability to enhance expression of nucleic acid molecules to which the terminator molecule is functionally linked might be used and the ET elements of the invention may be introduced by way of cloning methods, recombination or synthesis of the terminator molecule.

### DEFINITIONS

Abbreviations: NEENA - nucleic acid expression enhancing nucleic acid, GFP - green fluorescence protein, GUS - beta-Glucuronidase, BAP - 6-benzylaminopurine; 2,4-D - 2,4-dichlorophenoxyacetic acid; MS - Murashige and Skoog medium; NAA - 1-naphtaleneacetic acid; MES, 2-(N-morpholino-ethanesulfonic acid, IAA indole acetic acid; Kan: Kanamycin sulfate; GA3 - Gibberellic acid; Timentin™: ticarcillin disodium / clavulanate potassium, microl: Microliter.

It is to be understood that this invention is not limited to the particular methodology or protocols. It is also to be understood that the terminology used herein is for the purpose of describing particular embodiments only, and is not intended to limit the scope of the present invention which will be limited only by the appended claims. It must be noted that as used herein and in the appended claims, the singular forms "a," "and," and "the" include plural reference unless the context clearly dictates otherwise. Thus, for example, reference to "a vector" is a reference to one or more vectors and includes equivalents thereof known to those skilled in the art, and so forth. The term "about" is used herein to mean approximately, roughly, around, or in the region of. When the term "about" is used in conjunction with a numerical range, it modifies that range by extending the boundaries above and below the numerical values set forth. In general, the term "about" is used herein to modify a numerical value above and below the stated value by a variance of 20 percent, preferably 10 percent up or down (higher or lower). As used herein, the word "or" means any one member of a particular list and also includes any combination of members of that list. The words "comprise," "comprising," "include," "including," and "includes" when used in this specification and in the following claims are intended to specify the presence of one or more stated features, integers, components, or steps, but they do not preclude the presence or addition of one or more other features, integers, components, steps, or groups thereof. For clarity, certain terms used in the specification are defined and used as follows:
Agricultural product: The term " Agricultural product" as used in this application means any harvestable product from a plant. The plant products may be, but are not limited to, foodstuff, feedstuff, food supplement, feed supplement, fiber, cosmetic or pharmaceutical product. Foodstuffs are regarded as compositions used for nutrition or for supplementing nutrition. Animal feedstuffs and animal feed supplements, in particular, are regarded as foodstuffs. Agricultural products may as an example be plant extracts, proteins, amino acids, carbohydrates, fats, oils, polymers such as starch or fibers, vitamins, secondary plant products and the like.

Antiparallel: "Antiparallel" refers herein to two nucleotide sequences paired through hydrogen bonds between complementary base residues with phosphodiester bonds running in the 5'-3' direction in one nucleotide sequence and in the 3'-5' direction in the other nucleotide sequence.

Antisense: The term "antisense" refers to a nucleotide sequence that is inverted relative to its normal orientation for transcription or function and so expresses an RNA transcript that is complementary to a target gene mRNA molecule expressed within the host cell (e.g., it can hybridize to the target gene mRNA molecule or single stranded genomic DNA through Watson-Crick base pairing) or that is complementary to a target DNA molecule such as, for example genomic DNA present in the host cell.

Coding region: As used herein the term "coding region" when used in reference to a structural gene refers to the nucleotide sequences which encode the amino acids found in the nascent polypeptide as a result of translation of a mRNA molecule. The coding region is bounded, in eukaryotes, on the 5'-side by the nucleotide triplet "ATG" which encodes the initiator methionine and on the 3'-side by one of the three triplets which specify stop codons (i.e., TAA, TAG, TGA). In addition to containing introns, genomic forms of a gene may also include sequences located on both the 5'- and 3'-end of the sequences which are present on the RNA transcript. These sequences are referred to as "flanking" sequences or regions (these flanking sequences are located 5' or 3' to the non-translated sequences present on the mRNA transcript). The 5'-flanking region may contain regulatory sequences such as promoters and enhancers which control or influence the transcription of the gene. The 3'-flanking region may contain sequences which direct the termination of transcription, post-transcriptional cleavage and polyadenylation.

Complementary: "Complementary" or "complementarity" refers to two nucleotide sequences which comprise antiparallel nucleotide sequences capable of pairing with one another (by the base-pairing rules) upon formation of hydrogen bonds between the complementary base residues in the antiparallel nucleotide sequences. For example, the sequence 5'-AGT-3' is complementary to the sequence 5'-ACT-3'. Complementarity can be "partial" or "total." "Partial" complementarity is where one or more nucleic acid bases are not matched according to the base pairing rules. "Total" or "complete" complementarity between nucleic acid molecules is where each and every nucleic acid base is matched with another base under the base pairing rules. The degree of complementarity between nucleic acid molecule strands has significant effects on the efficiency and strength of hybridization between nucleic acid molecule strands. A "complement" of a nucleic acid sequence as used herein refers to a nucleotide sequence whose nucleic acid molecules show total complementarity to the nucleic acid molecules of the nucleic acid sequence.

Double-stranded RNA: A "double-stranded RNA" molecule or "dsRNA" molecule comprises a sense RNA fragment of a nucleotide sequence and an antisense RNA fragment of the nucleotide sequence, which both comprise nucleotide sequences complementary to one another, thereby allowing the sense and antisense RNA fragments to pair and form a double-stranded RNA molecule.

Endogenous: An "endogenous" nucleotide sequence refers to a nucleotide sequence, which is present in the genome of the untransformed plant cell.

Enhanced expression: "enhance" or "increase" the expression of a nucleic acid molecule in a plant cell are used equivalently herein and mean that the level of expression of the nucleic acid molecule in a plant, part of a plant or plant cell after applying a method of the present invention is higher than its expression in the plant, part of the plant or plant cell before applying the method, or compared to a reference plant lacking a recombinant nucleic acid molecule of the invention. For example, the reference plant is comprising the same construct which is only lacking the respective enhancing terminator of the invention. The term "enhanced" or "increased" as used herein are synonymous and means herein higher, preferably significantly higher expression of the nucleic acid molecule to be expressed. As used herein, an "enhancement" or" increase" of the level of an agent such as a protein, mRNA or RNA means that the level is increased relative to a substantially identical plant, part of a plant or plant cell grown under substantially identical conditions, lacking a recombinant nucleic acid molecule of the invention, for example lacking the enhancing terminator of the invention molecule, the recombinant construct or recombinant vector of the invetion. As used herein, "enhancement" or "increase" of the level of an agent, such as for example a preRNA, mRNA, rRNA, tRNA, snoRNA, snRNA expressed by the target gene and/or of the protein product encoded by it, means that the level is increased 20% or more, for example 50% or more, preferably 100% or more, more preferably 3 fold or more, even more preferably 15 fold or more, most preferably 10 fold or more for example 20 fold relative to a cell or organism lacking a recombinant nucleic acid molecule of the invention. The enhancement or increase can be determined by methods with which the skilled worker is familiar. Thus, the enhancement or increase of the nucleic acid or protein quantity can be determined for example by an immunological detection of the protein. Moreover, techniques such as protein assay, fluorescence, Northern hybridization, nuclease protection assay, reverse transcription (quantitative RT-PCR), ELISA (enzyme-linked immunosorbent assay), Western blotting, radioimmunoassay (RIA) or other immunoassays and fluorescence-activated cell analysis (FACS) can be employed to measure a specific protein or RNA in a plant or plant cell. Depending on the type of the induced protein product, its activity or the effect on the phenotype of the organism or the cell may also be determined. Methods for determining the protein quantity are known to the skilled worker. Examples, which may be mentioned, are: the micro-Biuret method (Goa J (1953) Scand J Clin Lab Invest 5:218-222), the Folin-Ciocalteau method (Lowry OH et al. (1951) J Biol Chem 193:265-275) or measuring the absorption of CBB G-250 (Bradford MM (1976) Analyt Biochem 72:248-254). As one example for quantifying the activity of a protein, the detection of luciferase activity is described in the Examples below.

Enhancing Terminator element (ET): Short nucleic acid sequences between 5 to 30 bases in length defining gene expressing enhancing terminators that confer enhanced expression to a functionally linked gene. They can be defined as nucleotide weight matrices. Enhancing Terminator elements may show conservation across homologous terminator sequences. Presence of individual Enhancing Terminator elements or combinations thereof as defined in table 1 and 2 are sufficient to classify a terminator sequence as expression enhancing.

Expression: "Expression" refers to the biosynthesis of a gene product, preferably to the transcription and/or translation of a nucleotide sequence, for example an endogenous gene or a heterologous gene, in a cell. For example, in the case of a structural gene, expression involves transcription of the structural gene into mRNA and - optionally - the subsequent translation of mRNA into one or more polypeptides. In other cases, expression may refer only to the transcription of the DNA harboring an RNA molecule.

Expression construct: "Expression construct" as used herein mean a DNA sequence capable of directing expression of a particular nucleotide sequence in an appropriate part of a plant or plant cell, comprising a promoter functional in said part of a plant or plant cell into which it will be introduced, operatively linked to the nucleotide sequence of interest which is - optionally - operatively linked to termination signals. If translation is required, it also typically comprises sequences required for proper translation of the nucleotide sequence. The coding region may code for a protein of interest but may also code for a functional RNA of interest, for example RNAa, siRNA, snoRNA, snRNA, microRNA, ta-siRNA or any other noncoding regulatory RNA, in the sense or antisense direction. The expression construct comprising the nucleotide sequence of interest may be chimeric, meaning that one or more of its components is heterologous with respect to one or more of its other components. The expression construct may also be one, which is naturally occurring but has been obtained in a recombinant form useful for heterologous expression. Typically, however, the expression construct is heterologous with respect to the host, i.e., the particular DNA sequence of the expression construct does not occur naturally in the host cell and must have been introduced into the host cell or an ancestor of the host cell by a transformation event. The expression of the nucleotide sequence in the expression construct may be under the control of a seed-specific and/or seed-preferential promoter or of an inducible promoter, which initiates transcription only when the host cell is exposed to some particular external stimulus. In the case of a plant, the promoter can also be specific to a particular tissue or organ or stage of development.

Foreign: The term "foreign" refers to any nucleic acid molecule (e.g., gene sequence) which is introduced into the genome of a cell by experimental manipulations and may include sequences found in that cell so long as the introduced sequence contains some modification (e.g., a point mutation, the presence of a selectable marker gene, etc.) and is therefore distinct relative to the naturally-occurring sequence.

Functional linkage: The term "functional linkage" or "functionally linked" is to be understood as meaning, for example, the sequential arrangement of a regulatory element (e.g. a promoter) with a nucleic acid sequence to be expressed and, if appropriate, further regulatory elements (such as e.g., a terminator or a NEENA) in such a way that each of the regulatory elements can fulfill its intended function to allow, modify, facilitate or otherwise influence expression of said nucleic acid sequence. As a synonym the wording "operable linkage" or "operably linked" may be used. The expression may result depending on the arrangement of the nucleic acid sequences in relation to sense or antisense RNA. To this end, direct linkage in the chemical sense is not necessarily required. Genetic control sequences such as, for example, enhancer sequences, can also exert their function on the target sequence from positions which are further away, or indeed from other DNA molecules. Preferred arrangements are those in which the nucleic acid sequence to be expressed recombinantly is positioned behind the sequence acting as promoter, so that the two sequences are linked covalently to each other. The distance between the promoter sequence and the nucleic acid sequence to be expressed recombinantly is preferably less than 200 base pairs, especially preferably less than 100 base pairs, very especially preferably less than 50 base pairs. In a preferred embodiment, the nucleic acid sequence to be transcribed is located behind the promoter in such a way that the transcription start is identical with the desired beginning of the chimeric RNA of the invention. Functional linkage, and an expression construct, can be generated by means of customary recombination and cloning techniques as described (e.g., in Maniatis T, Fritsch EF and Sambrook J (1989) Molecular Cloning: A Laboratory Manual, 2nd Ed., Cold Spring Harbor Laboratory, Cold Spring Harbor (NY); Silhavy et al. (1984) Experiments with Gene Fusions, Cold Spring Harbor Laboratory, Cold Spring Harbor (NY); Ausubel et al. (1987) Current Protocols in Molecular Biology, Greene Publishing Assoc. and Wiley Interscience; Gelvin et al. (Eds) (1990) Plant Molecular Biology Manual; Kluwer Academic Publisher, Dordrecht, The Netherlands). However, further sequences, which, for example, act as a linker with specific cleavage sites for restriction enzymes, or as a signal peptide, may also be positioned between the two sequences. The insertion of sequences may also lead to the expression of fusion proteins. Preferably, the expression construct, consisting of a linkage of a regulatory region for example a promoter and nucleic acid sequence to be expressed, can exist in a vector-integrated form and be inserted into a plant genome, for example by transformation.

Gene: The term "gene" refers to a region operably joined to appropriate regulatory sequences capable of regulating the expression of the gene product (e.g., a polypeptide or a functional RNA) in some manner. A gene includes untranslated regulatory regions of DNA (e.g., promoters, enhancers, repressors, etc.) preceding (up-stream) and following (downstream) the coding region (open reading frame, ORF) as well as, where applicable, intervening sequences (i.e., introns) between individual coding regions (i.e., exons). The term "structural gene" as used herein is intended to mean a DNA sequence that is transcribed into mRNA which is then translated into a sequence of amino acids characteristic of a specific polypeptide.

Genome and genomic DNA: The terms "genome" or "genomic DNA" is referring to the heritable genetic information of a host organism. Said genomic DNA comprises the DNA of the nucleus (also referred to as chromosomal DNA) but also the DNA of the plastids (e.g., chloroplasts) and other cellular organelles (e.g., mitochondria). Preferably the terms genome or genomic DNA is referring to the chromosomal DNA of the nucleus.

Heterologous: The term "heterologous" with respect to a nucleic acid molecule or DNA refers to a nucleic acid molecule which is operably linked to, or is manipulated to become operably linked to, a second nucleic acid molecule to which it is not operably linked in nature, or to which it is operably linked at a different location in nature. A heterologous expression construct comprising a nucleic acid molecule and one or more regulatory nucleic acid molecule (such as a promoter or a transcription termination signal) linked thereto for example is a constructs originating by experimental manipulations in which either a) said nucleic acid molecule, or b) said regulatory nucleic acid molecule or c) both (i.e. (a) and (b)) is not located in its natural (native) genetic environment or has been modified by experimental manipulations, an example of a modification being a substitution, addition, deletion, inversion or insertion of one or more nucleotide residues. Natural genetic environment refers to the natural chromosomal locus in the organism of origin, or to the presence in a genomic library. In the case of a genomic library, the natural genetic environment of the sequence of the nucleic acid molecule is preferably retained, at least in part. The environment flanks the nucleic acid sequence at least at one side and has a sequence of at least 50 bp, preferably at least 500 bp, especially preferably at least 1,000 bp, very especially preferably at least 5,000 bp, in length. A naturally occurring expression construct - for example the naturally occurring combination of a promoter with the corresponding gene - becomes a transgenic expression construct when it is modified by non-natural, synthetic "artificial" methods such as, for example, mutagenization. Such methods have been described (US 5,565,350; WO 00/15815). For example a protein encoding nucleic acid molecule operably linked to a promoter, which is not the native promoter of this molecule, is considered to be heterologous with respect to the promoter. Preferably, heterologous DNA is not endogenous to or not naturally associated with the cell into which it is introduced, but has been obtained from another cell or has been synthesized. Heterologous DNA also includes an endogenous DNA sequence, which contains some modification, non-naturally occurring, multiple copies of an endogenous DNA sequence, or a DNA sequence which is not naturally associated with another DNA sequence physically linked thereto. Generally, although not necessarily, heterologous DNA encodes RNA or proteins that are not normally produced by the cell into which it is expressed.

Hybridization: The term "hybridization" as used herein includes "any process by which a strand of nucleic acid molecule joins with a complementary strand through base pairing." (J. Coombs (1994) Dictionary of Biotechnology, Stockton Press, New York). Hybridization and the strength of hybridization (i.e., the strength of the association between the nucleic acid molecules) is impacted by such factors as the degree of complementarity between the nucleic acid molecules, stringency of the conditions involved, the Tm of the formed hybrid, and the G:C ratio within the nucleic acid molecules. As used herein, the term "Tm" is used in reference to the "melting temperature." The melting temperature is the temperature at which a population of double-stranded nucleic acid molecules becomes half dissociated into single strands. The equation for calculating the Tm of nucleic acid molecules is well known in the art. As indicated by standard references, a simple estimate of the Tm value may be calculated by the equation: Tm=81.5+0.41 (% G+C), when a nucleic acid molecule is in aqueous solution at 1 M NaCl [see e.g., Anderson and Young, Quantitative Filter Hybridization, in Nucleic Acid Hybridization (1985)]. Other references include more sophisticated computations, which take structural as well as sequence characteristics into account for the calculation of Tm. Stringent conditions, are known to those skilled in the art and can be found in Current Protocols in Molecular Biology, John Wiley & Sons, N.Y. (1989), 6.3.1-6.3.6.

"Identity": "Identity" when used in respect to the comparison of two or more nucleic acid or amino acid molecules means that the sequences of said molecules share a certain degree of sequence similarity, the sequences being partially identical.
To determine the percentage identity (homology is herein used interchangeably) of two amino acid sequences or of two nucleic acid molecules, the sequences are written one underneath the other for an optimal comparison (for example gaps may be inserted into the sequence of a protein or of a nucleic acid in order to generate an optimal alignment with the other protein or the other nucleic acid).

The amino acid residues or nucleic acid molecules at the corresponding amino acid positions or nucleotide positions are then compared. If a position in one sequence is occupied by the same amino acid residue or the same nucleic acid molecule as the corresponding position in the other sequence, the molecules are homologous at this position (i.e. amino acid or nucleic acid "homology" as used in the present context corresponds to amino acid or nucleic acid "identity". The percentage homology between the two sequences is a function of the number of identical positions shared by the sequences (i.e. % homology = number of identical positions/total number of positions x 100). The terms "homology" and "identity" are thus to be considered as synonyms.

For the determination of the percentage identity of two or more amino acids or of two or more nucleotide sequences several computer software programs have been developed. The identity of two or more sequences can be calculated with for example the software fasta, which presently has been used in the version fasta 3 (W. R. Pearson and D. J. Lipman, PNAS 85, 2444 (1988); W. R. Pearson, Methods in Enzymology 183, 63 (1990); W. R. Pearson and D. J. Lipman, PNAS 85, 2444 (1988); W. R. Pearson, Enzymology 183, 63 (1990)). Another useful program for the calculation of identities of different sequences is the standard blast program, which is included in the Biomax pedant software (Biomax, Munich, Federal Republic of Germany). This leads unfortunately sometimes to suboptimal results since BLAST does not always include complete sequences of the subject and the query. Nevertheless as this program is very efficient it can be used for the comparison of a huge number of sequences. The following settings are typically used for such a comparisons of sequences:
-p Program Name [String]; -d Database [String]; default = nr; -i Query File [File In]; default = stdin; -e Expectation value (E) [Real]; default = 10.0; -m alignment view options: 0 = pairwise; 1 = query-anchored showing identities; 2 = query-anchored no identities; 3 = flat query-anchored, show identities; 4 = flat query-anchored, no identities; 5 = query-anchored no identities and blunt ends; 6 = flat query-anchored, no identities and blunt ends; 7 = XML Blast output; 8 = tabular; 9 tabular with comment lines [Integer]; default = 0; -o BLAST report Output File [File Out] Optional; default = stdout; -F Filter query sequence (DUST with blastn, SEG with others) [String]; default = T; -G Cost to open a gap (zero invokes default behavior) [Integer]; default = 0; -E Cost to extend a gap (zero invokes default behavior) [Integer]; default = 0; -X X dropoff value for gapped alignment (in bits) (zero invokes default behavior); blastn 30, megablast 20, tblastx 0, all others 15 [Integer]; default = 0; -I Show Gl's in deflines [T/F]; default = F; -q Penalty for a nucleotide mismatch (blastn only) [Integer]; default = -3; -r Reward for a nucleotide match (blastn only) [Integer]; default = 1; -v Number of database sequences to show oneline descriptions for (V) [Integer]; default = 500; -b Number of database sequence to show alignments for (B) [Integer]; default = 250; -f Threshold for extending hits, default if zero; blastp 11, blastn 0, blastx 12, tblastn 13; tblastx 13, megablast 0 [Integer]; default = 0; -g Perfom gapped alignment (not available with tblastx) [T/F]; default = T; -Q Query Genetic code to use [Integer]; default = 1; -D DB Genetic code (for tblast[nx] only) [Integer]; default = 1; -a Number of processors to use [Integer]; default = 1; -O SeqAlign file [File Out] Optional; -J Believe the query defline [T/F]; default = F; -M Matrix [String]; default = BLOSUM62; -W Word size, default if zero (blastn 11, megablast 28, all others 3) [Integer]; default = 0; -z Effective length of the database (use zero for the real size) [Real]; default = 0; -K Number of best hits from a region to keep (off by default, if used a value of 100 is recommended) [Integer]; default = 0; -P 0 for multiple hit, 1 for single hit [Integer]; default = 0; -Y Effective length of the search space (use zero for the real size) [Real]; default = 0; -S Query strands to search against database (for blast[nx], and tblastx); 3 is both, 1 is top, 2 is bottom [Integer]; default = 3; -T Produce HTML output [T/F]; default = F; -I Restrict search of database to list of Gl's [String] Optional; -U Use lower case filtering of FASTA sequence [T/F] Optional; default = F; -y X dropoff value for ungapped extensions in bits (0.0 invokes default behavior); blastn 20, megablast 10, all others 7 [Real]; default = 0.0; -Z X dropoff value for final gapped alignment in bits (0.0 invokes default behavior);
blastn/megablast 50, tblastx 0, all others 25 [Integer]; default = 0; -R PSI-TBLASTN checkpoint file [File In] Optional; -n MegaBlast search [T/F]; default = F; -L Location on query sequence [String] Optional; -A Multiple Hits window size, default if zero (blastn/megablast 0, all others 40 [Integer]; default = 0; -w Frame shift penalty (OOF algorithm for blastx) [Integer]; default = 0; -t Length of the largest intron allowed in tblastn for linking HSPs (0 disables linking) [Integer]; default = 0.

Results of high quality are reached by using the algorithm of Needleman and Wunsch or Smith and Waterman. Therefore programs based on said algorithms are preferred. Advantageously the comparisons of sequences can be done with the program PileUp (J. Mol. Evolution., 25, 351 (1987), Higgins et al., CABIOS 5, 151 (1989)) or preferably with the programs "Gap" and "Needle", which are both based on the algorithms of Needleman and Wunsch (J. Mol. Biol. 48; 443 (1970)), and "BestFit", which is based on the algorithm of Smith and Waterman (Adv. Appl. Math. 2; 482 (1981)). "Gap" and "BestFit" are part of the GCG software-package (Genetics Computer Group, 575 Science Drive, Madison, Wisconsin, USA 53711 (1991); Altschul et al., (Nucleic Acids Res. 25, 3389 (1997)), "Needle" is part of the The European Molecular Biology Open Software Suite (EMBOSS) (Trends in Genetics 16 (6), 276 (2000)). Therefore preferably the calculations to determine the percentages of sequence homology are done with the programs "Gap" or "Needle" over the whole range of the sequences. The following standard adjustments for the comparison of nucleic acid sequences were used for "Needle" : matrix: EDNAFULL, Gap_penalty: 10.0, Extend_penalty: 0.5. The following standard adjustments for the comparison of nucleic acid sequences were used for "Gap" : gap weight: 50, length weight: 3, average match: 10.000, average mismatch: 0.000.

For example a sequence, which is said to have 80% identity with sequence SEQ ID NO: 1 at the nucleic acid level is understood as meaning a sequence which, upon comparison with the sequence represented by SEQ ID NO: 1 by the above program " Needle" with the above parameter set, has a 80% identity. Preferably the homology is calculated on the complete length of the query sequence, for example SEQ ID NO:1.

Intron: refers to sections of DNA (intervening sequences) within a gene that do not encode part of the protein that the gene produces, and that is spliced out of the mRNA that is transcribed from the gene before it is exported from the cell nucleus. Intron sequence refers to the nucleic acid sequence of an intron. Thus, introns are those regions of DNA sequences that are transcribed along with the coding sequence (exons) but are removed during the formation of mature mRNA. Introns can be positioned within the actual coding region or in either the 5' or 3' untranslated leaders of the pre-mRNA (unspliced mRNA). Introns in the primary transcript are excised and the coding sequences are simultaneously and precisely ligated to form the mature mRNA. The junctions of introns and exons form the splice site. The sequence of an intron begins with GU and ends with AG. Furthermore, in plants, two examples of AU-AC introns have been described: the fourteenth intron of the RecA-like protein gene and the seventh intron of the G5 gene from Arabidopsis thaliana are AT-AC introns. Pre-mRNAs containing introns have three short sequences that are - beside other sequences- essential for the intron to be accurately spliced. These sequences are the 5' splice-site, the 3' splice-site, and the branchpoint. mRNA splicing is the removal of intervening sequences (introns) present in primary mRNA transcripts and joining or ligation of exon sequences. This is also known as cis-splicing which joins two exons on the same RNA with the removal of the intervening sequence (intron). The functional elements of an intron is comprising sequences that are recognized and bound by the specific protein components of the spliceosome (e.g. splicing consensus sequences at the ends of introns). The interaction of the functional elements with the spliceosome results in the removal of the intron sequence from the premature mRNA and the rejoining of the exon sequences. Introns have three short sequences that are essential -although not sufficient- for the intron to be accurately spliced. These sequences are the 5' splice site, the 3' splice site and the branch point. The branchpoint sequence is important in splicing and splice-site selection in plants. The branchpoint sequence is usually located 10-60 nucleotides upstream of the 3' splice site.

Isogenic: organisms (e.g., plants), which are genetically identical, except that they may differ by the presence or absence of a heterologous DNA sequence.

Isolated: The term "isolated" or "isolation" as used herein means that a material has been removed by the hand of man and exists apart from its original, native environment and is therefore not a product of nature. An isolated material or molecule (such as a DNA molecule or enzyme) may exist in a purified form or may exist in a non-native environment such as, for example, in a transgenic host cell. For example, a naturally occurring polynucleotide or polypeptide present in a living plant is not isolated, but the same polynucleotide or polypeptide, separated from some or all of the coexisting materials in the natural system, is isolated. Such polynucleotides can be part of a vector and/or such polynucleotides or polypeptides could be part of a composition, and would be isolated in that such a vector or composition is not part of its original environment. Preferably, the term "isolated" when used in relation to a nucleic acid molecule, as in "an isolated nucleic acid sequence" refers to a nucleic acid sequence that is identified and separated from at least one contaminant nucleic acid molecule with which it is ordinarily associated in its natural source. Isolated nucleic acid molecule is nucleic acid molecule present in a form or setting that is different from that in which it is found in nature. In contrast, non-isolated nucleic acid molecules are nucleic acid molecules such as DNA and RNA, which are found in the state they exist in nature. For example, a given DNA sequence (e.g., a gene) is found on the host cell chromosome in proximity to neighboring genes; RNA sequences, such as a specific mRNA sequence encoding a specific protein, are found in the cell as a mixture with numerous other mRNAs, which encode a multitude of proteins. However, an isolated nucleic acid sequence comprising for example SEQ ID NO: 1 includes, by way of example, such nucleic acid sequences in cells which ordinarily contain SEQ ID NO:1 where the nucleic acid sequence is in a chromosomal or extrachromosomal location different from that of natural cells, or is otherwise flanked by a different nucleic acid sequence than that found in nature. The isolated nucleic acid sequence may be present in single-stranded or double-stranded form. When an isolated nucleic acid sequence is to be utilized to express a protein, the nucleic acid sequence will contain at a minimum at least a portion of the sense or coding strand (i.e., the nucleic acid sequence may be single-stranded). Alternatively, it may contain both the sense and anti-sense strands (i.e., the nucleic acid sequence may be double-stranded).

Minimal Promoter: promoter elements, particularly a TATA element, that are inactive or that have greatly reduced promoter activity in the absence of upstream activation. In the presence of a suitable transcription factor, the minimal promoter functions to permit transcription.

NEENA: see " Nucleic acid expression enhancing nucleic acid".

Non-coding: The term "non-coding" refers to sequences of nucleic acid molecules that do not encode part or all of an expressed protein. Non-coding sequences include but are not limited to introns, enhancers, promoter regions, 3' untranslated regions, and 5' untranslated regions.

Nucleic acid expression enhancing nucleic acid (NEENA): The term "nucleic acid expression enhancing nucleic acid" refers to a sequence and/or a nucleic acid molecule of a specific sequence having the intrinsic property to enhance expression of a nucleic acid under the control of a promoter to which the NEENA is functionally linked. Unlike promoter sequences, the NEENA as such is not able to drive expression. In order to fulfill the function of enhancing expression of a nucleic acid molecule functionally linked to the NEENA, the NEENA itself has to be functionally linked to a promoter. In distinction to enhancer sequences known in the art, the NEENA is acting in cis but not in trans and has to be located close to the transcription start site of the nucleic acid to be expressed.

Nucleic acids and nucleotides: The terms "Nucleic Acids" and "Nucleotides" refer to naturally occurring or synthetic or artificial nucleic acid or nucleotides. The terms "nucleic acids" and "nucleotides" comprise deoxyribonucleotides or ribonucleotides or any nucleotide analogue and polymers or hybrids thereof in either single- or double-stranded, sense or antisense form. Unless otherwise indicated, a particular nucleic acid sequence also implicitly encompasses conservatively modified variants thereof (e.g., degenerate codon substitutions) and complementary sequences, as well as the sequence explicitly indicated. The term "nucleic acid" is used interchangeably herein with "gene", "cDNA, "mRNA", "oligonucleotide," and "polynucleotide". Nucleotide analogues include nucleotides having modifications in the chemical structure of the base, sugar and/or phosphate, including, but not limited to, 5-position pyrimidine modifications, 8-position purine modifications, modifications at cytosine exocyclic amines, substitution of 5-bromo-uracil, and the like; and 2'-position sugar modifications, including but not limited to, sugar-modified ribonucleotides in which the 2'-OH is replaced by a group selected from H, OR, R, halo, SH, SR, NH2, NHR, NR2, or CN. Short hairpin RNAs (shRNAs) also can comprise non-natural elements such as non-natural bases, e.g., ionosin and xanthine, non-natural sugars, e.g., 2'-methoxy ribose, or non-natural phosphodiester linkages, e.g., methylphosphonates, phosphorothioates and peptides.

Nucleic acid sequence: The phrase "nucleic acid sequence" refers to a single or double-stranded polymer of deoxyribonucleotide or ribonucleotide bases read from the 5'- to the 3'-end. It includes chromosomal DNA, self-replicating plasmids, infectious polymers of DNA or RNA and DNA or RNA that performs a primarily structural role. "Nucleic acid sequence" also refers to a consecutive list of abbreviations, letters, characters or words, which represent nucleotides. In one embodiment, a nucleic acid can be a "probe" which is a relatively short nucleic acid, usually less than 100 nucleotides in length. Often a nucleic acid probe is from about 50 nucleotides in length to about 10 nucleotides in length. A "target region" of a nucleic acid is a portion of a nucleic acid that is identified to be of interest. A "coding region" of a nucleic acid is the portion of the nucleic acid, which is transcribed and translated in a sequence-specific manner to produce into a particular polypeptide or protein when placed under the control of appropriate regulatory sequences. The coding region is said to encode such a polypeptide or protein.

Oligonucleotide: The term "oligonucleotide" refers to an oligomer or polymer of ribonucleic acid (RNA) or deoxyribonucleic acid (DNA) or mimetics thereof, as well as oligonucleotides having non-naturally-occurring portions which function similarly. Such modified or substituted oligonucleotides are often preferred over native forms because of desirable properties such as, for example, enhanced cellular uptake, enhanced affinity for nucleic acid target and increased stability in the presence of nucleases. An oligonucleotide preferably includes two or more nucleomonomers covalently coupled to each other by linkages (e.g., phosphodiesters) or substitute linkages.

Overhang: An "overhang" is a relatively short single-stranded nucleotide sequence on the 5'- or 3'-hydroxyl end of a double-stranded oligonucleotide molecule (also referred to as an "extension," "protruding end," or "sticky end").

Plant: is generally understood as meaning any eukaryotic single-or multi-celled organism or a cell, tissue, organ, part or propagation material (such as seeds or fruit) of same which is capable of photosynthesis. Included for the purpose of the invention are all genera and species of higher and lower plants of the Plant Kingdom. Annual, perennial, monocotyledonous and dicotyledonous plants are preferred. The term includes the mature plants, seed, shoots and seedlings and their derived parts, propagation material (such as seeds or microspores), plant organs, tissue, protoplasts, callus and other cultures, for example cell cultures, and any other type of plant cell grouping to give functional or structural units. Mature plants refer to plants at any desired developmental stage beyond that of the seedling. Seedling refers to a young immature plant at an early developmental stage. Annual, biennial, monocotyledonous and dicotyledonous plants are preferred host organisms for the generation of transgenic plants. The expression of genes is furthermore advantageous in all ornamental plants, useful or ornamental trees, flowers, cut flowers, shrubs or lawns. Plants which may be mentioned by way of example but not by limitation are angiosperms, bryophytes such as, for example, Hepaticae (liverworts) and Musci (mosses); Pteridophytes such as ferns, horsetail and club mosses; gymnosperms such as conifers, cycads, ginkgo and Gnetatae; algae such as Chlorophyceae, Phaeophpyceae, Rhodophyceae, Myxophyceae, Xanthophyceae, Bacillariophyceae (diatoms), and Euglenophyceae. Preferred are plants which are used for food or feed purpose such as the families of the Leguminosae such as pea, alfalfa and soya; Gramineae such as rice, maize, wheat, barley, sorghum, millet, rye, triticale, or oats; the family of the Umbelliferae, especially the genus Daucus, very especially the species carota (carrot) and Apium, very especially the species Graveolens dulce (celery) and many others; the family of the Solanaceae, especially the genus Lycopersicon, very especially the species esculentum (tomato) and the genus Solanum, very especially the species tuberosum (potato) and melongena (egg plant), and many others (such as tobacco); and the genus Capsicum, very especially the species annuum (peppers) and many others; the family of the Leguminosae, especially the genus Glycine, very especially the species max (soybean), alfalfa, pea, lucerne, beans or peanut and many others; and the family of the Cruciferae (Brassicacae), especially the genus Brassica, very especially the species napus (oil seed rape), campestris (beet), oleracea cv Tastie (cabbage), oleracea cv Snowball Y (cauliflower) and oleracea cv Emperor (broccoli); and of the genus Arabidopsis, very especially the species thaliana and many others; the family of the Compositae, especially the genus Lactuca, very especially the species sativa (lettuce) and many others; the family of the Asteraceae such as sunflower, Tagetes, lettuce or Calendula and many other; the family of the Cucurbitaceae such as melon, pumpkin/squash or zucchini, and linseed. Further preferred are cotton, sugar cane, hemp, flax, chillies, and the various tree, nut and wine species.

Polypeptide: The terms "polypeptide", "peptide", "oligopeptide", "polypeptide", "gene product", "expression product" and "protein" are used interchangeably herein to refer to a polymer or oligomer of consecutive amino acid residues.

Pre-protein: Protein, which is normally targeted to a cellular organelle, such as a chloroplast, and still comprising its transit peptide.

Primary transcript: The term "primary transcript" as used herein refers to a premature RNA transcript of a gene. A" primary transcript" for example still comprises introns and/or is not yet comprising a polyA tail or a cap structure and/or is missing other modifications necessary for its correct function as transcript such as for example trimming or editing.

Promoter: The terms "promoter", or "promoter sequence" are equivalents and as used herein, refer to a DNA sequence which when ligated to a nucleotide sequence of interest is capable of controlling the transcription of the nucleotide sequence of interest into RNA. Such promoters can for example be found in the following public databases
http://www.grassius.org/grasspromdb.html,
http://mendel.cs.rhul.ac.uk/mendel.php?topic=plantprom, http://ppdb.gene.nagoya-u.ac.jp/cgibin/index.cgi. Promoters listed there may be addressed with the methods of the invention and are herewith included by reference. A promoter is located 5' (i.e., upstream), proximal to the transcriptional start site of a nucleotide sequence of interest whose transcription into mRNA it controls, and provides a site for specific binding by RNA polymerase and other transcription factors for initiation of transcription. Said promoter comprises for example the at least 10 kb, for example 5 kb or 2 kb proximal to the transcription start site. It may also comprise the at least 1500 bp proximal to the transcriptional start site, preferably the at least 1000 bp, more preferably the at least 500 bp, even more preferably the at least 400 bp, the at least 300 bp, the at least 200 bp or the at least 100 bp. In a further preferred embodiment, the promoter comprises the at least 50 bp proximal to the transcription start site, for example, at least 25 bp. The promoter does not comprise exon and/or intron regions or 5' untranslated regions. The promoter may for example be heterologous or homologous to the respective plant. A polynucleotide sequence is "heterologous to" an organism or a second polynucleotide sequence if it originates from a foreign species, or, if from the same species, is modified from its original form. For example, a promoter operably linked to a heterologous coding sequence refers to a coding sequence from a species different from that from which the promoter was derived, or, if from the same species, a coding sequence which is not naturally associated with the promoter (e.g. a genetically engineered coding sequence or an allele from a different ecotype or variety). Suitable promoters can be derived from genes of the host cells where expression should occur or from pathogens for this host cells (e.g., plants or plant pathogens like plant viruses). A plant specific promoter is a promoter suitable for regulating expression in a plant. It may be derived from a plant but also from plant pathogens or it might be a synthetic promoter designed by man. If a promoter is an inducible promoter, then the rate of transcription increases in response to an inducing agent. Also, the promoter may be regulated in a tissue-specific or tissue preferred manner such that it is only or predominantly active in transcribing the associated coding region in a specific tissue type(s) such as leaves, roots or meristem. The term "tissue specific" as it applies to a promoter refers to a promoter that is capable of directing selective expression of a nucleotide sequence of interest to a specific type of tissue (e.g., petals) in the relative absence of expression of the same nucleotide sequence of interest in a different type of tissue (e.g., roots). Tissue specificity of a promoter may be evaluated by, for example, operably linking a reporter gene to the promoter sequence to generate a reporter construct, introducing the reporter construct into the genome of a plant such that the reporter construct is integrated into every tissue of the resulting transgenic plant, and detecting the expression of the reporter gene (e.g., detecting mRNA, protein, or the activity of a protein encoded by the reporter gene) in different tissues of the transgenic plant. The detection of a greater level of expression of the reporter gene in one or more tissues relative to the level of expression of the reporter gene in other tissues shows that the promoter is specific for the tissues in which greater levels of expression are detected. The term "cell type specific" as applied to a promoter refers to a promoter, which is capable of directing selective expression of a nucleotide sequence of interest in a specific type of cell in the relative absence of expression of the same nucleotide sequence of interest in a different type of cell within the same tissue. The term "cell type specific" when applied to a promoter also means a promoter capable of promoting selective expression of a nucleotide sequence of interest in a region within a single tissue. Cell type specificity of a promoter may be assessed using methods well known in the art, e.g., GUS activity staining, GFP protein or immunohistochemical staining. The term "constitutive" when made in reference to a promoter or the expression derived from a promoter means that the promoter is capable of directing transcription of an operably linked nucleic acid molecule in the absence of a stimulus (e.g., heat shock, chemicals, light, etc.) in the majority of plant tissues and cells throughout substantially the entire lifespan of a plant or part of a plant. Typically, constitutive promoters are capable of directing expression of a transgene in substantially any cell and any tissue.

Promoter specificity: The term "specificity" when referring to a promoter means the pattern of expression conferred by the respective promoter. The specificity describes the tissues and/or developmental status of a plant or part thereof, in which the promoter is conferring expression of the nucleic acid molecule under the control of the respective promoter. Specificity of a promoter may also comprise the environmental conditions, under which the promoter may be activated or down-regulated such as induction or repression by biological or environmental stresses such as cold, drought, wounding or infection.

Purified: As used herein, the term "purified" refers to molecules, either nucleic or amino acid sequences that are removed from their natural environment, isolated or separated. "Substantially purified" molecules are at least 60% free, preferably at least 75% free, and more preferably at least 90% free from other components with which they are naturally associated. A purified nucleic acid sequence may be an isolated nucleic acid sequence.

Recombinant: The term "recombinant" with respect to nucleic acid molecules refers to nucleic acid molecules produced by recombinant DNA techniques. Recombinant nucleic acid molecules may also comprise molecules, which as such does not exist in nature but are modified, changed, mutated or otherwise manipulated by man. Preferably, a "recombinant nucleic acid molecule" is a non-naturally occurring nucleic acid molecule that differs in sequence from a naturally occurring nucleic acid molecule by at least one nucleic acid. A "recombinant nucleic acid molecule" may also comprise a "recombinant construct" which comprises, preferably operably linked, a sequence of nucleic acid molecules not naturally occurring in that order. Preferred methods for producing said recombinant nucleic acid molecule may comprise cloning techniques, directed or non-directed mutagenesis, synthesis or recombination techniques.

"Seed-specific promoter" in the context of this invention means a promoter which is regulating transcription of a nucleic acid molecule under control of the respective promoter in seeds wherein the transcription in any tissue or cell of the seeds contribute to more than 90%, preferably more than 95%, more preferably more than 99% of the entire quantity of the RNA transcribed from said nucleic acid sequence in the entire plant during any of its developmental stage. The term "seed-specific expression" is to be understood accordingly.

"Seed-preferential promoter" in the context of this invention means a promoter which is regulating transcription of a nucleic acid molecule under control of the respective promoter in seeds wherein the transcription in any tissue or cell of the seeds contribute to more than 50%, preferably more than 70%, more preferably more than 80% of the entire quantity of the RNA transcribed from said nucleic acid sequence in the entire plant during any of its developmental stage. The term "seed-preferential expression" is to be understood accordingly.

Sense: The term "sense" is understood to mean a nucleic acid molecule having a sequence which is complementary or identical to a target sequence, for example a sequence which binds to a protein transcription factor and which is involved in the expression of a given gene. According to a preferred embodiment, the nucleic acid molecule comprises a gene of interest and elements allowing the expression of the said gene of interest.

Significant increase or decrease: An increase or decrease, for example in enzymatic activity or in gene expression, that is larger than the margin of error inherent in the measurement technique, preferably an increase or decrease by about 2-fold or greater of the activity of the control enzyme or expression in the control cell, more preferably an increase or decrease by about 5-fold or greater, and most preferably an increase or decrease by about 10-fold or greater.

Small nucleic acid molecules: "small nucleic acid molecules" are understood as molecules consisting of nucleic acids or derivatives thereof such as RNA or DNA. They may be double-stranded or single-stranded and are between about 15 and about 30 bp, for example between 15 and 30 bp, more preferred between about 19 and about 26 bp, for example between 19 and 26 bp, even more preferred between about 20 and about 25 bp for example between 20 and 25 bp. In a especially preferred embodiment the oligonucleotides are between about 21 and about 24 bp, for example between 21 and 24 bp. In a most preferred embodiment, the small nucleic acid molecules are about 21 bp and about 24 bp, for example 21 bp and 24 bp.

Substantially complementary: In its broadest sense, the term "substantially complementary", when used herein with respect to a nucleotide sequence in relation to a reference or target nucleotide sequence, means a nucleotide sequence having a percentage of identity between the substantially complementary nucleotide sequence and the exact complementary sequence of said reference or target nucleotide sequence of at least 60%, more desirably at least 70%, more desirably at least 80% or 85%, preferably at least 90%, more preferably at least 93%, still more preferably at least 95% or 96%, yet still more preferably at least 97% or 98%, yet still more preferably at least 99% or most preferably 100% (the later being equivalent to the term "identical" in this context). Preferably identity is assessed over a length of at least 19 nucleotides, preferably at least 50 nucleotides, more preferably the entire length of the nucleic acid sequence to said reference sequence (if not specified otherwise below). Sequence comparisons are carried out using default GAP analysis with the University of Wisconsin GCG, SEQWEB application of GAP, based on the algorithm of Needleman and Wunsch (Needleman and Wunsch (1970) J Mol. Biol. 48: 443-453; as defined above). A nucleotide sequence "substantially complementary " to a reference nucleotide sequence hybridizes to the reference nucleotide sequence under low stringency conditions, preferably medium stringency conditions, most preferably high stringency conditions (as defined above).

Terminator: The term "terminator" "transcription terminator" or "transcription terminator sequence" as used herein is intended to mean a sequence located in the 3'UTR of a gene that causes a polymerase to stop forming phosphodiester bonds and release the nascent transcript. As used herein, the terminator comprises the entire 3'UTR structure necessary for efficient production of a messenger RNA. The terminator sequence leads to or initiates a stop of transcription of a nucleic acid sequence initiated from a promoter. Preferably, a transcription terminator sequences is furthermore comprising sequences which cause polyadenylation of the transcript. A transcription terminator may, for example, comprise one or more polyadenylation signal sequences, one or more polyadenylation attachment sequences, and downstream sequence of various lengths which causes termination of transcription. It has to be understood that also sequences downstream of sequences coding for the 3'-untranslated region of an expressed RNA transcript may be part of a transcription terminator although the sequence itself is not expressed as part of the RNA transcript. Furthermore, a transcription terminator may comprise additional sequences, which may influence its functionality, such a 3'-untranslated sequences (*i.e.* sequences of a gene following the stop-codon of the coding sequence). Transcription termination may involve various mechanisms including but not limited to induced dissociation of RNA polymerase II from their DNA template.

Transgene: The term "transgene" as used herein refers to any nucleic acid sequence, which is introduced into the genome of a cell by experimental manipulations. A transgene may be an "endogenous DNA sequence," or a "heterologous DNA sequence" (i.e., "foreign DNA"). The term "endogenous DNA sequence" refers to a nucleotide sequence, which is naturally found in the cell into which it is introduced so long as it does not contain some modification (e.g., a point mutation, the presence of a selectable marker gene, etc.) relative to the naturally-occurring sequence.

Transgenic: The term transgenic when referring to an organism means transformed, preferably stably transformed, with a recombinant DNA molecule that preferably comprises a suitable promoter operatively linked to a DNA sequence of interest.

Vector: As used herein, the term "vector" refers to a nucleic acid molecule capable of transporting another nucleic acid molecule to which it has been linked. One type of vector is a genomic integrated vector, or "integrated vector", which can become integrated into the chromosomal DNA of the host cell. Another type of vector is an episomal vector, i.e., a nucleic acid molecule capable of extra-chromosomal replication. Vectors capable of directing the expression of genes to which they are operatively linked are referred to herein as "expression vectors". In the present specification, "plasmid" and "vector" are used interchangeably unless otherwise clear from the context. Expression vectors designed to produce RNAs as described herein in vitro or in vivo may contain sequences recognized by any RNA polymerase, including mitochondrial RNA polymerase, RNA pol I, RNA pol II, and RNA pol III. These vectors can be used to transcribe the desired RNA molecule in the cell according to this invention. A plant transformation vector is to be understood as a vector suitable in the process of plant transformation.

Wild-type: The term "wild-type", "natural" or "natural origin" means with respect to an organism, polypeptide, or nucleic acid sequence, that said organism is naturally occurring or available in at least one naturally occurring organism which is not changed, mutated, or otherwise manipulated by man.

### Examples

### Chemicals and common methods

Unless indicated otherwise, cloning procedures carried out for the purposes of the present invention including restriction digest, agarose gel electrophoresis, purification of nucleic acids, ligation of nucleic acids, transformation, selection and cultivation of bacterial cells were performed as described (Sambrook et al., 1989). Sequence analyses of recombinant DNA were performed with a laser fluorescence DNA sequencer (Applied Biosystems, Foster City, CA, USA) using the Sanger technology (Sanger et al., 1977). Unless described otherwise, chemicals and reagents were obtained from Sigma Aldrich (Sigma Aldrich, St. Louis, USA), from Promega (Madison, WI, USA), Duchefa (Haarlem, The Netherlands) or Invitrogen (Carlsbad, CA, USA). Restriction endonucleases were from New England Biolabs (Ipswich, MA, USA) or Roche Diagnostics GmbH (Penzberg, Germany). Oligonucleotides were synthesized by Eurofins MWG Operon (Ebersberg, Germany).

### Example 1: Identification of Oryza sativa terminators putatively enhancing gene expression

Sequence elements of terminators enhancing gene expression of a functionally linked gene were identified. Table 1 gives an overview over the 43 Enhancing Terminator (ET) elements.

**Table1: Expression enhancing terminator (ET) sequence elements**

| **Line No**. | **ET ID** | **SEQ ID NO** | **IUPAC sequence** |
|---|---|---|---|
| 1 | ET1 | SEQ ID N05 | NRYCTTCCCWTYWWNNTDNNNCN |
| 2 | ET2 | SEQ ID N06 | NGTGATWTTNCWNSN |
| 3 | ET3 | SEQ ID NO7 | BTMMTTTTCCSTTV |
| 4 | ET4 | SEQ ID NO8 | DAVAGCCATCAVT |
| 5 | ET5 | SEQ ID NO9 | DCTTRNTATTTKAV |
| 6 | ET6 | SEQ ID NO10 | NADHATNTNNDKWTGGTTTGTHNNAN |
| 7 | ET7 | SEQ ID NO11 | NWANAATGASANNNNAHNAN |
| 8 | ET8 | SEQ ID NO12 | NAAAAGTAN |
| 9 | ET9 | SEQ ID NO13 | WKWNNTGGAAGCAT |
| 10 | ET10 | SEQ ID NO14 | NWNNNHNWNTGNTATTN |
| 11 | ET11 | SEQ ID NO15 | WYNNNHNNMNSNAAACTCANVAN |
| 12 | ET12 | SEQ ID NO16 | NWWKWNTNNTHATTATGMTN |
| 13 | ET13 | SEQ ID NO17 | YGATGGCNNTAN |
| 14 | ET14 | SEQ ID NO18 | YHNNTTGTKTCNKNNKNMNNNVM |
| 15 | ET15 | SEQ ID NO19 | NHNTYNNKTGCTTTKTNDN |
| 16 | ET16 | SEQ ID NO20 | ATKTTTCCTGYDNMAY |
| 17 | ET17 | SEQ ID NO21 | WMCTATTGTNMWWWNKTA |
| 18 | ET18 | SEQ ID NO22 | TTTTCTCYTWCYTCTSMY |
| 19 | ET19 | SEQ ID NO23 | KTTGRTTCYN |
| 20 | ET20 | SEQ ID NO24 | TKATATTGYNDWAYWWR |
| 21 | ET21 | SEQ ID NO25 | WSNWAACTWGAW |
| 22 | ET22 | SEQ ID NO26 | NWTNTTATGNTM |
| 23 | ET23 | SEQ ID NO27 | WMWWBTCAATAAB |
| 24 | ET24 | SEQ ID NO28 | NTYANKDTTCYTGTGAA |
| 25 | ET25 | SEQ ID NO29 | TNNRWKNNGTGTTCTN |
| 26 | ET26 | SEQ ID NO30 | NTATTGTSRHD |
| 27 | ET27 | SEQ ID NO31 | NWTTGTTTCN |
| 28 | ET28 | SEQ ID NO32 | NWNNMCCTKTCCNNNRN |
| 29 | ET29 | SEQ ID NO33 | NTTASYKNAWTDKCACCAAN |
| 30 | ET30 | SEQ ID NO34 | NNTTACTGSNWNNNNRN |
| 31 | ET31 | SEQ ID NO35 | NRRWNTTAATAANKWT |
| 32 | ET32 | SEQ ID NO36 | NTNTCTGNTAN |
| 33 | ET33 | SEQ ID NO37 | NNNHNNTTGTTTCNNHWKNMN |
| 34 | ET34 | SEQ ID NO38 | NTYANKDTTCYTGTGAA |
| 35 | ET35 | SEQ ID NO39 | YKTNNTTGCTTTN |
| 36 | ET36 | SEQ ID NO40 | NGTGATWTTNCWNSN |
| 37 | ET37 | SEQ ID NO41 | MWNSRNNNBTNBRHGGCTTGTWN |
| 38 | ET38 | SEQ ID NO42 | NYCTTTTSCNNNANYWAAN |
| 39 | ET39 | SEQ ID NO43 | NWTGCTACCN |
| 40 | ET40 | SEQ ID NO44 | NAWTYTGATGANNAWNAW |
| 41 | ET41 | SEQ ID NO45 | WGWNABNMKMNAGMTCCACN |
| 42 | ET42 | SEQ ID NO46 | NTCATAAGNRBA |
| 43 | ET43 | SEQ ID NO47 | DTMATTTTSY |

| | | | |
|---|---|---|---|
| A = adenine; C = cytosine; G = guanine; T = thymine; U = uracil; R = G A (purine); Y = T C (pyrimidine); K = G T (keto); M = A C (amino); S = G C ; W = A T; B = G T C; D = G A T; H = A C T; V = G C A; N = A G C T (any) | | | |

ET elements were used to screen for termiantor sequences with gene expression enhancing properties. Terminators enhancing expression were defined by a combination of ET elements. Table 2 lists combination groups (one line represents one group) that were sufficient to identify a gene expression enhancing terminator molecule. Each line defines a group of ET elements characteristic for gene expression enhancing terminator molecules.

**Table2: Combination groups of expression Enhancing Terminator (ET) sequence elements**

| **Line No.** | **ET element 1** | **ET element 2** | **ET element 3** | **ET element 4** | **ET element 5** | **ET element 6** |
|---|---|---|---|---|---|---|
| 1 | SEQ ID NO5 | SEQ ID NO6 | | | | |
| 2 | SEQ ID NO5 | | | | | |
| 3 | SEQ ID NO6 | | | | | |
| 4 | SEQ ID NO43 | SEQ ID NO47 | | | | |
| 5 | SEQ ID NO44 | SEQ ID NO32 | | | | |
| 6 | SEQ ID NO44 | SEQ ID NO36 | | | | |
| 7 | SEQ ID NO33 | SEQ ID NO44 | | | | |
| 8 | SEQ ID NO45 | SEQ ID NO26 | | | | |
| 9 | SEQ ID NO46 | SEQ ID NO31 | | | | |
| 10 | SEQ ID NO34 | SEQ ID NO11 | | | | |
| 11 | SEQ ID NO18 | SEQ ID NO46 | | | | |
| 12 | SEQ ID NO41 | SEQ ID NO44 | | | | |
| 13 | SEQ ID NO30 | SEQ ID NO8 | | | | |
| 14 | SEQ ID NO16 | SEQ ID NO36 | SEQ ID NO31 | | | |
| 15 | SEQ ID NO16 | SEQ ID NO10 | SEQ ID NO31 | | | |
| 16 | SEQ ID NO16 | SEQ ID NO18 | SEQ ID NO36 | | | |
| 17 | SEQ ID NO16 | SEQ ID NO18 | SEQ ID NO10 | | | |
| 18 | SEQ ID NO16 | SEQ ID NO15 | SEQ ID NO37 | | | |
| 19 | SEQ ID NO20 | SEQ ID NO25 | SEQ ID NO32 | | | |
| 20 | SEQ ID NO20 | SEQ ID NO25 | SEQ ID NO36 | | | |
| 21 | SEQ ID NO8 | SEQ ID NO32 | SEQ ID NO11 | | | |
| 22 | SEQ ID NO8 | SEQ ID NO22 | SEQ ID NO32 | | | |
| 23 | SEQ ID NO8 | SEQ ID NO22 | SEQ ID NO36 | | | |
| 24 | SEQ ID NO8 | SEQ ID NO36 | SEQ ID NO11 | | | |
| 25 | SEQ ID NO8 | SEQ ID NO13 | SEQ ID NO32 | | | |
| 26 | SEQ ID NO43 | SEQ ID NO11 | SEQ ID NO31 | | | |
| 27 | SEQ ID NO43 | SEQ ID NO13 | SEQ ID NO31 | | | |
| 28 | SEQ ID NO43 | SEQ ID NO13 | SEQ ID NO18 | | | |
| 29 | SEQ ID NO43 | SEQ ID NO18 | SEQ ID NO11 | | | |
| 30 | SEQ ID NO23 | SEQ ID NO14 | SEQ ID NO25 | | | |
| 31 | SEQ ID NO23 | SEQ ID NO15 | SEQ ID NO25 | | | |
| 32 | SEQ ID NO33 | SEQ ID NO8 | SEQ ID NO22 | | | |
| 33 | SEQ ID NO33 | SEQ ID NO8 | SEQ ID NO11 | | | |
| 34 | SEQ ID NO33 | SEQ ID NO27 | SEQ ID NO17 | | | |
| 35 | SEQ ID NO33 | SEQ ID NO27 | SEQ ID NO42 | | | |
| 36 | SEQ ID NO33 | SEQ ID NO19 | SEQ ID NO25 | | | |
| 37 | SEQ ID NO45 | SEQ ID NO43 | SEQ ID NO23 | | | |
| 38 | SEQ ID NO45 | SEQ ID NO46 | SEQ ID NO37 | | | |
| 39 | SEQ ID NO35 | SEQ ID NO36 | SEQ ID NO37 | | | |
| 40 | SEQ ID NO27 | SEQ ID NO36 | SEQ ID NO37 | | | |
| 41 | SEQ ID NO27 | SEQ ID NO17 | SEQ ID NO32 | | | |
| 42 | SEQ ID NO27 | SEQ ID NO17 | SEQ ID NO36 | | | |
| 43 | SEQ ID NO27 | SEQ ID NO17 | SEQ ID NO41 | | | |
| 44 | SEQ ID NO27 | SEQ ID NO41 | SEQ ID NO42 | | | |
| 45 | SEQ ID NO27 | SEQ ID NO42 | SEQ ID NO32 | | | |
| 46 | SEQ ID NO27 | SEQ ID NO42 | SEQ ID NO36 | | | |
| 47 | SEQ ID NO17 | SEQ ID NO9 | SEQ ID NO36 | | | |
| 48 | SEQ ID NO17 | SEQ ID NO35 | SEQ ID NO32 | | | |
| 49 | SEQ ID NO17 | SEQ ID NO35 | SEQ ID NO36 | | | |
| 50 | SEQ ID NO17 | SEQ ID NO10 | SEQ ID NO9 | | | |
| 51 | SEQ ID NO17 | SEQ ID NO10 | SEQ ID NO39 | | | |
| 52 | SEQ ID NO17 | SEQ ID NO39 | SEQ ID NO36 | | | |
| 53 | SEQ ID NO17 | SEQ ID NO12 | SEQ ID NO9 | | | |
| 54 | SEQ ID NO17 | SEQ ID NO12 | SEQ ID NO39 | | | |
| 55 | SEQ ID NO12 | SEQ ID NO16 | SEQ ID NO31 | | | |
| 56 | SEQ ID NO12 | SEQ ID NO16 | SEQ ID NO18 | | | |
| 57 | SEQ ID NO12 | SEQ ID NO35 | SEQ ID NO37 | | | |
| 58 | SEQ ID NO12 | SEQ ID NO47 | SEQ ID NO25 | | | |
| 59 | SEQ ID NO29 | SEQ ID NO33 | SEQ ID NO27 | | | |
| 60 | SEQ ID NO29 | SEQ ID NO9 | SEQ ID NO36 | | | |
| 61 | SEQ ID NO29 | SEQ ID NO35 | SEQ ID NO32 | | | |
| 62 | SEQ ID NO29 | SEQ ID NO35 | SEQ ID NO36 | | | |
| 63 | SEQ ID NO29 | SEQ ID NO27 | SEQ ID NO32 | | | |
| 64 | SEQ ID NO29 | SEQ ID NO27 | SEQ ID NO36 | | | |
| 65 | SEQ ID NO29 | SEQ ID NO39 | SEQ ID NO36 | | | |
| 66 | SEQ ID NO29 | SEQ ID NO12 | SEQ ID NO9 | | | |
| 67 | SEQ ID NO29 | SEQ ID NO12 | SEQ ID NO39 | | | |
| 68 | SEQ ID NO40 | SEQ ID NO17 | SEQ ID NO36 | | | |
| 69 | SEQ ID NO40 | SEQ ID NO42 | SEQ ID NO36 | | | |
| 70 | SEQ ID NO41 | SEQ ID NO8 | SEQ ID NO11 | | | |
| 71 | SEQ ID NO41 | SEQ ID NO19 | SEQ ID NO25 | | | |
| 72 | SEQ ID NO42 | SEQ ID NO9 | SEQ ID NO36 | | | |
| 73 | SEQ ID NO42 | SEQ ID NO35 | SEQ ID NO32 | | | |
| 74 | SEQ ID NO42 | SEQ ID NO35 | SEQ ID NO36 | | | |
| 75 | SEQ ID NO42 | SEQ ID NO10 | SEQ ID NO9 | | | |
| 76 | SEQ ID NO42 | SEQ ID NO10 | SEQ ID NO39 | | | |
| 77 | SEQ ID NO42 | SEQ ID NO39 | SEQ ID NO36 | | | |
| 78 | SEQ ID NO42 | SEQ ID NO12 | SEQ ID NO9 | | | |
| 79 | SEQ ID NO42 | SEQ ID NO12 | SEQ ID NO39 | | | |
| 80 | SEQ ID NO47 | SEQ ID NO10 | SEQ ID NO25 | | | |
| 81 | SEQ ID NO22 | SEQ ID NO25 | SEQ ID NO36 | SEQ ID NO31 | | |
| 82 | SEQ ID NO33 | SEQ ID NO43 | SEQ ID NO42 | SEQ ID NO7 | | |
| 83 | SEQ ID NO33 | SEQ ID NO45 | SEQ ID NO17 | SEQ ID NO46 | | |
| 84 | SEQ ID NO33 | SEQ ID NO45 | SEQ ID NO42 | SEQ ID NO46 | | |
| 85 | SEQ ID NO33 | SEQ ID NO28 | SEQ ID NO15 | SEQ ID NO25 | | |
| 86 | SEQ ID NO33 | SEQ ID NO17 | SEQ ID NO16 | SEQ ID NO14 | | |
| 87 | SEQ ID NO33 | SEQ ID NO17 | SEQ ID NO16 | SEQ ID NO15 | | |
| 88 | SEQ ID NO33 | SEQ ID NO17 | SEQ ID NO43 | SEQ ID NO7 | | |
| 89 | SEQ ID NO33 | SEQ ID NO17 | SEQ ID NO35 | SEQ ID NO10 | | |
| 90 | SEQ ID NO33 | SEQ ID NO17 | SEQ ID NO12 | SEQ ID NO35 | | |
| 91 | SEQ ID NO33 | SEQ ID NO38 | SEQ ID NO15 | SEQ ID NO25 | | |
| 92 | SEQ ID NO33 | SEQ ID NO12 | SEQ ID NO20 | SEQ ID NO25 | | |
| 93 | SEQ ID NO33 | SEQ ID NO42 | SEQ ID NO16 | SEQ ID NO14 | | |
| 94 | SEQ ID NO33 | SEQ ID NO42 | SEQ ID NO16 | SEQ ID NO15 | | |
| 95 | SEQ ID NO33 | SEQ ID NO42 | SEQ ID NO35 | SEQ ID NO10 | | |
| 96 | SEQ ID NO33 | SEQ ID NO42 | SEQ ID NO12 | SEQ ID NO35 | | |
| 97 | SEQ ID NO45 | SEQ ID NO16 | SEQ ID NO36 | SEQ ID NO37 | | |
| 98 | SEQ ID NO45 | SEQ ID NO43 | SEQ ID NO13 | SEQ ID NO42 | | |
| 99 | SEQ ID NO45 | SEQ ID NO23 | SEQ ID NO10 | SEQ ID NO25 | | |
| 100 | SEQ ID NO45 | SEQ ID NO28 | SEQ ID NO25 | SEQ ID NO32 | | |
| 101 | SEQ ID NO45 | SEQ ID NO28 | SEQ ID NO25 | SEQ ID NO36 | | |
| 102 | SEQ ID NO45 | SEQ ID NO17 | SEQ ID NO16 | SEQ ID NO32 | | |
| 103 | SEQ ID NO45 | SEQ ID NO17 | SEQ ID NO16 | SEQ ID NO36 | | |
| 104 | SEQ ID NO45 | SEQ ID NO17 | SEQ ID NO43 | SEQ ID NO13 | | |
| 105 | SEQ ID NO45 | SEQ ID NO17 | SEQ ID NO21 | SEQ ID NO25 | | |
| 106 | SEQ ID NO45 | SEQ ID NO17 | SEQ ID NO41 | SEQ ID NO46 | | |
| 107 | SEQ ID NO45 | SEQ ID NO38 | SEQ ID NO25 | SEQ ID NO32 | | |
| 108 | SEQ ID NO45 | SEQ ID NO38 | SEQ ID NO25 | SEQ ID NO36 | | |
| 109 | SEQ ID NO45 | SEQ ID NO12 | SEQ ID NO16 | SEQ ID NO37 | | |
| 110 | SEQ ID NO45 | SEQ ID NO12 | SEQ ID NO23 | SEQ ID NO25 | | |
| 111 | SEQ ID NO45 | SEQ ID NO41 | SEQ ID NO42 | SEQ ID NO46 | | |
| 112 | SEQ ID NO45 | SEQ ID NO42 | SEQ ID NO16 | SEQ ID NO32 | | |
| 113 | SEQ ID NO45 | SEQ ID NO42 | SEQ ID NO16 | SEQ ID NO36 | | |
| 114 | SEQ ID NO45 | SEQ ID NO42 | SEQ ID NO21 | SEQ ID NO25 | | |
| 115 | SEQ ID NO25 | SEQ ID NO36 | SEQ ID NO11 | SEQ ID NO31 | | |
| 116 | SEQ ID NO10 | SEQ ID NO25 | SEQ ID NO11 | SEQ ID NO31 | | |
| 117 | SEQ ID NO17 | SEQ ID NO16 | SEQ ID NO14 | SEQ ID NO32 | | |
| 118 | SEQ ID NO17 | SEQ ID NO16 | SEQ ID NO14 | SEQ ID NO36 | | |
| 119 | SEQ ID NO17 | SEQ ID NO7 | SEQ ID NO25 | SEQ ID NO32 | | |
| 120 | SEQ ID NO17 | SEQ ID NO7 | SEQ ID NO25 | SEQ ID NO36 | | |
| 121 | SEQ ID NO17 | SEQ ID NO13 | SEQ ID NO24 | SEQ ID NO25 | | |
| 122 | SEQ ID NO17 | SEQ ID NO13 | SEQ ID NO15 | SEQ ID NO25 | | |
| 123 | SEQ ID NO17 | SEQ ID NO41 | SEQ ID NO16 | SEQ ID NO15 | | |
| 124 | SEQ ID NO17 | SEQ ID NO41 | SEQ ID NO12 | SEQ ID NO35 | | |
| 125 | SEQ ID NO7 | SEQ ID NO25 | SEQ ID NO36 | SEQ ID NO37 | | |
| 126 | SEQ ID NO12 | SEQ ID NO22 | SEQ ID NO25 | SEQ ID NO31 | | |
| 127 | SEQ ID NO12 | SEQ ID NO25 | SEQ ID NO11 | SEQ ID NO31 | | |
| 128 | SEQ ID NO12 | SEQ ID NO7 | SEQ ID NO25 | SEQ ID NO37 | | |
| 129 | SEQ ID NO12 | SEQ ID NO18 | SEQ ID NO22 | SEQ ID NO25 | | |
| 130 | SEQ ID NO12 | SEQ ID NO18 | SEQ ID NO25 | SEQ ID NO11 | | |
| 131 | SEQ ID NO29 | SEQ ID NO33 | SEQ ID NO16 | SEQ ID NO15 | | |
| 132 | SEQ ID NO29 | SEQ ID NO33 | SEQ ID NO45 | SEQ ID NO46 | | |
| 133 | SEQ ID NO29 | SEQ ID NO33 | SEQ ID NO12 | SEQ ID NO35 | | |
| 134 | SEQ ID NO29 | SEQ ID NO45 | SEQ ID NO16 | SEQ ID NO32 | | |
| 135 | SEQ ID NO29 | SEQ ID NO45 | SEQ ID NO16 | SEQ ID NO36 | | |
| 136 | SEQ ID NO29 | SEQ ID NO7 | SEQ ID NO25 | SEQ ID NO32 | | |
| 137 | SEQ ID NO29 | SEQ ID NO7 | SEQ ID NO25 | SEQ ID NO36 | | |
| 138 | SEQ ID NO29 | SEQ ID NO13 | SEQ ID NO15 | SEQ ID NO25 | | |
| 139 | SEQ ID NO13 | SEQ ID NO10 | SEQ ID NO25 | SEQ ID NO31 | | |
| 140 | SEQ ID NO13 | SEQ ID NO12 | SEQ ID NO25 | SEQ ID NO31 | | |
| 141 | SEQ ID NO13 | SEQ ID NO12 | SEQ ID NO18 | SEQ ID NO25 | | |
| 142 | SEQ ID NO13 | SEQ ID NO18 | SEQ ID NO10 | SEQ ID NO25 | | |
| 143 | SEQ ID NO13 | SEQ ID NO15 | SEQ ID NO25 | SEQ ID NO37 | | |
| 144 | SEQ ID NO13 | SEQ ID NO42 | SEQ ID NO24 | SEQ ID NO25 | | |
| 145 | SEQ ID NO13 | SEQ ID NO42 | SEQ ID NO15 | SEQ ID NO25 | | |
| 146 | SEQ ID NO18 | SEQ ID NO22 | SEQ ID NO25 | SEQ ID NO36 | | |
| 147 | SEQ ID NO18 | SEQ ID NO25 | SEQ ID NO36 | SEQ ID NO11 | | |
| 148 | SEQ ID NO18 | SEQ ID NO10 | SEQ ID NO25 | SEQ ID NO11 | | |
| 149 | SEQ ID NO41 | SEQ ID NO42 | SEQ ID NO16 | SEQ ID NO15 | | |
| 150 | SEQ ID NO41 | SEQ ID NO42 | SEQ ID NO12 | SEQ ID NO35 | | |
| 151 | SEQ ID NO30 | SEQ ID NO45 | SEQ ID NO17 | SEQ ID NO25 | | |
| 152 | SEQ ID NO30 | SEQ ID NO45 | SEQ ID NO42 | SEQ ID NO25 | | |
| 153 | SEQ ID NO15 | SEQ ID NO25 | SEQ ID NO37 | SEQ ID NO11 | | |
| 154 | SEQ ID NO42 | SEQ ID NO16 | SEQ ID NO14 | SEQ ID NO32 | | |
| 155 | SEQ ID NO42 | SEQ ID NO16 | SEQ ID NO14 | SEQ ID NO36 | | |
| 156 | SEQ ID NO42 | SEQ ID NO7 | SEQ ID NO25 | SEQ ID NO32 | | |
| 157 | SEQ ID NO42 | SEQ ID NO7 | SEQ ID NO25 | SEQ ID NO36 | | |
| 158 | SEQ ID NO33 | SEQ ID NO45 | SEQ ID NO43 | SEQ ID NO42 | SEQ ID NO11 | |
| 159 | SEQ ID NO33 | SEQ ID NO45 | SEQ ID NO17 | SEQ ID NO16 | SEQ ID NO10 | |
| 160 | SEQ ID NO33 | SEQ ID NO45 | SEQ ID NO17 | SEQ ID NO43 | SEQ ID NO11 | |
| 161 | SEQ ID NO33 | SEQ ID NO45 | SEQ ID NO17 | SEQ ID NO12 | SEQ ID NO16 | |
| 162 | SEQ ID NO33 | SEQ ID NO45 | SEQ ID NO38 | SEQ ID NO12 | SEQ ID NO25 | |
| 163 | SEQ ID NO33 | SEQ ID NO45 | SEQ ID NO12 | SEQ ID NO28 | SEQ ID NO25 | |
| 164 | SEQ ID NO33 | SEQ ID NO45 | SEQ ID NO42 | SEQ ID NO16 | SEQ ID NO10 | |
| 165 | SEQ ID NO33 | SEQ ID NO45 | SEQ ID NO42 | SEQ ID NO12 | SEQ ID NO16 | |
| 166 | SEQ ID NO33 | SEQ ID NO17 | SEQ ID NO22 | SEQ ID NO15 | SEQ ID NO25 | |
| 167 | SEQ ID NO33 | SEQ ID NO17 | SEQ ID NO10 | SEQ ID NO7 | SEQ ID NO25 | |
| 168 | SEQ ID NO33 | SEQ ID NO17 | SEQ ID NO12 | SEQ ID NO7 | SEQ ID NO25 | |
| 169 | SEQ ID NO33 | SEQ ID NO17 | SEQ ID NO14 | SEQ ID NO25 | SEQ ID NO11 | |
| 170 | SEQ ID NO33 | SEQ ID NO17 | SEQ ID NO15 | SEQ ID NO25 | SEQ ID NO11 | |
| 171 | SEQ ID NO33 | SEQ ID NO42 | SEQ ID NO22 | SEQ ID NO15 | SEQ ID NO25 | |
| 172 | SEQ ID NO33 | SEQ ID NO42 | SEQ ID NO10 | SEQ ID NO7 | SEQ ID NO25 | |
| 173 | SEQ ID NO33 | SEQ ID NO42 | SEQ ID NO12 | SEQ ID NO7 | SEQ ID NO25 | |
| 174 | SEQ ID NO33 | SEQ ID NO42 | SEQ ID NO14 | SEQ ID NO25 | SEQ ID NO11 | |
| 175 | SEQ ID NO33 | SEQ ID NO42 | SEQ ID NO15 | SEQ ID NO25 | SEQ ID NO11 | |
| 176 | SEQ ID NO45 | SEQ ID NO25 | SEQ ID NO36 | SEQ ID NO37 | SEQ ID NO11 | |
| 177 | SEQ ID NO45 | SEQ ID NO17 | SEQ ID NO22 | SEQ ID NO25 | SEQ ID NO32 | |
| 178 | SEQ ID NO45 | SEQ ID NO17 | SEQ ID NO22 | SEQ ID NO25 | SEQ ID NO36 | |
| 179 | SEQ ID NO45 | SEQ ID NO17 | SEQ ID NO25 | SEQ ID NO32 | SEQ ID NO11 | |
| 180 | SEQ ID NO45 | SEQ ID NO17 | SEQ ID NO25 | SEQ ID NO36 | SEQ ID NO11 | |
| 181 | SEQ ID NO45 | SEQ ID NO17 | SEQ ID NO13 | SEQ ID NO25 | SEQ ID NO32 | |
| 182 | SEQ ID NO45 | SEQ ID NO17 | SEQ ID NO13 | SEQ ID NO10 | SEQ ID NO25 | |
| 183 | SEQ ID NO45 | SEQ ID NO17 | SEQ ID NO13 | SEQ ID NO12 | SEQ ID NO25 | |
| 184 | SEQ ID NO45 | SEQ ID NO17 | SEQ ID NO41 | SEQ ID NO12 | SEQ ID NO16 | |
| 185 | SEQ ID NO45 | SEQ ID NO12 | SEQ ID NO25 | SEQ ID NO37 | SEQ ID NO11 | |
| 186 | SEQ ID NO45 | SEQ ID NO13 | SEQ ID NO12 | SEQ ID NO25 | SEQ ID NO37 | |
| 187 | SEQ ID NO45 | SEQ ID NO13 | SEQ ID NO42 | SEQ ID NO25 | SEQ ID NO32 | |
| 188 | SEQ ID NO45 | SEQ ID NO13 | SEQ ID NO42 | SEQ ID NO10 | SEQ ID NO25 | |
| 189 | SEQ ID NO45 | SEQ ID NO13 | SEQ ID NO42 | SEQ ID NO12 | SEQ ID NO25 | |
| 190 | SEQ ID NO45 | SEQ ID NO41 | SEQ ID NO42 | SEQ ID NO12 | SEQ ID NO16 | |
| 191 | SEQ ID NO45 | SEQ ID NO42 | SEQ ID NO22 | SEQ ID NO25 | SEQ ID NO32 | |
| 192 | SEQ ID NO45 | SEQ ID NO42 | SEQ ID NO22 | SEQ ID NO25 | SEQ ID NO36 | |
| 193 | SEQ ID NO45 | SEQ ID NO42 | SEQ ID NO25 | SEQ ID NO32 | SEQ ID NO11 | |
| 194 | SEQ ID NO45 | SEQ ID NO42 | SEQ ID NO25 | SEQ ID NO36 | SEQ ID NO11 | |
| 195 | SEQ ID NO17 | SEQ ID NO24 | SEQ ID NO25 | SEQ ID NO36 | SEQ ID NO11 | |
| 196 | SEQ ID NO17 | SEQ ID NO13 | SEQ ID NO14 | SEQ ID NO25 | SEQ ID NO32 | |
| 197 | SEQ ID NO17 | SEQ ID NO41 | SEQ ID NO12 | SEQ ID NO7 | SEQ ID NO25 | |
| 198 | SEQ ID NO17 | SEQ ID NO41 | SEQ ID NO15 | SEQ ID NO25 | SEQ ID NO11 | |
| 199 | SEQ ID NO17 | SEQ ID NO14 | SEQ ID NO25 | SEQ ID NO32 | SEQ ID NO11 | |
| 200 | SEQ ID NO17 | SEQ ID NO14 | SEQ ID NO25 | SEQ ID NO36 | SEQ ID NO11 | |
| 201 | SEQ ID NO29 | SEQ ID NO33 | SEQ ID NO45 | SEQ ID NO12 | SEQ ID NO16 | |
| 202 | SEQ ID NO29 | SEQ ID NO33 | SEQ ID NO12 | SEQ ID NO7 | SEQ ID NO25 | |
| 203 | SEQ ID NO29 | SEQ ID NO33 | SEQ ID NO15 | SEQ ID NO25 | SEQ ID NO11 | |
| 204 | SEQ ID NO29 | SEQ ID NO45 | SEQ ID NO25 | SEQ ID NO32 | SEQ ID NO11 | |
| 205 | SEQ ID NO29 | SEQ ID NO45 | SEQ ID NO25 | SEQ ID NO36 | SEQ ID NO11 | |
| 206 | SEQ ID NO29 | SEQ ID NO45 | SEQ ID NO13 | SEQ ID NO25 | SEQ ID NO32 | |
| 207 | SEQ ID NO29 | SEQ ID NO45 | SEQ ID NO13 | SEQ ID NO12 | SEQ ID NO25 | |
| 208 | SEQ ID NO13 | SEQ ID NO42 | SEQ ID NO14 | SEQ ID NO25 | SEQ ID NO32 | |
| 209 | SEQ ID NO41 | SEQ ID NO42 | SEQ ID NO12 | SEQ ID NO7 | SEQ ID NO25 | |
| 210 | SEQ ID NO41 | SEQ ID NO42 | SEQ ID NO15 | SEQ ID NO25 | SEQ ID NO11 | |
| 211 | SEQ ID NO42 | SEQ ID NO24 | SEQ ID NO25 | SEQ ID NO36 | SEQ ID NO11 | |
| 212 | SEQ ID NO42 | SEQ ID NO14 | SEQ ID NO25 | SEQ ID NO32 | SEQ ID NO11 | |
| 213 | SEQ ID NO42 | SEQ ID NO14 | SEQ ID NO25 | SEQ ID NO36 | SEQ ID NO11 | |
| 214 | SEQ ID NO33 | SEQ ID NO45 | SEQ ID NO17 | SEQ ID NO10 | SEQ ID NO25 | SEQ ID NO11 |
| 215 | SEQ ID NO33 | SEQ ID NO45 | SEQ ID NO17 | SEQ ID NO12 | SEQ ID NO22 | SEQ ID NO25 |
| 216 | SEQ ID NO33 | SEQ ID NO45 | SEQ ID NO17 | SEQ ID NO12 | SEQ ID NO25 | SEQ ID NO11 |
| 217 | SEQ ID NO33 | SEQ ID NO45 | SEQ ID NO42 | SEQ ID NO10 | SEQ ID NO25 | SEQ ID NO11 |
| 218 | SEQ ID NO33 | SEQ ID NO45 | SEQ ID NO42 | SEQ ID NO12 | SEQ ID NO22 | SEQ ID NO25 |
| 219 | SEQ ID NO33 | SEQ ID NO45 | SEQ ID NO42 | SEQ ID NO12 | SEQ ID NO25 | SEQ ID NO11 |
| 220 | SEQ ID NO45 | SEQ ID NO17 | SEQ ID NO41 | SEQ ID NO12 | SEQ ID NO25 | SEQ ID NO11 |
| 221 | SEQ ID NO45 | SEQ ID NO41 | SEQ ID NO42 | SEQ ID NO12 | SEQ ID NO25 | SEQ ID NO11 |
| 222 | SEQ ID NO29 | SEQ ID NO33 | SEQ ID NO45 | SEQ ID NO12 | SEQ ID NO25 | SEQ ID NO11 |

Short nucleic acid sequence elements (ET elements) combinations from table 2 were used to identify *Oryza sativa* terminators putatively enhancing gene expression of a functionally linked gene. A sequence search (Genomatix Genome Analyser; Genomatix Software GmbH, Germany) against plublicly available genome information of *Oryza sativa* (www.phytozome.net) yielded four terminator candidates with putatively enhanced expression. Candidates and control terminators without short enhancing signals are listed in table 3. Frequently used *Agrobacterium tu-mefaciens* terminators in plant biotech, t-OCS (octopine synthase transcriptional terminator and t-nos (nopalin synthase transcriptional terminator (Genbank V00087)) are listed as reference terminators in table 3:

**Table 3: Overview over rice terminators and standard terminators t-OCS and t-nos (SEQ ID NO see table 5**

| **Locus** | | **Locus** |
|---|---|---|
| **putative expression enhancing terminators (*O. sativa*)** | **Standard terminators** | **control terminators ( *O. sativa*)** |
| t-Os05g41900.1 | t-OCS 192bp | t-Os06g47230.1 |
| t-Os03g56790.1 | t-nos 253bp | t-Os02g38920.1 |
| t-Os02g33080.1 | | t-Os12g43600.1 |
| t-Os08g10480.1 | | t-Os02g52290.1 |
| | | t-Os05g33880.1 |
| | | t-Os01g02150.1 |
| | | t-Os10g33660.1 |
| | | t-Os05g42424.1 |
| | | t-Os03g46770.1 |

### Example 2: Verification of completeness of terminator sequence by 3'RACE PCR

RNA was extracted from green tissue of *O*. *sativa* plants with RNAeasy kit (QIAGEN; Hilden, Germany) according to the manufacturer's protocol. cDNA synthesis was performed using the Quantitect Kit (QIAGEN, Hilden, Germany) according to the manufacturer's protocol. With the SMARTer RACE cDNA Amplification Kit from BD Bioscience Clontech (Heidelberg, Germany) 3'RACE analysis was performed using the provided oligo-d(T) and anchor primers as well as respective gene specific primers listed in table 4. The PCR amplicons were cloned with the TOPO TA Cloning Kit from Invitrogen (Carlsbad, CA, USA) and sequenced to determine the position of the poly-adenylation signal. Confirmed 3'UTR lengths are listed in table 4:

**Table 4: Gene specific 3'RACE primers and confirmed 3'UTR lengths**

| **genomic loci of terminators** | **gene specific primer name** | **primer sequence** | **SEQ ID NO** | **3'UTR confirmed by RACE** |
|---|---|---|---|---|
| LOC_Os05g41900 | Loy 1798 | TATACTCGAGGCTGCCTATAGAT- GCTCGTATGCAATATCG | 67 | 225bp |
| LOC_Os03g56790 | Loy 1806 | TATACTCGAGGGCCCTGGCCCT- GATGATCGATCAC | 69 | 303bp |
| LOC_Os02g33080 | Loy 1810 | TATACTCGAGTAAGGTCCACCTTT- GTGGAGTCATCTATCC | 71 | 287bp |
| LOC_Os08g 10480 | Loy 1804 | TATACTCGAGAATGTCATTT- TATCTCCTGTGATATGTAAAGGTT- GA | 73 | 221bp |
| LOC_Os06g47230 | Loy 1790 | TATACTCGAGGGCTGATACCA- ATCTGTAATGCCTGAAAAA | 79 | 129bp |
| LOC_Os02g38920 | Loy 1808 | TATACTCGAGACGAGCCCTCCTCA- TGGA | 81 | 225bp |
| LOC_Os12g43600 | Loy 1794 | TATACTCGAGGCGGTGGGGCCCT- CATGG | 83 | 206bp |
| LOC_Os02g52290 | Loy 1800 | TATACTCGAGACGCATCATG- TAATTCCGGATGGATCTA | 85 | 155bp |
| LOC_Os05g33880 | Loy 1802 | TATACTCGAGATCTAGCTCCATG- GAGAGGATATG | 87 | 271 bp |
| LOC_Os01g02150 | Loy 1814 | TATACTCGAGTCGGCGACGTAT- GGTAATTAATTACACG | 89 | 286bp |
| LOC_Os10g33660 | Loy 1812 | TATACTCGAGAAGAGGGAACTT- CTCTGTAACCCAACATTT | 91 | 194bp |
| LOC_Os05g42424 | Loy 1830 | TATACTCGAGTCATTGATTGATG- GAATTGCTGCTGTACTG | 93 | 181bp |
| LOC_Os03g46770 | Loy 1796 | TATATACATATGTT- GGTGGGGCCCATCGTGG | 95 | 215bp |

### Example 3: Isolation of terminator sequences by Polymerase Chain Reaction

Genomic DNA was extracted from *O.sativa* green tissue using the Qiagen DNeasy Plant Mini Kit (Qiagen, Hilden, Germany). Genomic DNA fragments containing putative expression enhancing terminator sequences were isolated by conventional polymerase chain reaction (PCR). In addition a control group of terminators not containing any expression enhancing signals (compare table 1 and table 2) was selected.
The polymerase chain reaction comprised 15 sets of primers (Table 5). 13 primer sets were designed on the basis of the *O.sativa* genome sequence (www.phytozome.net) comprising the entire 3' UTR to the annotated and confirmed poly-adenylation signal plus ∼300 nt of the down-stream sequence. One primer pair each was designed to amplify the standard terminators t-OCS and t-nos from *Agrobacterium tumefaciens.*

The polymerase chain reaction followed the protocol outlined by Phusion High Fidelity DNA Polymerase (Cat No F-540L, New England Biolabs, Ipswich, MA, USA). The isolated DNA was used as template DNA in a PCR amplification using the following primers:

**Table 5: Primer sequences for terminators**

| **Locus** | **Primer name** | **Sequence** | **SEQ ID NO** | **PCR yielding SEQ ID NO** |
|---|---|---|---|---|
| t-Os05g41900.1 | Loy1798 | TATACTCGAGGCTGCCTATAGATGCTCGTATGCAATATCG | 67 | 1 |
| | Loy1799 | TATAAAGCTTGGGGAGAAAACAATCTTTACATCACATGGA | 68 | |
| t-Os03g56790.1 | Loy1806 | TATACTCGAGGGCCCTGGCCCTGATGATCGATCAC | 69 | 2 |
| | Loy1807 | TATAAAGCTTTTAGGATGGAGGGAGTAGGACAGAATAGCCTGC | 70 | |
| t-Os02g33080.1 | Loy1810 | TATACTCGAGTAAGGTCCACCTTTGTGGAGTCATCTATCC | 71 | 3 |
| | Loy1811 | TATAGAATTCTGTTGTGCACTTTGATGATTTAATTG | 72 | |
| t-Os08g10480.1 | Loy1804 | TATACTCGAGAATGTCATTTTATCTCCTGTGATATGTAAAGGTTGA | 73 | 4 |
| | Loy1805 | TATAAAGCTTGCATCGAAACTTTTTCTTTAATTCTTTGTCGT | 74 | |
| t-OCS 192bp | Loy0001 | TATACTCGAGCCCTGCTTTAATGAGATATGCGAG | 75 | 65 |
| | Loy0002 | TATAAAGCTTGGACAATCAGTAAATTGAACGGAG | 76 | |
| t-nos 253bp | Loy0003 | TATACTCGAGGATCGTTCAAACATTTGGCAATAAAG | 77 | 66 |
| | Loy0004 | TATAAAGCTTGATCTAGTAACATAGATGACACCG | 78 | |
| t-Os06g47230.1 | Loy1790 | TATACTCGAGGGCTGATACCAATCTGTAATGCCTGAAAAA | 79 | 48 |
| | Loy1791 | TATAAAGCTTTCGTTCAATTCGGTTCACAGTATGTTTCTG | 80 | |
| t-Os02g38920.1 | Loy1808 | TATACTCGAGACGAGCCCTCCTCATGGA | 81 | 49 |
| | Loy1809 | TATAAAGCTTATTCTAGAGATAAATCCTCGTGTGC | 82 | |
| t-Os12g43600.1 | Loy1794 | TATACTCGAGGCGGTGGGGCCCTCATGG | 83 | 50 |
| | Loy1795 | TATAAAGCTTTGCTCCAATCTATTTGTACACAGATC | 84 | |
| t-Os02g52290.1 | Loy1800 | TATACTCGAGACGCATCATGTAATTCCGGATGGATCTA | 85 | 51 |
| | Loy1801 | TATAAAGCTTATTGATTTCTGTAACTTTTCGCCGTTTGAA | 86 | |
| t-Os05g33880.1 | Loy1802 | TATACTCGAGATCTAGCTCCATGGAGAGGATATG | 87 | 52 |
| | Loy1803 | TATAAAGCTTCAATTGTCCATACCACTTTGTCAA | 88 | |
| t-Os01g02150.1 | Loy1814 | TATACTCGAGTCGGCGACGTATGGTAATTAATTACACG | 89 | 53 |
| | Loy1815 | TATAAAGCTTAGCGATGGAAGAATCAGAATGGTATAGTCA | 90 | |
| t-Os10g33660.1 | Loy1812 | TATACTCGAGAAGAGGGAACTTCTCTGTAACCCAACATTT | 91 | 54 |
| | Loy1813 | TATAGAATTCAATCTAAACTTTACGCTGAACTAACCACGG | 92 | |
| t-Os05g42424.1 | Loy1830 | TATACTCGAGTCATTGATTGATGGAATTGCTGCTGTACTG | 93 | 55 |
| | Loy1831 | TATAGAATTCAAGGCTTCAAGGCTTCAAACTTCCGA | 94 | |
| t-Os03g46770.1 | Loy1796 | TATATACATATGTTGGTGGGGCCCATCGTGG | 95 | 56 |
| | Toy1797 | TATAAAGCTTTCGACAATCAATCAATGATAAAAATCCTCA | 96 | |

Amplification during the PCR was carried out with the following composition (50 microl):
3.00 microl *O.sativa* genomic DNA (50 ng/microl genomic DNA)
10.00 microl 5x Phusion HF Buffer
4.00 microl dNTP (2.5 mM)
2.50 microl for Primer (10 microM)
2.50 microl rev Primer (10 microM)
0.50 microl Phusion HF DNA Polymerase (2 U/microl)

A touch-down approach was employed for the PCR with the following parameters: 98.0°C for 30 sec (1 cycle), 98.0°C for 30 sec, 56.0°C for 30 sec and 72.0°C for 60 sec (4 cycles), 4 additional cycles each for 54.0°C, 51.0°C and 49.0°C annealing temperature, followed by 20 cycles with 98.0°C for 30 sec, 46.0°C for 30 sec and 72.0°C for 60 sec (4 cycles) and 72.0°C for 5 min. The amplification products was loaded on a 2% (w/v) agarose gel and separated at 80V. The PCR products were excised from the gel and purified with the Qiagen Gel Extraction Kit (Qiagen, Hilden, Germany). Following a DNA restriction digest with *Xhol* (10 U/microl) and *HindIII* (10 U/microl) or *EcoRI* (10 U/microl) or *Smal* (10 U/microl) restriction endonuclease, the digested products were again purified with the Qiagen Gel Extraction Kit (Qiagen, Hilden, Germany).

**Table 6: Overview over restriction digest**

| **Primer name** | **restriction site** | **Primer name** | **restriction site** | **Primer name** | **restriction site** |
|---|---|---|---|---|---|
| Loy1798 | XhoI | Loy0003 | XhoI | Loy1802 | XhoI |
| Loy1799 | HindIII | Loy0004 | HindIII | Loy1803 | HindIII |
| Loy1806 | XhoI | Loy1790 | XhoI | Loy1814 | XhoI |
| Loy1807 | HindIII | Loy1791 | HindIII | Loy1815 | HindIII |
| Loy1810 | XhoI | Loy1808 | XhoI | Loy1812 | XhoI |
| Loy1811 | EcoRI | Loy1809 | HindIII | Loy1813 | EcoRI |
| Loy1804 | XhoI | Loy1794 | XhoI | Loy1830 | XhoI |
| Loy1805 | HindIII | Loy1795 | HindIII | Loy1831 | EcoRI |
| Loy0001 | XhoI | Loy1800 | XhoI | Loy1796 | XhoI |
| Loy0002 | HindIII | Loy1801 | HindIII | Loy1797 | SmaI |

### Example 4: Generation of vector constructs with terminator sequences

Using the Multisite Gateway System (Invitrogen, Carlsbad, CA, USA), the promoter::reporter-gene::terminator cassettes were assembled into binary constructs for plant transformation. The *O.sativa* p-OsGos2 (with the prefix p- denoting promoter) promoter was used in the reporter gene construct, and firefly luciferase (Promega, Madison, WI, USA) was utilized as reporter protein for quantitatively determining the expression enhancing effects of the terminator sequences to be analyzed.

An ENTR/B vector containing the firefly luciferase coding sequence (Promega, Madison, WI, USA) followed by the respective terminators (see above) was generated. Terminator PCR fragments were cloned separately down-stream of the firefly luciferase coding sequence using restriction enzymes indicated in table 6. The resulting pENTR/B vectors are summarized in table 7, with coding sequences having the prefix c-.

**Table 7: All pENTR/B vectors**

| **pENTR/B vector** | **Composition of the partial expression cassette reporter gene::SEQ ID NO** |
|---|---|
| LJK395 | c-LUC::SEQ ID NO1 |
| LJK416 | c-LUC::SEQ ID NO2 |
| LJK438 | c-LUC::SEQ ID NO3 |
| LJK415 | c-LUC::SEQ ID NO4 |
| LJK397 | c-LUC::SEQ ID NO65 |
| LJK2 | c-LUC::SEQ ID NO66 |
| LJK394 | c-LUC::SEQ ID NO48 |
| LJK396 | c-LUC::SEQ ID NO49 |
| LJK412 | c-LUC::SEQ ID NO50 |
| LJK413 | c-LUC::SEQ ID NO51 |
| LJK414 | c-LUC::SEQ ID NO52 |
| LJK417 | c-LUC::SEQ ID NO53 |
| LJK439 | c-LUC::SEQ ID NO54 |
| LJK440 | c-LUC::SEQ ID NO55 |
| LJK437 | c-LUC::SEQ ID NO56 |

By performing a site specific recombination (single site LR-reaction) according to the manufacturers (Invitrogen, Carlsbad, CA, USA) Gateway manual, the pENTR/B containing the partial expression cassette (c-LUC::SEQ ID NO1-NO4, c-LUC::SEQ ID NO65-NO66 and c-LUC::SEQ ID NO48-NO56) was combined with a destination vector (VC-CCP05050-1qcz) harboring the constitutive p-OsGos2 upstream of the recombination site. The reactions yielded binary vectors with the p-OsGos2 promoter, the firefly luciferase coding sequence c-LUC and the respective terminator sequences SEQ ID NO1-NO4, SEQ ID NO65-NO66 and SEQ ID NO48-NO56 down-stream of the firefly luciferase coding sequence (Table 8), for which the combination with SEQ ID NO1 and the control constructs (SEQ ID NO65 and NO66) is given exemplary (SEQ ID NO97, NO98 and NO99, respectively). Except for varying SEQ ID NO2 to NO4 and NO48 to NO56, the nucleotide sequence is identical in all vectors. The resulting plant transformation vectors are summarized in table 8:

**Table 8: Plant expression vectors for O.sativa transformation**

| **plant expression vector** | **Composition of the partial expression cassette** **p-OsGOS2::reporter gene::SEQ ID NO** | **SEQ ID NO** |
|---|---|---|
| LJK428 | p-OsGOS2::c-LUC::SEQ ID NO1 | 97 |
| LJK434 | p-OsGOS2::c-LUC::SEQ ID NO2 | |
| LJK441 | p-OsGOS2::c-LUC::SEQ ID NO3 | |
| LJK433 | p-OsGOS2::c-LUC::SEQ ID NO4 | |
| LJK445 | p-OsGOS2::c-LUC::SEQ ID NO65 | 98 |
| LJK444 | p-OsGOS2::c-LUC::SEQ ID NO66 | 99 |
| LJK427 | p-OsGOS2::c-LUC::SEQ ID NO48 | |
| LJK429 | p-OsGOS2::c-LUC::SEQ ID NO49 | |
| LJK430 | p-OsGOS2::c-LUC::SEQ ID NO50 | |
| LJK431 | p-OsGOS2::c-LUC::SEQ ID NO51 | |
| LJK432 | p-OsGOS2::c-LUC::SEQ ID NO52 | |
| LJK435 | p-OsGOS2::c-LUC::SEQ ID NO53 | |
| LJK442 | p-OsGOS2::c-LUC::SEQ ID NO54 | |
| LJK443 | p-OsGOS2::c-LUC::SEQ ID NO55 | |
| LJK447 | p-OsGOS2::c-LUC::SEQ ID NO56 | |

The resulting vectors LJK428, LJK434, LJK441, LJK433, LJK445, LJK444, LJK427, LJK429, LJK430, LJK431, LJK432, LJK435, LJK442, LJK443 and LJK447 were subsequently used to generate stable transgenic *O.sativa* plants.

### Example 5: Generation of transgenic rice plants

*Agrobacterium* cells containing the respective expression vectors were used to transform *Oryza sativa* plants. Mature dry seeds of the rice japonica cultivar Nipponbare were dehusked. Sterilization was carried out by incubating for one minute in 70% ethanol, followed by 30 minutes in 0.2% HgCl₂, followed by a 6 times 15 minutes wash with sterile distilled water. The sterile seeds were then germinated on a medium containing 2.4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were sub-cultured on fresh medium 3 days before co-cultivation (to boost cell division activity).

*Agrobacterium* strain LBA4404 containing the respective expression vector was used for co-cultivation. *Agrobacterium* was inoculated on AB medium with the appropriate antibiotics and cultured for 3 days at 28°C. The bacteria were then collected and suspended in liquid cocultivation medium to a density (OD₆₀₀) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25°C. Co-cultivated calli were grown on 2.4-D-containing medium for 4 weeks in the dark at 28°C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

Approximately 35 independent T0 rice transformants were generated for one construct. The primary transformants were transferred from a tissue culture chamber to a greenhouse. After a quantitative PCR analysis to verify copy number of the T-DNA insert, only single copy transgenic plants that exhibited tolerance to the selection agent were kept for harvest of T1 seed. Seeds were then harvested three to five months after transplanting. The method yielded single locus transformants at a rate of over 50% (Aldemita and Hodges, 1996, Chan et al., 1993, Hiei et al., 1994).

### Example 6: Plant analysis

Leaf material of adult transgenic *O.sativa* plants was sampled, frozen in liquid nitrogen and subjected to Luciferase reporter gene assays (amended protocol according to Ow et al., 1986). After grinding the frozen tissue samples were resuspended in 800 microl of buffer I (0.1 M Phosphate buffer pH 7.8, 1 mM DTT (Sigma Aldrich, St. Louis, MO, USA), 0.05 % Tween 20 (Sigma Aldrich, St. Louis, MO, USA)) followed by centrifugation at 10 000 g for 10 min. 75 microl of the aqueous supernatant were transferred to 96-well plates. After addition of 25 microl of buffer II (80 mM gycine-glycyl (Carl Roth, Karlsruhe, Germany), 40 mM MgSO₄ (Duchefa, Haarlem, The Netherlands), 60 mM ATP (Sigma Aldrich, St. Louis, MO, USA), pH 7.8) and D-Luciferin to a final concentration of 0.5 mM (Cat No: L-8220, BioSynth, Staad, Switzerland), luminescence was recorded in a MicroLumat Plus LB96V (Berthold Technologies, Bad Wildbad, Germany) yielding the unit relative light unit RLU per minute (RLU/min).

In order to normalize the luciferase activity between samples, the protein concentration was determined in the aqueous supernatant in parallel to the luciferase activity (adapted from Bradford, 1976, Anal. Biochem. 72, 248). 5 microl of the aqueous cell extract in buffer I were mixed with 250 microl of Bradford reagent (Sigma Aldrich, St. Louis, MO, USA), incubated for 10 min at room temperature. Absorption was determined at 595 nm in a plate reader (Thermo Electron Corporation, Multiskan Ascent 354). The total protein amounts in the samples were calculated with a previously generated standard concentration curve. Values resulting from a ratio of RLU/min and mg protein/ml sample were averaged for transgenic plants harboring identical constructs and fold change values were calculated to assess the impact of expression enhancing terminator sequences.

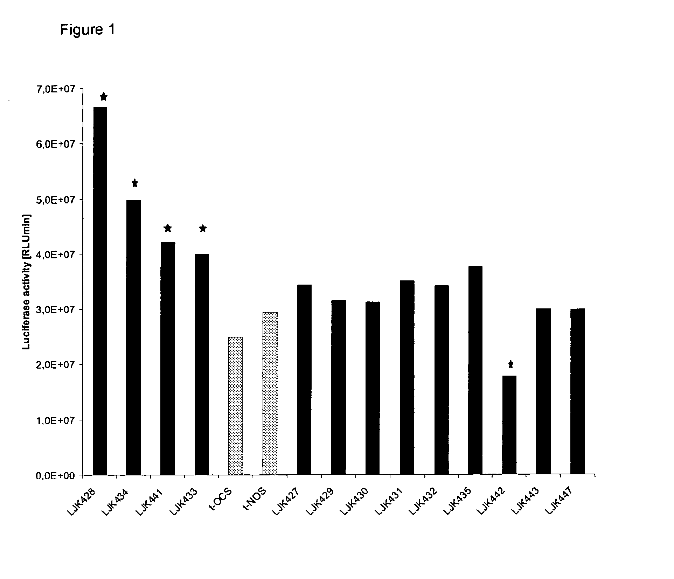

Relative to the reporter gene construct coupled with the t-OCS terminator sequence, LJK428, LJK434, LJK441 and LJK433 showed 2.7-fold, 2.0-fold, 1.7-fold and 1.6-fold higher luciferase activity as a direct indication of expression levels of the luciferase reporter gene (Figure 1). In the isogenic context of the expression construct only the four tested terminator sequences containing the expression enhancing elements (table 1 and 2) caused significantly higher luciferase activity compared to the t-OCS terminator (p<0.0005) (Figure 1).

Similarly, relative to the reporter gene construct coupled with the t-nos terminator sequence, LJK428, LJK434, LJK441 and LJK433 showed 2.3-fold, 1.7-fold, 1.4-fold and 1.4-fold higher luciferase activity as a direct indication of expression levels of the luciferase reporter gene (Figure 1). In the isogenic context of the expression construct only the four tested terminator sequences containing the expression enhancing elements (table 1 and 2) caused significantly higher luciferase activity compared to the t-nos terminator (p<0.005) (Figure 1).

The terminator sequences in constructs LJK428, LJK434, LJK441 and LJK433 can thus serve to enhance expression levels significantly compared to the the standard terminators t-OCS and t-nos. The control terminator sequences from *O.sativa* without any short enhancement elements showed comparable expression to t-OCS and t-nos (Figure 1).

### Example 7: Microarray analysis of expression enhancing terminator sequences in their native context

To test whether SEQ ID NO1 to NO4 positively influenced expression in their native sequence contexts of *O.sativa* transcripts as well, rice plants were treated with transcriptional inhibitors. Transcript stability, assayed by the transcript level after inhibitor treatment, was assessed for the functionally linked transcripts of SEQ ID NO 1-NO4 in their native contexts (LOC_Os05g41900.1, LOC_Os03g56790.1, LOC_Os02g33080.1 and LOC_Os08g10480.1) by microarray analysis (Affymetrix GeneChip Rice (48,564 transcripts); provided by ATLAS-Biotech, Berlin, Germany).

### 7.1 Treatment of transgenic rice plants with transcript inhibitor

Per time point two 10-day old rice plants were shreddered with 40 ml medium (1 mM PIPES, 1mM sodiumcitrate, 1mM KCI, 15 mM sucrose; pH 6.25) for 7 sec in a waring commercial blender to increase the surface for treatment. 75 µg/mL ActinomycinD (Sigma Aldrich, St. Louis, USA) and 200 µg/mL Cordycepin (Sigma Aldrich, St. Louis, USA) were added to the medium and the samples were vacuum-infiltrated (1x100 mbar, no incubation time). The liquid tissue suspensions were incubated under constant agitation (80 rpm). Samples were taken after 0h, 6h, 12h, 24h and 36h.

### 7.2 Microarray analysis and data interpretation

RNA was extracted from the samples with the RNAeasy kit (QIAGEN; Hilden, Germany) and hybridized to the Affymetrix Gene Chip Rice.

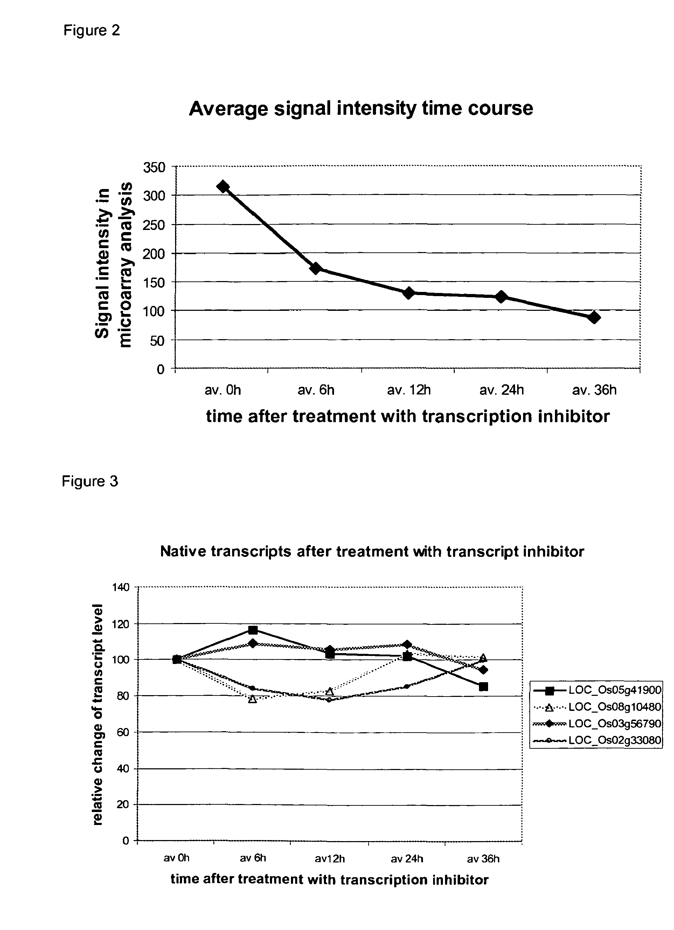

Avaraging over all 48,564 transcripts data analysis showed decreasing transcript levels (∼3-fold) after inhibitor treatment in the time course of 36h (Figure 2). In contrast, transcripts from LOC_Os05g41900.1, LOC_OsO3g56790.1, LOC_Os02g33080.1 and LOC_Os08g10480.1 transcript levels did change less than 20% between 0h and 36h after inhibitor treatment (Figure 3).

The native transcripts functionally coupled with SEQ ID NO1 to NO4 thus show higher than average transcript stability after treatment with transcript inhibitors ActinomycinD and Cordycepin (Figures 2 and 3).

### Example 8: Identification of gene expression enhancing terminators in other monocotyledonous plant species

As described for O.sativa, other monocotyledonous plant species, that is *Zea mays, Sorghum bicolourand Brachypodium distachion,* were screened for expression enhancing terminator sequences with the ET elements listed in tables 1 and 2. Putative expression enhancing terminator sequences (SEQ ID NO57-64) were identified (table 9). Analysis showed that SEQ ID NO57-64 were orthologous to SEQ ID NO1-NO3. The respective ET sequences were thus functionally conserved between monocot species.

**Table 9: Expression enhancing terminator sequences from Zea mays, Brachipodium distachion and Sorghum bicolor, orthologous sequences in O.sativa**

| **Gene locus of monocotyledonous plant** | **Species** | **SEQ ID NO** | **Homologous *O.sativa* gene locus** | **Corresponding SEQ ID from *O.sativa* locus** |
|---|---|---|---|---|
| Bradi2g20920.1 | *Brachipodium distachion* | 63 | LOC_Os05g41900.1 | SEQ ID NO1 |
| Sb09g024530.1 | *Sorghum bicolor* | 62 | | |
| GRMZM2G113414_T01 | *Zea mays* | 64 | | |
| Bradi1g06820.1 | *Brachipodium distachion* | 60 | LOC_Os03g56790.1 | SEQ ID NO2 |
| GRMZM2G130678_T02 | *Zea mays* | 61 | | |
| Bradi3g44960.1 | *Brachipodium distachion* | 58 | LOC_Os02g33080.1 | SEQ ID NO3 |
| Sb04g021790.1 | *Sorghum bicolor* | 57 | | |
| GRMZM2G073950_T01 | *Zea mays* | 59 | | |

Monocotyledonous plant terminator sequences (SEQ ID NO57-64) are analogously cloned and tested in a luciferase reporter gene context as described for *O.sativa* terminator in examples 2 to 6. All tested terminators show increased luciferase activity levels, that is enhanced expression, compared to the standard terminators t-OCS and t-nos.

### Figure legends:

### Figure 1: Luciferase reporter gene assay for O.sativa terminator sequences

Luciferase activity values [RLU/min] averaged for transgenic plants (n>17) harboring identical constructs and fold change values were calculated to assess the impact of expression enhancing terminator sequences.

Grey bars are used for standard terminator construct t-OCS and t-nos. Relative to the reporter gene construct coupled with the t-OCS terminator sequence, LJK428, LJK434, LJK441 and LJK433 showed 2.7-fold, 2.0-fold, 1.7-fold and 1.6-fold higher luciferase activity (p<0.0005) Similarly, relative to the reporter gene construct coupled with the t-nos terminator sequence, LJK428, LJK434, LJK441 and LJK433 showed 2.3-fold, 1.7-fold, 1.4-fold and 1.4-fold higher luciferase activity (p<0.005)

Significantly (p<0.005) distinct expression from the t-nos terminator expression construct is marked by an asterisk. Control *O.sativa* terminator constructs LJK427, LJK429, LJK430, LJK431, LJK432, LJK435, LJK443 and LJK447 show comparable expression levels to the t-nos standard terminator construct. LJK442 has significantly lower luciferase activity levels in the reporter gene context

### Figure 2: Avarage signal intensity of O.sativa transcripts in microarray analysis over time

Average signal intensity for 48,564 transcriptson the Affymetrix Gene Chip Rice is measured after treatment with transcription inhibitor (ActinomycinD and Cordycepin). Samples were taken at 0h, 6h, 12h, 24h, and 36h after inhibitor treatment. Average signal intensities after 36h decrease to 1/3 of the initial intensity before inhibitor treatment. This reflects the average transcript stability of *O.sativa* transcripts.

### Figure 3: Signal intensities of native transcripts coupled to SEQ ID NO1-NO4 terminator sequences in O.sativa; Microarray analysis after treatment with transcription inhibitor over time course of 36h

Signal intensity of the native transcripts coupled to the transcription enhancing terminator sequences (SEQ ID NO1-NO4) are measured over a time course of 36h after treatment with transcription inhibitor. Signal intensity changes are <20% relative to the initial intensity for the four analyzed transcripts. Compared to the average transcript stability (figure2), the transcripts coupled to SEQ ID NO1-NO4 show high stability over a time course of 36h after inhibitor treatment.

**Tabelle 10:**

| **SEQ ID NO5 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | 10 | 11 | 12 | 13 | 14 | 15 | 16 | 17 | 18 | 19 |
| A | 36,36 | 54,55 | 0 | 0 | 0 | 0 | 9,09 | 9,09 | 0 | 45,45 | 18,18 | 0 | 45,45 | 27,27 | 27,27 | 36,36 | 0 | 36,36 | 27,27 |
| c | 18,18 | 18,18 | 54,55 | 100 | 0 | 0 | 72,73 | 90,91 | 90,91 | 9,09 | 18,18 | 54,55 | 9,09 | 9,09 | 9,09 | 9,09 | 9,09 | 0 | 18,18 |
| G | 27,27 | 27,27 | 18,18 | 0 | 0 | 0 | 18,18 | 0 | 0 | 9,09 | 9,09 | 9,09 | 9,09 | 9,09 | 45,45 | 27,27 | 27,27 | 36,36 | 18,18 |
| T | 18,18 | 0 | 27,27 | 0 | 100 | 100 | 0 | 0 | 9,09 | 36,36 | 54,55 | 36,36 | 36,36 | 54,55 | 18,18 | 27,27 | 63,64 | 27,27 | 36,36 |

| Pos | 20 | 21 | 22 | 23 | | | | | | | | | | | | | | | |
|---|---|---|---|---|---|---|---|---|---|---|---|---|---|---|---|---|---|---|---|
| A | 36,36 | 9,09 | 0 | 36,36 | | | | | | | | | | | | | | | |
| C | 27,27 | 36,36 | 72,73 | 36,36 | | | | | | | | | | | | | | | |
| G | 9,09 | 36,36 | 9,09 | 9,09 | | | | | | | | | | | | | | | |
| T | 27,27 | 18,18 | 18,18 | 18,18 | | | | | | | | | | | | | | | |

| **SEQ ID NO6 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | 10 | 11 | 12 | 13 | 14 | 15 | 16 | 17 | 18 | 19 |
| A | 16,67 | 41,67 | 50 | 16,67 | 16,67 | 25 | 0 | 0 | 25 | 75 | 8,33 | 0 | 0 | 16,67 | 41,67 | 33,33 | 16,67 | 16,67 | 0 |
| C | 33,33 | 16,67 | 8,33 | 41,67 | 0 | 25 | 0 | 0 | 0 | 0 | 8,33 | 0 | 0 | 33,33 | 33,33 | 41,67 | 16,67 | 41,67 | 8,33 |
| G | 33,33 | 0 | 25 | 8,33 | 25 | 8,33 | 0 | 100 | 0 | 16,67 | 8,33 | 0 | 0 | 41,67 | 8,33 | 16,67 | 0 | 16,67 | 16,67 |
| T | 16,67 | 41,67 | 16,67 | 33,33 | 58,33 | 41,67 | 100 | 0 | 75 | 8,33 | 75 | 100 | 100 | 8,33 | 16,67 | 8,33 | 66,67 | 25 | 75 |

| Pos | 20 | | | | | | | | | | | | | | | | | | |
|---|---|---|---|---|---|---|---|---|---|---|---|---|---|---|---|---|---|---|---|
| A | 16,67 | | | | | | | | | | | | | | | | | | |
| C | 33,33 | | | | | | | | | | | | | | | | | | |
| G | 16,67 | | | | | | | | | | | | | | | | | | |
| T | 33,33 | | | | | | | | | | | | | | | | | | |

| **SEQ ID NO7 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | 10 | 11 | 12 | 13 | 14 | | | | | |
| A | 0 | 0 | 50 | 50 | 0 | 0 | 0 | 0 | 0 | 0 | 0 | 25 | 0 | 25 | | | | | |
| C | 25 | 25 | 50 | 50 | 0 | 25 | 0 | 0 | 100 | 100 | 50 | 0 | 0 | 25 | | | | | |
| G | 25 | 0 | 0 | 0 | 0 | 0 | 0 | 0 | 0 | 0 | 50 | 0 | 25 | 50 | | | | | |
| T | 50 | 75 | 0 | 0 | 100 | 75 | 100 | 100 | 0 | 0 | 0 | 75 | 75 | 0 | | | | | |

| **SEQ ID NO8 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | 10 | 11 | 12 | 13 | | | | | | |
| A | 25 | 100 | 25 | 75 | 25 | 0 | 0 | 100 | 0 | 0 | 75 | 25 | 25 | | | | | | |
| C | 0 | 0 | 25 | 25 | 0 | 75 | 100 | 0 | 0 | 100 | 0 | 25 | 0 | | | | | | |
| G | 25 | 0 | 50 | 0 | 75 | 25 | 0 | 0 | 0 | 0 | 25 | 50 | 0 | | | | | | |
| T | 50 | 0 | 0 | 0 | 0 | 0 | 0 | 0 | 100 | 0 | 0 | 0 | 75 | | | | | | |

| **SEQ ID NO9 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | 10 | 11 | 12 | 13 | 14 | | | | | |
| A | 25 | 0 | 25 | 25 | 50 | 25 | 0 | 75 | 0 | 0 | 0 | 0 | 100 | 50 | | | | | |
| C | 0 | 100 | 0 | 0 | 0 | 25 | 0 | 0 | 0 | 0 | 0 | 0 | 0 | 25 | | | | | |
| G | 25 | 0 | 0 | 0 | 50 | 25 | 0 | 0 | 0 | 0 | 0 | 50 | 0 | 25 | | | | | |
| T | 50 | 0 | 75 | 75 | 0 | 25 | 100 | 25 | 100 | 100 | 100 | 50 | 0 | 0 | | | | | |

| **SEQ ID NO10 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | 10 | 11 | 12 | 13 | 14 | 15 | 16 | 17 | 18 | 19 |
| A | 41,67 | 58,33 | 50 | 50 | 58,33 | 16,67 | 50 | 0 | 16,67 | 33,33 | 33,33 | 8,33 | 50 | 0 | 0 | 0 | 8,33 | 25 | 0 |
| C | 33,33 | 8,33 | 0 | 25 | 8,33 | 16,67 | 16,67 | 25 | 25 | 33.33 | 0 | 8,33 | 0 | 0 | 0 | 0 | 25 | 0 | 0 |
| G | 16,67 | 25 | 25 | 0 | 8,33 | 8,33 | 25 | 8,33 | 41,67 | 16,67 | 41,67 | 33,33 | 16,67 | 0 | 100 | 100 | 8,33 | 16,67 | 0 |
| T | 8,33 | 8,33 | 25 | 25 | 25 | 58,33 | 8,33 | 66,67 | 16,67 | 16,67 | 25 | 50 | 33,33 | 100 | 0 | 0 | 58,33 | 58,33 | 100 |

| Pos | 20 | 21 | 22 | 23 | 24 | 25 | 26 | | | | | | | | | | | | |
|---|---|---|---|---|---|---|---|---|---|---|---|---|---|---|---|---|---|---|---|
| A | 0 | 16,67 | 25 | 16,67 | 25 | 75 | 25 | | | | | | | | | | | | |
| C | 0 | 8,33 | 33.33 | 41,67 | 8,33 | 0 | 16,67 | | | | | | | | | | | | |
| G | 100 | 8,33 | 0 | 16,67 | 33,33 | 8,33 | 25 | | | | | | | | | | | | |
| T | 0 | 66,67 | 41,67 | 25 | 33,33 | 16,67 | 33,33 | | | | | | | | | | | | |

| **SEQ ID NO11 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | 10 | 11 | 12 | 13 | 14 | 15 | 16 | 17 | 18 | 19 |
| A | 16,67 | 41,67 | 66,67 | 16,67 | 100 | 75 | 8,33 | 0 | 91,67 | 0 | 100 | 41,67 | 16,67 | 41,67 | 25 | 66,67 | 33,33 | 16,67 | 100 |
| C | 33,33 | 8,33 | 8,33 | 25 | 0 | 0 | 8,33 | 0 | 0 | 41,67 | 0 | 25 | 16,67 | 8,33 | 41,67 | 8,33 | 41,67 | 50 | 0 |
| G | 25 | 0 | 0 | 33,33 | 0 | 0 | 0 | 100 | 8,33 | 58,33 | 0 | 16,67 | 33,33 | 33,33 | 8,33 | 25 | 0 | 8,33 | 0 |
| T | 25 | 50 | 25 | 25 | 0 | 25 | 83,33 | 0 | 0 | 0 | | 16.67 | 33,33 | 16,67 | 25 | 0 | 25 | 25 | 0 |

| Pos | 20 | | | | | | | | | | | | | | | | | | |
|---|---|---|---|---|---|---|---|---|---|---|---|---|---|---|---|---|---|---|---|
| A | 41,67 | | | | | | | | | | | | | | | | | | |
| | 25 | | | | | | | | | | | | | | | | | | |
| G | 16,67 | | | | | | | | | | | | | | | | | | |
| T | 16,67 | | | | | | | | | | | | | | | | | | |

| **SEQ ID NO12 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | | | | | | | | | | |
| A | 16,67 | 75 | 100 | 100 | 75 | 0 | 0 | 100 | 33,33 | | | | | | | | | | |
| C | 16,67 | 0 | 0 | 0 | 16,67 | 0 | 0 | 0 | 16.67 | | | | | | | | | | |
| G | 33,33 | 25 | 0 | 0 | 8,33 | 100 | 25 | 0 | 16,67 | | | | | | | | | | |
| T | 33,33 | 0 | 0 | 0 | 0 | 0 | 75 | 0 | 33,33 | | | | | | | | | | |

| **SEQ ID NO13 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | 10 | 11 | 12 | 13 | 14 | | | | | |
| A | 50 | 16,67 | 50 | 8,33 | 16,67 | 8,33 | 0 | 0 | 66,67 | 100 | 8,33 | 0 | 66,67 | 16,67 | | | | | |
| C | 8,33 | 0 | 8,33 | 25 | 25 | 16,67 | 0 | 0 | 16,67 | 0 | 25 | 100 | 16,67 | 25 | | | | | |
| G | 0 | 33,33 | 0 | 33,33 | 25 | 16,67 | 100 | 100 | 16,67 | 0 | 66,67 | 0 | 0 | 0 | | | | | |
| T | 41,67 | 50 | 41,67 | 33,33 | 33,33 | 58,33 | 0 | 0 | 0 | 0 | 0 | 0 | 16,67 | 58,33 | | | | | |

| **SEQ ID NO14 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | 10 | 11 | 12 | 13 | 14 | 15 | 16 | 17 | | |
| A | 8,33 | 50 | 25 | 25 | 33,33 | 25 | 25 | 41,67 | 33,33 | 0 | 0 | 33,33 | 0 | 91,67 | 0 | 0 | 25 | | |
| C | 50 | 0 | 8,33 | 41,67 | 8,33 | 50 | 8,33 | 8,33 | 16,67 | 0 | 0 | 16,67 | 25 | 0 | 0 | 0 | 16,67 | | |
| G | 16,67 | 0 | 16,67 | 16,67 | 33,33 | 0 | 41,67 | 8,33 | 41,67 | 0 | 100 | 41,67 | 0 | 8,33 | 0 | 0 | 25 | | |
| T | 25 | 50 | 50 | 16,67 | 25 | 25 | 25 | 41,67 | 8,33 | 100 | 0 | 8,33 | 75 | 0 | 100 | 100 | 33,33 | | |

| **SEQ ID NO15 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | 10 | 11 | 12 | 13 | 14 | 15 | 16 | 17 | 18 | 19 |
| A | 41,67 | 8,33 | 25 | 25 | 25 | 50 | 50 | 16,67 | 50 | 50 | 8,33 | 33,33 | 100 | 100 | 83,33 | 8,33 | 25 | 0 | 100 |
| C | 8,33 | 50 | 33,33 | 33,33 | 41,67 | 25 | 16,67 | 25 | 33,33 | 8,33 | 33,33 | 33,33 | 0 | 0 | 0 | 75 | 0 | 83,33 | 0 |
| G | 8,33 | 0 | 8,33 | 8,33 | 16,67 | 0 | 8,33 | 41,67 | 16,67 | 16,67 | 58,33 | 16,67 | 0 | 0 | 16,67 | 0 | 0 | 8,33 | 0 |
| T | 41,67 | 41,67 | 33,33 | 33,33 | 16,67 | 25 | 25 | 16,67 | 0 | 25 | 0 | 16,67 | 0 | 0 | 0 | 16,67 | 75 | 8,33 | 0 |

| Pos | 20 | 21 | 22 | 23 | | | | | | | | | | | | | | | |
|---|---|---|---|---|---|---|---|---|---|---|---|---|---|---|---|---|---|---|---|
| A | 33,33 | 33,33 | 58,33 | 25 | | | | | | | | | | | | | | | |
| C | 8,33 | 25 | 25 | 16,67 | | | | | | | | | | | | | | | |
| G | 33,33 | 41,67 | 16,67 | 8,33 | | | | | | | | | | | | | | | |
| T | 25 | 0 | 0 | 50 | | | | | | | | | | | | | | | |

| **SEQ ID NO16 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | 10 | 11 | 12 | 13 | 14 | 15 | 16 | 17 | 18 | 19 |
| A | 16,67 | 50 | 50 | 8,33 | 41,67 | 8,33 | 16,67 | 33,33 | 25 | 25 | 25 | 58,33 | 0 | 0 | 91,67 | 0 | 25 | 41,67 | 8,33 |
| C | 16,67 | 0 | 16,67 | 8,33 | 16,67 | 33,33 | 0 | 41,67 | 50 | 16,67 | 41,67 | 16,67 | 0 | 0 | 0 | 0 | 0 | 50 | 0 |
| G | 50 | 8,33 | 0 | 41,67 | 0 | 16,67 | 16,67 | 16,67 | 8,33 | 0 | 0 | 16,67 | 0 | 0 | 0 | 0 | 75 | 0 | 0 |
| T | 16,67 | 41,67 | 33,33 | 41,67 | 41,67 | 41,67 | 66,67 | 8,33 | 16,67 | 58,33 | 33,33 | 8,33 | 100 | 100 | 8,33 | 100 | 0 | 8,33 | 91,67 |

| Pos | 20 | | | | | | | | | | | | | | | | | | |
|---|---|---|---|---|---|---|---|---|---|---|---|---|---|---|---|---|---|---|---|
| A | 25 | | | | | | | | | | | | | | | | | | |
| C | 41,67 | | | | | | | | | | | | | | | | | | |
| G | 25 | | | | | | | | | | | | | | | | | | |
| T | 8,33 | | | | | | | | | | | | | | | | | | |

| **SEQ ID NO17 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | 10 | 11 | 12 | | | | | | | |
| A | 8,33 | 0 | 75 | 0 | 0 | 0 | 8,33 | 16,67 | 16,67 | 0 | 66,67 | 33,33 | | | | | | | |
| C | 33,33 | 0 | 0 | 0 | 0 | 16,67 | 91,67 | 16,67 | 25 | 0 | 16,67 | 16,67 | | | | | | | |
| G | 8,33 | 100 | 8,33 | 0 | 100 | 83,33 | 0 | 16,67 | 8,33 | 0 | 0 | 25 | | | | | | | |
| T | 50 | 0 | 16,67 | 100 | 0 | 0 | 0 | 50 | 50 | 100 | 16,67 | 25 | | | | | | | |

| **SEQ ID NO18 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | 10 | 11 | 12 | 13 | 14 | 15 | 16 | 17 | 18 | 19 |
| A | 0 | 30 | 40 | 30 | 0 | 0 | 0 | 0 | 0 | 0 | 0 | 10 | 0 | 20 | 50 | 0 | 40 | 40 | 30 |
| C | 40 | 40 | 30 | 10 | 0 | 0 | 0 | 0 | 0 | 0 | 90 | 30 | 10 | 40 | 20 | 0 | 30 | 40 | 20 |
| G | 10 | 0 | 10 | 20 | 0 | 10 | 100 | 0 | 60 | 0 | 0 | 30 | 40 | 20 | 20 | 40 | 10 | 20 | 30 |
| T | 50 | 30 | 20 | 40 | 100 | 90 | 0 | 100 | 40 | 100 | 10 | 30 | 50 | 20 | 10 | 60 | 20 | 0 | 20 |

| Pos | 20 | 21 | 22 | 23 | | | | | | | | | | | | | | | |
|---|---|---|---|---|---|---|---|---|---|---|---|---|---|---|---|---|---|---|---|
| A | 40 | 20 | 30 | 40 | | | | | | | | | | | | | | | |
| C | 10 | 30 | 40 | 40 | | | | | | | | | | | | | | | |
| G | 30 | 10 | 30 | 10 | | | | | | | | | | | | | | | |
| T | 20 | 40 | 0 | 10 | | | | | | | | | | | | | | | |

| **SEQ ID NO19 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | 10 | 11 | 12 | 13 | 14 | 15 | 16 | 17 | 18 | 19 |
| A | 25 | 33,33 | 16,67 | 8,33 | 8,33 | 16,67 | 41,67 | 0 | 8,33 | 0 | 0 | 8,33 | 0 | 0 | 0 | 0 | 41,67 | 41,67 | 8,33 |
| C | 33,33 | 25 | 33,33 | 8,33 | 41,67 | 50 | 8,33 | 8,33 | 0 | 0 | 100 | 0 | 0 | 0 | 16,67 | 16,67 | 16,67 | 0 | 16,67 |
| G | 33,33 | 0 | 8,33 | 25 | 8,33 | 16,67 | 16,67 | 58,33 | 0 | 100 | 0 | 0 | 0 | 8,33 | 41,67 | 0 | 25 | 33,33 | 25 |
| T | 8,33 | 41,67 | 41,67 | 58,33 | 41,67 | 16,67 | 33,33 | 33,33 | 91,67 | 0 | 0 | 91,67 | 100 | 91,67 | 41,67 | 83,33 | 16,67 | 25 | 50 |

| **SEQ ID NO20 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | 10 | 11 | 12 | 13 | 14 | 15 | 16 | | | |
| A | 71,43 | 14,29 | 0 | | 0 | 0 | 0 | 0 | 0 | 0 | 0 | 28,57 | 28,57 | 57,14 | 71,43 | 0 | | | |
| C | 0 | 0 | 0 | 0 | 0 | 0 | 71,43 | 100 | 0 | 28,57 | 42,86 | 0 | 28,57 | 42,86 | 14,29 | 42,86 | | | |
| G | 14,29 | 14,29 | 57,14 | 0 | 0 | 0 | 28,57 | 0 | 0 | 71,43 | 0 | 28,57 | 14,29 | 0 | 14,29 | 14,29 | | | |
| T | 14,29 | 71,43 | 42,86 | 100 | 100 | 100 | 0 | 0 | 100 | 0 | 57,14 | 42,86 | 28,57 | 0 | 0 | 42,86 | | | |

| **SEQ ID NO21 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | 10 | 11 | 12 | 13 | 14 | 15 | 16 | 17 | 18 | |
| A | 40 | 50 | 0 | | 100 | 0 | 0 | 0 | 0 | 10 | 50 | 40 | 50 | 40 | 30 | 0 | 20 | 60 | |
| C | 20 | 30 | 60 | 0 | 0 | 0 | 0 | 0 | 0 | 30 | 30 | 20 | 0 | 10 | 20 | 20 | 0 | 10 | |
| G | 0 | 0 | 20 | 0 | 0 | 0 | 10 | 100 | 0 | 30 | 20 | 0 | 20 | 0 | 10 | 50 | 10 | 10 | |
| T | 40 | 20 | 20 | 100 | 0 | 100 | 90 | 0 | 100 | 30 | 0 | 40 | 30 | 50 | 40 | 30 | 70 | 20 | |

| **SEQ ID NO22 MATRIX:** | | | | | | | | | | | | | | | | | | | |
|---|---|---|---|---|---|---|---|---|---|---|---|---|---|---|---|---|---|---|---|
| Pos | 1 | 2 | 3 | 14 | 5 | 6 | 7 | 8 | 9 | 10 | 11 | 12 | 13 | 14 | 15 | 16 | 17 | 18 | |
| A | 22,22 | 0 | 0 | 0 | 0 | 11,11 | 11,11 | 0 | 22,22 | 44,44 | 0 | 11,11 | 22,22 | 22,22 | 11,11 | 0 | 55,56 | 0 | |
| C | 11,11 | 0 | 0 | 0 | 100 | 0 | 77,78 | 55,56 | 11,11 | 0 | 66,67 | 55,56 | 0 | 55,56 | 22,22 | 44,44 | 44,44 | 44,44 | |
| G | 11,11 | 0 | 0 | 0 | 0 | 0 | 11,11 | 0 | 0 | 22,22 | 11,11 | 0 | 22,22 | 0 | 11,11 | 33,33 | 0 | 22,22 | |
| T | 55,56 | 100 | 100 | 100 | 0 | 88,89 | 0 | 44,44 | 66,67 | 33,33 | 22,22 | 33,33 | 55,56 | 22,22 | 55,56 | 22,22 | 0 | 33,33 | |

| **SEQ ID NO23 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | 10 | | | | | | | | | |
| A | 0 | 20 | 0 | 0 | 40 | 10 | 0 | 0 | 0 | 40 | | | | | | | | | |
| C | 10 | 0 | 0 | 0 | 10 | 0 | 0 | 100 | 50 | 30 | | | | | | | | | |
| G | 30 | 10 | 0 | 100 | 40 | 0 | 0 | 0 | 0 | 10 | | | | | | | | | |
| T | 60 | 70 | 100 | 0 | 10 | 90 | 100 | 0 | 50 | 20 | | | | | | | | | |

| **SEQ ID NO24 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | 10 | 11 | 12 | 13 | 14 | 15 | 16 | 17 | | |
| A | 11,11 | 0 | 100 | 0 | 77,78 | 0 | 0 | 0 | 11,11 | 22,22 | 33,33 | 55,56 | 55,56 | 11,11 | 33,33 | 44,44 | 33,33 | | |
| C | 11,11 | 0 | 0 | 0 | 0 | 0 | 0 | 0 | 44,44 | 11,11 | 0 | 0 | 11,11 | 33,33 | 0 | 11,11 | 0 | | |
| G | 22,22 | 44,44 | 0 | 0 | 0 | 0 | 0 | 100 | 0 | 22,22 | 33,33 | 11,11 | 11,11 | 0 | 11,11 | 0 | 44,44 | | |
| T | 55,56 | 55,56 | 0 | 100 | 22,22 | 100 | 100 | 0 | 44,44 | 44,44 | 33,33 | 33,33 | 22,22 | 55,56 | 55,56 | 44,44 | 22,22 | | |

| **SEQ ID NO25 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | 10 | 11 | 12 | | | | | | | |
| A | 33,33 | 11,11 | 33,33 | 33,33 | 77,78 | 100 | 0 | 22,22 | 66,67 | 0 | 100 | 44,44 | | | | | | | |
| C | 22,22 | 55,56 | 22,22 | 11,11 | 22,22 | 0 | 100 | 11,11 | 0 | 0 | 0 | 11,11 | | | | | | | |
| G | 0 | 33,33 | 22,22 | 0 | 0 | 0 | 0 | 0 | 0 | 100 | 0 | 11,11 | | | | | | | |
| T | 44,44 | 0 | 22,22 | 55,56 | 0 | 0 | 0 | 66,67 | 33,33 | 0 | 0 | 33,33 | | | | | | | |

| **SEQ ID NO26 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | 10 | 11 | 12 | | | | | | | |
| A | 12,5 | 62,5 | 12,5 | 25 | 12,5 | 0 | 100 | 0 | 0 | 37,5 | 12,5 | 37,5 | | | | | | | |
| C | 25 | 0 | 12,5 | 37,5 | 0 | | 0 | 0 | 0 | 12,5 | 0 | 50 | | | | | | | |
| G | 25 | 0 | 0 | 25 | 0 | 0 | 0 | 0 | 100 | 25 | 25 | 0 | | | | | | | |
| T | 37,5 | 37,5 | 75 | 12,5 | 87,5 | 100 | 0 | 100 | 0 | 25 | 62,5 | 12,5 | | | | | | | |

| **SEQ ID NO27 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | 10 | 11 | 12 | 13 | | | | | | |
| A | 42,86 | 28,57 | 42,86 | 42,86 | 0 | 0 | 0 | 85,71 | 100 | 0 | 85,71 | 85,71 | 0 | | | | | | |
| C | 14,29 | 57,14 | 14,29 | 14,29 | 28,57 | 14,29 | 71,43 | 0 | 0 | 0 | 0 | 0 | 42,86 | | | | | | |
| G | 0 | 14,29 | 0 | 0 | 28,57 | 0 | | 14,29 | 0 | 0 | 14,29 | 0 | 28,57 | | | | | | |
| T | 42,86 | 0 | 42,86 | 42,86 | 42,86 | 85.71 | 28,57 | 0 | 0 | 100 | 0 | 14,29 | 28,57 | | | | | | |

| **SEQ ID NO28 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | 10 | 11 | 12 | 13 | 14 | 15 | 16 | 17 | | |
| A | 9,09 | 0 | 0 | 63,64 | 36,36 | 0 | 27,27 | 0 | 0 | 0 | 9,09 | 0 | 0 | 0 | 18,18 | 72,73 | 63,64 | | |
| C | 36,36 | 27,27 | 54,55 | 0 | 9,09 | 18,18 | 0 | 18,18 | 0 | 100 | 36,36 | 0 | 0 | 0 | 0 | 18,18 | 9,09 | | |
| G | 18,18 | 9,09 | 9,09 | 18,18 | 27,27 | 54,55 | 36,36 | 9,09 | 0 | 0 | 0 | 0 | 100 | 0 | 81,82 | 0 | 9,09 | | |
| T | 36,36 | 63,64 | 36,36 | 18,18 | 27,27 | 27,27 | 36,36 | 72,73 | 100 | 0 | 54,55 | 100 | 0 | 100 | 0 | 9,09 | 18,18 | | |

| **SEQ ID NO29 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | 10 | 11 | 12 | 13 | 14 | 15 | 16 | | | |
| A | 0 | 18,18 | 27,27 | 45,45 | 45,45 | 0 | 9,09 | 27,27 | 0 | 0 | 0 | 18,18 | 0 | 0 | 0 | 27,27 | | | |
| C | 18,18 | 18,18 | 36,36 | 18,18 | 9,09 | 18,18 | 18,18 | 9,09 | 0 | 0 | 0 | 0 | 9,09 | 72,73 | 0 | 36,36 | | | |
| G | 18,18 | 45,45 | 18,18 | 36.36 | 9,09 | 54,55 | 36,36 | 36,36 | 100 | 0 | 100 | 9,09 | 0 | 18,18 | 0 | 18,18 | | | |
| T | 63,64 | 18,18 | 18,18 | 0 | 36,36 | 27,27 | 36,36 | 27,27 | 0 | 100 | 0 | 72,73 | 90,91 | 9,09 | 100 | 18,18 | | | |

| **SEQ ID NO30 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | 10 | 11 | | | | | | | | |
| A | 10 | 0 | 90 | 0 | 0 | 0 | 0 | 10 | 50 | 30 | 40 | | | | | | | | |
| C | 40 | 0 | 10 | 0 | 0 | 0 | 0 | 50 | 20 | 30 | 0 | | | | | | | | |
| G | 30 | 0 | 0 | 0 | 0 | 100 | 0 | 40 | 30 | 0 | 30 | | | | | | | | |
| T | 20 | 100 | 0 | 100 | 100 | 0 | 100 | 0 | 0 | 40 | 30 | | | | | | | | |

| **SEQ ID NO31 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | 10 | | | | | | | | | |
| A | 36,36 | 45,45 | 0 | 0 | 0 | 18,18 | 9,09 | 0 | 0 | 9,09 | | | | | | | | | |
| C | 18,18 | 0 | 0 | 0 | 0 | 0 | 18,18 | 0 | 100 | 36,36 | | | | | | | | | |
| G | 18,18 | 0 | 0 | 0 | 100 | 0 | 18,18 | 9,09 | 0 | 27,27 | | | | | | | | | |
| T | 27,27 | 54,55 | 100 | 100 | 0 | 81,82 | 54,55 | 90,91 | 0 | 27,27 | | | | | | | | | |

| **SEQ ID NO32 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | 10 | 11 | 12 | 13 | 14 | 15 | 16 | 17 | | |
| A | 18,18 | 54,55 | 18,18 | 27,27 | 45,45 | 0 | 0 | 0 | 0 | 0 | 0 | 9,09 | 36,36 | 27,27 | 27,27 | 45,45 | 36,36 | | |
| C | 9,09 | 0 | 27,27 | 18,18 | 45,45 | 90,91 | 100 | 0 | 18,18 | 9,09 | 100 | 72,73 | 27,27 | 18,18 | 45,45 | 18,18 | 18,18 | | |
| G | 27,27 | 0 | 27,27 | 36,36 | 0 | 0 | 0 | 0 | 27,27 | 0 | 0 | 9,09 | 18,18 | 18,18 | 18,18 | 36,36 | 9,09 | | |
| T | 45,45 | 45,45 | 27,27 | 18,18 | 9,09 | 9,09 | 0 | 100 | 54,55 | 90,91 | 0 | 9,09 | 18,18 | 36,36 | 9,09 | 0 | 36,36 | | |

| **SEQ ID NO33 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | 10 | 11 | 12 | 13 | 14 | 15 | 16 | 17 | 18 | 19 |
| A | 37,5 | 25 | 12,5 | 62,5 | 0 | 0 | 12,5 | 25 | 87,5 | 50 | 25 | 37,5 | 0 | 0 | 100 | 12,5 | 0 | 100 | 100 |
| C | 25 | 0 | 25 | 0 | 50 | 37,5 | 0 | 12,5 | 0 | 0 | 0 | 0 | 12,5 | 100 | 0 | 87,5 | 87,5 | 0 | 0 |
| G | 25 | 0 | 0 | 25 | 37,5 | 0 | 37,5 | 12,5 | 0 | 0 | 12,5 | 25 | 50 | 0 | 0 | 0 | 0 | 0 | 0 |
| T | 12,5 | 75 | 62,5 | 12,5 | 12,5 | 62,5 | 50 | 50 | 12,5 | 50 | 62,5 | 37,5 | 37,5 | 0 | 0 | 0 | 12,5 | 0 | 0 |

| Pos | 20 | | | | | | | | | | | | | | | | | | |
|---|---|---|---|---|---|---|---|---|---|---|---|---|---|---|---|---|---|---|---|
| A | 50 | | | | | | | | | | | | | | | | | | |
| c | 12,5 | | | | | | | | | | | | | | | | | | |
| G | 12,5 | | | | | | | | | | | | | | | | | | |
| T | 25 | | | | | | | | | | | | | | | | | | |

| **SEQ ID NO34 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | 10 | 11 | 12 | 13 | 14 | 15 | 16 | 17 | | |
| A | 20 | 30 | 0 | 0 | 100 | 20 | 0 | 0 | 10 | 30 | 60 | 30 | 20 | 30 | 30 | 30 | 30 | | |
| C | 10 | 20 | 0 | 0 | 0 | 60 | 0 | 0 | 50 | 30 | 0 | 10 | 40 | 30 | 20 | 10 | 30 | | |
| G | 50 | 40 | 0 | 0 | 0 | 0 | 0 | 90 | 30 | 30 | 0 | 20 | 20 | 10 | 10 | 60 | 10 | | |
| T | 20 | 10 | 100 | 100 | 0 | 20 | 100 | 10 | 10 | 10 | 40 | 40 | 20 | 30 | 40 | 0 | 30 | | |

| **SEQ ID NO35 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | 10 | 11 | 12 | 13 | 14 | 15 | 16 | | | |
| A | 22,22 | 55,56 | 55,56 | 55,56 | 44,44 | 0 | 22,22 | 100 | 100 | 0 | 77,78 | 66,67 | 22,22 | 11,11 | 55,56 | 11,11 | | | |
| C | 33,33 | 0 | 0 | 0 | 22,22 | 0 | 0 | 0 | 0 | 0 | 22,22 | 11,11 | 22,22 | 0 | 0 | 0 | | | |
| G | 11,11 | 44,44 | 33,33 | 11,11 | 11,11 | 0 | 0 | 0 | 0 | 0 | 0 | 0 | 33,33 | 44,44 | 0 | 22,22 | | | |
| T | 33,33 | 0 | 11,11 | 33,33 | 22,22 | 100 | 77,78 | 0 | 0 | 100 | 0 | 22,22 | 22,22 | 44,44 | 44,44 | 66,67 | | | |

| **SEQ ID NO36 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | 10 | 11 | | | | | | | | |
| A | 11,11 | 11,11 | 22,22 | 22,22 | 0 | 0 | 0 | 22,22 | 11,11 | 100 | 22,22 | | | | | | | | |
| C | 33,33 | 0 | 11,11 | 11,11 | 100 | 0 | 0 | 22,22 | 0 | 0 | 11,11 | | | | | | | | |
| G | 33,33 | 0 | 33,33 | 11,11 | 0 | 0 | 100 | 44,44 | 11,11 | 0 | 22,22 | | | | | | | | |
| T | 22,22 | 88,89 | 33,33 | 55,56 | 0 | 100 | 0 | 11,11 | 77,78 | 0 | 44,44 | | | | | | | | |

| **SEQ ID NO37 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | 10 | 11 | 12 | 13 | 14 | 15 | 16 | 17 | 18 | 19 |
| A | 33,33 | 16,67 | 16,67 | 25 | 33,33 | 25 | 0 | 0 | 0 | 25 | 0 | 0 | 0 | 8,33 | 33,33 | 41,67 | 41,67 | 0 | 16,67 |
| C | 25 | 50 | 50 | 25 | 25 | 8,33 | 0 | 0 | 0 | 0 | 0 | 8,33 | 100 | 41,67 | 25 | 33,33 | 0 | 0 | 33,33 |
| G | 33,33 | 25 | 8,33 | 0 | 16,67 | 25 | 0 | 0 | 100 | 0 | 25 | 8,33 | 0 | 16,67 | 25 | 0 | 16,67 | 41,67 | 16,67 |
| T | 8,33 | 8,33 | 25 | 50 | 25 | 41,67 | 100 | 100 | 0 | 75 | 75 | 83,33 | 0 | 33,33 | 16,67 | 25 | 41,67 | 58,33 | 33,33 |

| Pos | 20 | 21 | | | | | | | | | | | | | | | | | |
|---|---|---|---|---|---|---|---|---|---|---|---|---|---|---|---|---|---|---|---|
| A | 50 | 25 | | | | | | | | | | | | | | | | | |
| C | 33,33 | 33,33 | | | | | | | | | | | | | | | | | |
| G | 16,67 | 16,67 | | | | | | | | | | | | | | | | | |
| T | 0 | 25 | | | | | | | | | | | | | | | | | |

| **SEQ ID NO38 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | 10 | 11 | 12 | 13 | 14 | 15 | 16 | 17 | | |
| A | 9,09 | 0 | 0 | 63,64 | 36,36 | 0 | 27,27 | 0 | 0 | 0 | 9,09 | 0 | 0 | 0 | 18,18 | 72,73 | 63,64 | | |
| C | 36,36 | 27,27 | 54,55 | 0 | 9,09 | 18,18 | 0 | 18,18 | 0 | 100 | 36,36 | 0 | 0 | 0 | 0 | 18,18 | 9,09 | | |
| G | 18,18 | 9,09 | 9,09 | 18,18 | 27,27 | 54,55 | 36,36 | 9,09 | 0 | 0 | 0 | 0 | 100 | 0 | 81,82 | 0 | 9,09 | | |
| T | 36,36 | 63,64 | 36,36 | 18,18 | 27,27 | 27,27 | 36,36 | 72,73 | 100 | 0 | 54,55 | 100 | 0 | 100 | 0 | 9,09 | 18,18 | | |

| **SEQ ID NO39 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | 10 | 11 | 12 | 13 | | | | | | |
| A | 0 | 0 | 16,67 | 16,67 | 33,33 | 0 | 0 | 16,67 | 0 | 0 | 0 | 0 | 25 | | | | | | |
| C | 41,67 | 0 | 16,67 | 25 | 16,67 | 0 | 8,33 | 0 | 75 | 0 | 0 | 0 | 16,67 | | | | | | |
| G | 16,67 | 41,67 | 8,33 | 33,33 | 16,67 | 0 | 16,67 | 83,33 | 25 | 0 | 0 | 0 | 33,33 | | | | | | |
| T | 41,67 | 58,33 | 58,33 | 25 | 33,33 | 100 | 75 | 0 | 0 | 100 | 100 | 100 | 25 | | | | | | |

| **SEQ ID NO40 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | 10 | 11 | 12 | 13 | 14 | 15 | | | | |
| A | 33,33 | 0 | 0 | 8,33 | 75 | 0 | 33,33 | 0 | 0 | 16,67 | 0 | 33,33 | 33,33 | 0 | 41,67 | | | | |
| C | 16,67 | 0 | 0 | 16,67 | 0 | 0 | 0 | 0 | 0 | 25 | 58,33 | 8,33 | 25 | 50 | 16,67 | | | | |
| G | 8,33 | 100 | 0 | 75 | 0 | 0 | 0 | 0 | 16,67 | 25 | 16,67 | 0 | 25 | 33,33 | 8,33 | | | | |
| T | 41,67 | 0 | 100 | 0 | 25 | 100 | 66,67 | 100 | 83,33 | 33,33 | 25 | 58,33 | 16,67 | 16,67 | 33,33 | | | | |

| **SEQ ID NO41 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | 10 | 11 | 12 | 13 | 14 | 15 | 16 | 17 | 18 | 19 |
| A | 27,27 | 45,45 | 36,36 | 9,09 | 54,55 | 18,18 | 36,36 | 9,09 | 0 | 0 | 27,27 | 0 | 54,55 | 27,27 | 27,27 | 9,09 | 0 | 0 | 0 |
| C | 54,55 | 9,09 | 18,18 | 54,55 | 0 | 27,27 | 9,09 | 45,45 | 36,36 | 18,18 | 9,09 | 27,27 | 0 | 36,36 | 0 | 0 | 100 | 0 | 0 |
| G | 9,09 | 0 | 18,18 | 27,27 | 27,27 | 18,18 | 18,18 | 27,27 | 36,36 | 9,09 | 27,27 | 36,36 | 27,27 | 0 | 72,73 | 72,73 | 0 | 0 | 0 |
| T | 9,09 | 45,45 | 27,27 | 9,09 | 18,18 | 36,36 | 36,36 | 18,18 | 27,27 | 72,73 | 36,36 | 36,36 | 18,18 | 36,36 | 0 | 18,18 | 0 | 100 | 100 |

| Pos | 20 | 21 | 22 | 23 | | | | | | | | | | | | | | | |
|---|---|---|---|---|---|---|---|---|---|---|---|---|---|---|---|---|---|---|---|
| A | 0 | 0 | 45,45 | 9,09 | | | | | | | | | | | | | | | |
| C | 9,09 | 0 | 18,18 | 45,45 | | | | | | | | | | | | | | | |
| G | 81,82 | 0 | 0 | 18,18 | | | | | | | | | | | | | | | |
| T | 9,09 | 100 | 36,36 | 27,27 | | | | | | | | | | | | | | | |

| **SEQ ID NO42 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | 10 | 11 | 12 | 13 | 14 | 15 | 16 | 17 | 18 | 19 |
| A | 25 | 16,67 | 0 | 0 | 0 | 0 | 8,33 | 0 | 0 | 41,67 | 41,67 | 8,33 | 58,33 | 33,33 | 8,33 | 50 | 66,67 | 58,33 | 8,33 |
| C | 8,33 | 50 | 100 | 0 | 0 | 8,33 | 8,33 | 50 | 100 | 25 | 16,67 | 25 | 8,33 | 25 | 58,33 | 8,33 | 0 | 16,67 | 33,33 |
| G | 25 | 0 | 0 | 0 | 8,33 | 8,33 | 8,33 | 50 | 0 | 8,33 | 8,33 | 50 | 25 | 16,67 | 0 | 0 | 8,33 | 16,67 | 41,67 |
| T | 41,67 | 33,33 | 0 | 100 | 91,67 | 83,33 | 75 | 0 | 0 | 25 | 33,33 | 16,67 | 8,33 | 25 | 33,33 | 41,67 | 25 | 8,33 | 16,67 |

| **SEQ ID NO43 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | 10 | | | | | | | | | |
| A | 40 | 60 | 0 | 0 | 0 | 0 | 90 | 0 | 20 | 50 | | | | | | | | | |
| C | 20 | 0 | 0 | 10 | 100 | 20 | 0 | 100 | 80 | 20 | | | | | | | | | |
| G | 10 | 0 | 20 | 90 | 0 | 0 | 10 | 0 | 0 | 10 | | | | | | | | | |
| T | 30 | 40 | 80 | 0 | 0 | 80 | 0 | 0 | 0 | 20 | | | | | | | | | |

| **SEQ ID NO44 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | 10 | 11 | 12 | 13 | 14 | 15 | 16 | 17 | 18 | |
| A | 27,27 | 63,64 | 45,45 | 0 | 0 | 9,09 | 0 | 63,64 | 0 | 0 | 54,55 | 36,36 | 36,36 | 72,73 | 54,55 | 45,45 | 63,64 | 45,45 | |
| C | 27,27 | 9,09 | 0 | 0 | 36,36 | 0 | 0 | 18,18 | 0 | 0 | 9,09 | 18,18 | 18,18 | 0 | 9,09 | 18,18 | 18,18 | 0 | |
| G | 18,18 | 0 | 9,09 | 0 | 0 | 9,09 | 100 | 18,18 | 0 | 100 | 18,18 | 9,09 | 9,09 | 9,09 | 9,09 | 27,27 | 18,18 | 18,18 | |
| T | 27,27 | 27,27 | 45,45 | 100 | 63,64 | 81,82 | 0 | 0 | 100 | 0 | 18,18 | 36,36 | 36,36 | 18,18 | 27,27 | 9,09 | 0 | 36,36 | |

| **SEQ ID NO45 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | 10 | 11 | 12 | 13 | 14 | 15 | 16 | 17 | 18 | 19 |
| A | 50 | 10 | 50 | 20 | 60 | 0 | 30 | 40 | 20 | 40 | 20 | 70 | 0 | 60 | 10 | 0 | 20 | 100 | 10 |
| C | 10 | 10 | 0 | 40 | 20 | 40 | 10 | 40 | 0 | 40 | 30 | 10 | 0 | 40 | 0 | 100 | 80 | 0 | 70 |
| G | 10 | 60 | 20 | 10 | 10 | 30 | 40 | 0 | 40 | 0 | 40 | 10 | 100 | 0 | 0 | 0 | 0 | 0 | 20 |
| T | 30 | 20 | 30 | 30 | 10 | 30 | 20 | 20 | 40 | 20 | 10 | 10 | 0 | 0 | 90 | 0 | 0 | 0 | 0 |

| Pos | 20 | | | | | | | | | | | | | | | | | | |
|---|---|---|---|---|---|---|---|---|---|---|---|---|---|---|---|---|---|---|---|
| A | 30 | | | | | | | | | | | | | | | | | | |
| C | 20 | | | | | | | | | | | | | | | | | | |
| G | 30 | | | | | | | | | | | | | | | | | | |
| T | 20 | | | | | | | | | | | | | | | | | | |

| **SEQ ID NO46 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | 10 | 11 | 12 | | | | | | | |
| A | 45,45 | 27,27 | 0 | 100 | 0 | 90,91 | 90,91 | 0 | 27,27 | 45,45 | 0 | 54,55 | | | | | | | |
| C | 18,18 | 0 | 100 | 0 | 0 | 9,09 | 9,09 | 9,09 | 9,09 | 9,09 | 27,27 | 18,18 | | | | | | | |
| G | 27,27 | 0 | 0 | 0 | 0 | 0 | 0 | 72,73 | 27,27 | 36,36 | 36,36 | 9,09 | | | | | | | |
| T | 9,09 | 72,73 | 0 | 0 | 100 | 0 | 0 | 18,18 | 36,36 | 9,09 | 36,36 | 18,18 | | | | | | | |

| **SEQ ID NO47 MATRIX:** | | | | | | | | | | | | | | | | | | | |
|---|---|---|---|---|---|---|---|---|---|---|---|---|---|---|---|---|---|---|---|
| Pos | 1 | 2 | 3 | 4 | 5 | 6 | 7 | 8 | 9 | 10 | | | | | | | | | |
| A | 33,33 | 0 | 33,33 | 100 | 0 | 0 | 0 | 0 | 0 | 0 | | | | | | | | | |
| C | 0 | 0 | 66,67 | 0 | 0 | 0 | 0 | 0 | 66,67 | 66,67 | | | | | | | | | |
| G | 33,33 | 0 | 0 | 0 | 0 | 0 | 0 | 0 | 33,33 | 0 | | | | | | | | | |
| T | 33,33 | 100 | 0 | 0 | 100 | 100 | 100 | 100 | 0 | 33,33 | | | | | | | | | |

Table 10: Matrix weight table defining the frequency of each A, T, G or C base at each positions in the defined motives. Pos. Position in the motive, frequency is given in %. A sum higher or lower than 100% in one position is due to round-off error.

### REFERENCES

The references listed below and all references cited herein are incorporated herein by reference to the extent that they supplement, explain, provide a background for, or teach methodology, techniques, and/or compositions employed herein.
Dunwell (2000) J Exp Bot 51 Spec No:487-96
Zhao et al. (1999) Microbiol Mol Biol Rev 63:405-445
Proudfoot (1986) Nature 322:562-565
Kim et al. (2003) Biotechnology Progress 19:1620-1622
Yonaha & Proudfoot (2000) EMBO J. 19:3770-3777
Cramer et al. (2001) FEBS Letters 498:179-182
Kuersten & Goodwin (2003) Nature Reviews Genetics 4:626-637
R. R. Aldemita and T. K. Hodges. Agrobacterium tumefaciens-mediated transformation of japonica and indica rice varieties. Planta 199 (4):612-617, 1996.
M. M. Bradford. Rapid and Sensitive Method for Quantitation of Microgram Quantities of Protein Utilizing Principle of Protein-Dye Binding. Analytical Biochemistry 72 (1-2):248-254, 1976.
M. T. Chan, H. H. Chang, S. L. Ho, W. F. Tong, and S. M. Yu. Agrobacterium-Mediated Production of Transgenic Rice Plants Expressing A Chimeric Alpha-Amylase Promoter Beta-Glucuronidase Gene. Plant Molecular Biology 22 (3):491-506, 1993.
Cartharius K, Frech K, Grote K. (2005) Matlnspector and beyond: promoter analysis based on transcription factor binding sites, Bioinformatics 21 (13) 2933 - 2942
Cartharius K (2005), DNA Press
Genbank V00087
Y. Hiei, S. Ohta, T. Komari, and T. Kumashiro. Efficient Transformation of Rice (Oryza-Sativa L) Mediated by Agrobacterium and Sequence-Analysis of the Boundaries of the T-Dna. Plant Journal6 (2):271-282, 1994.
D. W. Ow, K. V. Wood, M. Deluca, J. R. Dewet, D. R. Helinski, and S. H. Howell. Transient and Stable Expression of the Firefly Luciferase Gene in Plant-Cells and Transgenic Plants. Science 234 (4778):856-859, 1986.
J. Sambrook, E. F. Fritsch, and T. Maniatis. Molecular Cloning A Laboratory Manual Second Edition Vols. 1 2 and 3. Sambrook, J., E.F.Fritsch and T.Maniatis.Molecular Cloning: A Laboratory Manual, Second Edition, Vols.1, 2 and 3*.*Xxxix+Pagination Varies(Vol.1*);* Xxxiii+Pagination Varies(Vol.2*):* Xxxii+Pagination Varies(Vol.3) Cold Spring Harbor Laboratory Press: 1989.
F. Sanger, S. Nicklen, and A. R. Coulson. Dna Sequencing with Chain-Terminating Inhibitors. Proceedings of the National Academy of Sciences of the United States of America 74 (12):5463-5467, 1977.
www.phytozome.net
Mapendano, C. K., et al. "Crosstalk between mRNA 3' End Processing and Transcription Initiation." Molecular Cell 40.3 (2010): 410-22.
Nagaya, S., et al. "The HSP Terminator of Arabidopsis thaliana Increases Gene Expression in Plant Cells." Plant and Cell Physiology 51.2 (2010): 328-32.
Narsai, R., et al. "Genome-wide analysis of mRNA decay rates and their determinants thaliana." Plant Cell 19.11 (2007): 3418-36.
K. Quandt, K. Frech, H Karas, E Wingender and T Werner (1995), Matind and Matinspector: new fast and versatile tools for detection of consensus matches in nucleotide sequence data Nucleic Acid Research 23 (23) 4878 - 4884

## Claims

1. Terminator molecules comprising a sequence selected from the group consisting of
a) the nucleic acid molecules having a sequence as defined in SEQ ID NO: 1, 2, 3, 4, 57, 58, 59, 60, 61, 62, 63 and 64, or
b) a nucleic acid molecule having a sequence with an identity of at least 60% to SEQ ID NO: 1, 2, 3, 4, 57, 58, 59, 60, 61, 62, 63 and 64,or
c) a fragment of 100 or more consecutive bases of a nucleic acid molecule of i) or ii) which has expression enhancing activity as the corresponding nucleic acid molecule having the sequence of SEQ ID NO: 1, 2, 3, 4, 57, 58, 59, 60, 61, 62, 63 and 64, or
d) a nucleic acid molecule which is the complement or reverse complement of any of the previously mentioned nucleic acid molecules under i) or ii), or
e) a nucleic acid molecule which is obtainable by PCR using oligonucleotide primers described by SEQ ID NO: 63 to 96 as shown in Table 5 or
f) a nucleic acid molecule of 100 nucleotides or more, hybridizing under conditions equivalent to hybridization in 7% sodium dodecyl sulfate (SDS), 0.5 M NaPO₄, 1 mM EDTA at 50°C with washing in 2 X SSC, 0.1 % SDS at 50°C to a nucleic acid molecule comprising at least 50 consecutive nucleotides of a transcription enhancing terminator described by SEQ ID NO: 1, 2, 3, 4, 57, 58, 59, 60, 61, 62, 63 and 64 or the complement thereof,
wherein the terminator molecules cause enhanced expression of heterologous nucleic acid molecules to which they are functionally linked.

2. A recombinant expression construct comprising one or more terminator molecule comprising a terminator molecule as defined in claim 1 a) to f).

3. The recombinant expression construct of claim 2 wherein the terminator molecule is functionally linked to one or more promoter and one or more expressed nucleic acid molecule at least the latter being heterologous to said one or more terminator.

4. A recombinant expression vector comprising one or more recombinant expression construct of claims 2 or 3.

5. A transgenic plant or part thereof comprising a heterologous terminator molecule as defined in claim 1 a) to f).

6. A transgenic cell or transgenic plant or part thereof comprising a recombinant expression vector as claimed in claim 4 or a recombinant expression construct of claims 2 or 3.

7. The transgenic cell, transgenic plant or part thereof of claim 6, selected or derived from the group consisting of bacteria, fungi, yeasts or plants.

8. A transgenic cell culture, transgenic seed, parts or propagation material derived from a transgenic cell or plant or part thereof of claim 5 to 7 comprising a heterologous terminator molecule as defined in claim 1 a) to f), recombinant expression construct of claim 2 or 3 or recombinant vector of claim 4.

9. A use of the terminator molecule as defined in claim 1 a) to f) or the recombinant construct or recombinant vector as defined in any of claims 2 to 4 for enhancing expression in plants or parts thereof.

10. A method for the production of an agricultural product by introducing a terminator molecule as defined in claim 1 a) to f) or the recombinant construct or recombinant vector as defined in any of claims 2 to 4 into a plant, growing the plant, harvesting and processing the plant or parts thereof.


SEQUENCE LISTING

<110>  BASF Plant Science Company GmbH

<120>  Method for identification and isolation of terminator sequences
causing
       enhanced transcription

<130>  PF 72573

<160>  100

<170>  PatentIn version 3.4

<210>  1
<211>  588
<212>  DNA
<213>  Oryza sativa

<400>  1
gctgcctata gatgctcgta tgcaatatcg tgtgctgcca gatattggga agcctctgaa      60

gctaccagtt actgttctct atatttgaag tcataagact atttgttgct attaaagcga     120

ttcttgcttg atgcaagttg tgtcctcatt atgcactacc ggcatattat gagtatggtt     180

tgtctgggat attgtcaatc taataaaagt acttgctatt tgactataca tctggttttg     240

gttcctgtgt ctgctatcat cgtggttact tccaacatgc tggctacctg ttgatctgtc     300

atagtaatat ttcaacatct ggcgccattt tgaatttcct tgtatcggta ttaattttcc     360

gtgatgtcct tgcttttttt tcctatggtt caattgtatc ggagtgtaag ctgtgccgtt     420

gtgcgtcttg tcccgccggt agtgctagga gaaggcaatt tctactacct ctccgtccca     480

gaatattgag aattttttcc taagtacagc catcaaattt ttcctccgtc ccacggatcc     540

aatatcttgg aagcaatgtc catgtgatgt aaagattgtt ttctcccc                  588


<210>  2
<211>  794
<212>  DNA
<213>  Oryza sativa

<400>  2
ggccctggcc ctgatgatcg atcacctgcg aatctcaagt caagacgcaa attcatccta      60

tcagtgatgc tcccttgcga tactattgtt aataagaaag atatccatgt ttcctgtaaa     120

acgtatcccc accatcatca tcatcatcat cgtttagcct tgcctgcatg attctagctg     180

cagatggagt gttgtcggag atttacccgt atgaggtctc agagcctgta aaacgtatga     240

aacaccctct cccaaactct tatattgtgg tattaatatg gattactggg tgtggattgt     300

cctacatgtt ttttgtcgct ttgcctctcg ttgtctcgtg tcattctgga acattatgct     360

agctggaatc accttatggc caccaacctg catgatactg taattctact gaattggtca     420

gtactcttaa gaaacattcg ttctcggctt tttgagtgtt tttgacccgt cattttccct     480

tgtgtcgtgc ggcggcggcc acttggttcc gtgtaaaacg ccatcgctac gatctggtct 540

acctacctgg acctggctac caaccagcca attgtttctc ttgcaacgta cctggatctc 600

tcgcgtgtcg tacagaggcg tttggctgtg cacaaggcca cagggggaca attttatcga 660

acctgttcaa ttcgataaga tgcactctct tttgtttaaa aaaacaagac aaagcagtag 720

taattctgat gaagaaaatg tctgacaaaa ctttgtacac cgcaggctat tctgtcctac 780

tccctccatc ctaa 794

<210> 3
<211> 718
<212> DNA
<213> Oryza sativa

<400> 3
taaggtccac ctttgtggag tcatctatcc ttgaaaaggt acatgagctc cacaacattg 60

ggaggacata cacagggtcc ataaatgctg ccactacaat catggtagat cacccctctt 120

cccttctcag gaatctctgt tgtacacggc ttctctgtgt ttgctgctta gtgatttttc 180

accaatttcc gattccacaa taaaatgctc gtggtgtgtc gttctggcag acaattgaga 240

atataacata ttaaaacctc tatgttatgc accacaatgt ttcttttttt tttgaggggt 300

acaccacaat gtttctcgaa tcattttgtt tttgaaactg ggtttcttga aacatttcga 360

tctgaaacgg agtttctgat aacattattg tgctgctgct aaatatttta attattggat 420

tcaagaaatg gatcataact agaagaataa atcaggatct tttgtttcag aatagcatcc 480

gtgatggctt tagagaacca tcagttagct ttcacgtgct atctgctcct tttccatcaa 540

ctaaacccaa ggttcttgtg aaccctggca cgtgaatcct ggaaaggtcg tctctaatgt 600

gattttcagc tgtgcttagt tgcatgcctg cttggacatc gttgccagtg cttggtcact 660

ttattttgtt ttttttattc ctgtagcaat taaatcatca aagtgcacaa cagaattc 718

<210> 4
<211> 625
<212> DNA
<213> Oryza sativa

<400> 4
aatgtcattt tatctcctgt gatatgtaaa ggttgatgta acaatttccc agtggaactg 60

tcttgtgaga tcacaaagtt gtctgttctt atgatcataa cttgaagcta tttcaaataa 120

tatcagacag ctagttacac atatgtacat ctttgtattt ctctatccta ccagtattgg 180

actattggta tgatcaattt ggtaaaaata catgggtcat cacattgttt ctgtggcata 240

tgcgaaagtt tgtttccaag acttccaagt tactgtatta tcaccaaatg aaaactgatg 300

cctctatcga aagtgatttg gttattgagc tgttgcacct ttctttgctt tgtagtttca 360

gtaccttctg aaagggtttt ttgatcactg taggttatta agatggtaag atatattgta 420

aaacaagtct aacaattatg attaagttca tagtatttga gagatttaga gctggatctg 480

tattgtaact aattactttt ccgatggtat acagaaatac tttattttgg gcagaaaata 540

cataaaaaaa ataaattaat taaattaggc tagtgttctc cattagtaga agaacgacaa 600

agaattaaag aaaaagtttc gatgc 625

<210> 5
<211> 23
<212> DNA
<213> artificial

<220>
<223> synthetic sequence

<220>
<221> misc_feature
<222> (1)..(1)
<223> n is a, c, g, or t

<220>
<221> misc_feature
<222> (15)..(16)
<223> n is a, c, g, or t

<220>
<221> misc_feature
<222> (19)..(21)
<223> n is a, c, g, or t

<220>
<221> misc_feature
<222> (23)..(23)
<223> n is a, c, g, or t

<400> 5
   nrycttcccw tywwnntdnn ncn 23

<210> 6
<211> 15
<212> DNA
<213> artificial

<220>
<223> synthetic sequence

<220>
<221> misc_feature
<222> (1)..(1)
<223> n is a, c, g, or t

<220>
<221> misc_feature
<222> (10)..(10)
<223> n is a, c, g, or t

<220>

<221>  misc_feature
<222>  (13)..(13)
<223>  n is a, c, g, or t

<220>
<221>  misc_feature
<222>  (15)..(15)
<223>  n is a, c, g, or t

<400>  6
ngtgatwttn cwnsn                                                        15


<210>  7
<211>  14
<212>  DNA
<213>  artificial

<220>
<223>  synthetic sequence

<400>  7
btmmttttcc sttv                                                         14


<210>  8
<211>  13
<212>  DNA
<213>  artificial

<220>
<223>  synthetic sequence

<400>  8
davagccatc avt                                                          13


<210>  9
<211>  14
<212>  DNA
<213>  artificial

<220>
<223>  synthetic sequence


<220>
<221>  misc_feature
<222>  (6)..(6)
<223>  n is a, c, g, or t

<400>  9
dcttrntatt tkav                                                         14


<210>  10
<211>  26
<212>  DNA
<213>  artificial

<220>
<223>  synthetic sequence

<220>
<221>  misc_feature
<222>  (1)..(1)
<223>  n is a, c, g, or t

<220>
<221>  misc_feature
<222>  (7)..(7)
<223>  n is a, c, g, or t

<220>
<221>  misc_feature
<222>  (9)..(10)
<223>  n is a, c, g, or t

<220>
<221>  misc_feature
<222>  (23)..(24)
<223>  n is a, c, g, or t

<220>
<221>  misc_feature
<222>  (26)..(26)
<223>  n is a, c, g, or t

<400>  10
nadhatntnn dkwtggtttg thnnan                                            26


<210>  11
<211>  20
<212>  DNA
<213>  artificial

<220>
<223>  synthetic sequence


<220>
<221>  misc_feature
<222>  (1)..(1)
<223>  n is a, c, g, or t

<220>
<221>  misc_feature
<222>  (4)..(4)
<223>  n is a, c, g, or t

<220>
<221>  misc_feature
<222>  (12)..(15)
<223>  n is a, c, g, or t

<220>
<221>  misc_feature
<222>  (18)..(18)
<223>  n is a, c, g, or t

<220>
<221>  misc_feature
<222>  (20)..(20)
<223>  n is a, c, g, or t

<400>  11
nwanaatgas annnnahnan                                                    20


<210>  12
<211>  9
<212>  DNA
<213>  artificial

<220>
<223>  synthetic sequence


<220>
<221>  misc_feature
<222>  (1)..(1)
<223>  n is a, c, g, or t

<220>
<221>  misc_feature
<222>  (9)..(9)
<223>  n is a, c, g, or t

<400>  12
naaaagtan                                                                 9


<210>  13
<211>  14
<212>  DNA
<213>  artificial

<220>
<223>  synthetic sequence


<220>
<221>  misc_feature
<222>  (4)..(5)
<223>  n is a, c, g, or t

<400>  13
wkwnntggaa gcat                                                          14


<210>  14
<211>  17
<212>  DNA
<213>  artificial

<220>
<223>  synthetic sequence


<220>
<221>  misc_feature
<222>  (1)..(1)
<223>  n is a, c, g, or t

<220>
<221>  misc_feature
<222>  (3)..(5)

<223>  n is a, c, g, or t

<220>
<221>  misc_feature
<222>  (7)..(7)
<223>  n is a, c, g, or t

<220>
<221>  misc_feature
<222>  (9)..(9)
<223>  n is a, c, g, or t

<220>
<221>  misc_feature
<222>  (12)..(12)
<223>  n is a, c, g, or t

<220>
<221>  misc_feature
<222>  (17)..(17)
<223>  n is a, c, g, or t

<400>  14
nwnnnhnwnt gntattn                                                    17


<210>  15
<211>  23
<212>  DNA
<213>  artificial

<220>
<223>  synthetic sequence


<220>
<221>  misc_feature
<222>  (3)..(5)
<223>  n is a, c, g, or t

<220>
<221>  misc_feature
<222>  (7)..(8)
<223>  n is a, c, g, or t

<220>
<221>  misc_feature
<222>  (10)..(10)
<223>  n is a, c, g, or t

<220>
<221>  misc_feature
<222>  (12)..(12)
<223>  n is a, c, g, or t

<220>
<221>  misc_feature
<222>  (20)..(20)
<223>  n is a, c, g, or t

<220>
<221>  misc_feature
<222>  (23)..(23)

<223>  n is a, c, g, or t

<400>  15
wynnnhnnmn snaaactcan van                                              23


<210>  16
<211>  20
<212>  DNA
<213>  artificial

<220>
<223>  synthetic sequence


<220>
<221>  misc_feature
<222>  (1)..(1)
<223>  n is a, c, g, or t

<220>
<221>  misc_feature
<222>  (6)..(6)
<223>  n is a, c, g, or t

<220>
<221>  misc_feature
<222>  (8)..(9)
<223>  n is a, c, g, or t

<220>
<221>  misc_feature
<222>  (20)..(20)
<223>  n is a, c, g, or t

<400>  16
nwwkwntnnt hattatgmtn                                                  20


<210>  17
<211>  12
<212>  DNA
<213>  artificial

<220>
<223>  synthetic sequence


<220>
<221>  misc_feature
<222>  (8)..(9)
<223>  n is a, c, g, or t

<220>
<221>  misc_feature
<222>  (12)..(12)
<223>  n is a, c, g, or t

<400>  17
ygatggcnnt an                                                          12


<210>  18

<211>  23
<212>  DNA
<213>  artificial

<220>
<223>  synthetic sequence


<220>
<221>  misc_feature
<222>  (3)..(4)
<223>  n is a, c, g, or t

<220>
<221>  misc_feature
<222>  (12)..(12)
<223>  n is a, c, g, or t

<220>
<221>  misc_feature
<222>  (14)..(15)
<223>  n is a, c, g, or t

<220>
<221>  misc_feature
<222>  (17)..(17)
<223>  n is a, c, g, or t

<220>
<221>  misc_feature
<222>  (19)..(21)
<223>  n is a, c, g, or t

<400>  18
yhnnttgtkt cnknnknmnn nvm                                              23


<210>  19
<211>  19
<212>  DNA
<213>  artificial

<220>
<223>  synthetic sequence


<220>
<221>  misc_feature
<222>  (1)..(1)
<223>  n is a, c, g, or t

<220>
<221>  misc_feature
<222>  (3)..(3)
<223>  n is a, c, g, or t

<220>
<221>  misc_feature
<222>  (6)..(7)
<223>  n is a, c, g, or t

<220>
<221>  misc_feature

<222>  (17)..(17)
<223>  n is a, c, g, or t

<220>
<221>  misc_feature
<222>  (19)..(19)
<223>  n is a, c, g, or t

<400>  19
nhntynnktg ctttktndn                                                    19


<210>  20
<211>  16
<212>  DNA
<213>  artificial

<220>
<223>  synthetic sequence


<220>
<221>  misc_feature
<222>  (13)..(13)
<223>  n is a, c, g, or t

<400>  20
atktttcctg ydnmay                                                       16


<210>  21
<211>  18
<212>  DNA
<213>  artificial

<220>
<223>  synthetic sequence


<220>
<221>  misc_feature
<222>  (10)..(10)
<223>  n is a, c, g, or t

<220>
<221>  misc_feature
<222>  (15)..(15)
<223>  n is a, c, g, or t

<400>  21
wmctattgtn mwwwnkta                                                     18


<210>  22
<211>  18
<212>  DNA
<213>  artificial

<220>
<223>  synthetic sequence

<400>  22
ttttctcytw cytctsmy                                                     18

<210>  23
<211>  10
<212>  DNA
<213>  artificial

<220>
<223>  synthetic sequence


<220>
<221>  misc_feature
<222>  (10)..(10)
<223>  n is a, c, g, or t

<400>  23
kttgrttcyn                                                              10


<210>  24
<211>  17
<212>  DNA
<213>  artificial

<220>
<223>  synthetic sequence


<220>
<221>  misc_feature
<222>  (10)..(10)
<223>  n is a, c, g, or t

<400>  24
tkatattgyn dwaywwr                                                      17


<210>  25
<211>  12
<212>  DNA
<213>  artificial

<220>
<223>  synthetic sequence


<220>
<221>  misc_feature
<222>  (3)..(3)
<223>  n is a, c, g, or t

<400>  25
wsnwaactwg aw                                                           12


<210>  26
<211>  12
<212>  DNA
<213>  artificial

<220>
<223>  synthetic sequence

<220>
<221> misc_feature
<222> (1)..(1)
<223> n is a, c, g, or t

<220>
<221> misc_feature
<222> (4)..(4)
<223> n is a, c, g, or t

<220>
<221> misc_feature
<222> (10)..(10)
<223> n is a, c, g, or t

<400> 26
nwtnttatgn tm 12

<210> 27
<211> 13
<212> DNA
<213> artificial

<220>
<223> synthetic sequence

<400> 27
wmwwbtcaat aab 13

<210> 28
<211> 17
<212> DNA
<213> artificial

<220>
<223> synthetic sequence

<220>
<221> misc_feature
<222> (1)..(1)
<223> n is a, c, g, or t

<220>
<221> misc_feature
<222> (5)..(5)
<223> n is a, c, g, or t

<400> 28
ntyankdttc ytgtgaa 17

<210> 29
<211> 16
<212> DNA
<213> artificial

<220>
<223> synthetic sequence

<220>
<221>  misc_feature
<222>  (2)..(3)
<223>  n is a, c, g, or t

<220>
<221>  misc_feature
<222>  (7)..(8)
<223>  n is a, c, g, or t

<220>
<221>  misc_feature
<222>  (16)..(16)
<223>  n is a, c, g, or t

<400>  29
tnnrwknngt gttctn                                                        16


<210>  30
<211>  11
<212>  DNA
<213>  artificial

<220>
<223>  synthetic sequence


<220>
<221>  misc_feature
<222>  (1)..(1)
<223>  n is a, c, g, or t

<400>  30
ntattgtsrh d                                                             11


<210>  31
<211>  10
<212>  DNA
<213>  artificial

<220>
<223>  synthetic sequence


<220>
<221>  misc_feature
<222>  (1)..(1)
<223>  n is a, c, g, or t

<220>
<221>  misc_feature
<222>  (10)..(10)
<223>  n is a, c, g, or t

<400>  31
nwttgtttcn                                                               10


<210>  32

<211>  17
<212>  DNA
<213>  artificial

<220>
<223>  synthetic sequence


<220>
<221>  misc_feature
<222>  (1)..(1)
<223>  n is a, c, g, or t

<220>
<221>  misc_feature
<222>  (3)..(4)
<223>  n is a, c, g, or t

<220>
<221>  misc_feature
<222>  (13)..(15)
<223>  n is a, c, g, or t

<220>
<221>  misc_feature
<222>  (17)..(17)
<223>  n is a, c, g, or t

<400>  32
nwnnmcctkt ccnnnrn                                                      17


<210>  33
<211>  20
<212>  DNA
<213>  artificial

<220>
<223>  synthetic sequence


<220>
<221>  misc_feature
<222>  (1)..(1)
<223>  n is a, c, g, or t

<220>
<221>  misc_feature
<222>  (8)..(8)
<223>  n is a, c, g, or t

<220>
<221>  misc_feature
<222>  (20)..(20)
<223>  n is a, c, g, or t

<400>  33
nttasyknaw tdkcaccaan                                                   20


<210>  34
<211>  17
<212>  DNA

<213>  artificial

<220>
<223>  synthetic sequence


<220>
<221>  misc_feature
<222>  (1)..(2)
<223>  n is a, c, g, or t

<220>
<221>  misc_feature
<222>  (10)..(10)
<223>  n is a, c, g, or t

<220>
<221>  misc_feature
<222>  (12)..(15)
<223>  n is a, c, g, or t

<220>
<221>  misc_feature
<222>  (17)..(17)
<223>  n is a, c, g, or t

<400>  34
nnttactgsn wnnnnrn                                                    17


<210>  35
<211>  16
<212>  DNA
<213>  artificial

<220>
<223>  synthetic sequence


<220>
<221>  misc_feature
<222>  (1)..(1)
<223>  n is a, c, g, or t

<220>
<221>  misc_feature
<222>  (5)..(5)
<223>  n is a, c, g, or t

<220>
<221>  misc_feature
<222>  (13)..(13)
<223>  n is a, c, g, or t

<400>  35
nrrwnttaat aankwt                                                     16


<210>  36
<211>  11
<212>  DNA
<213>  artificial

<220>
<223>  synthetic sequence


<220>
<221>  misc_feature
<222>  (1)..(1)
<223>  n is a, c, g, or t

<220>
<221>  misc_feature
<222>  (3)..(3)
<223>  n is a, c, g, or t

<220>
<221>  misc_feature
<222>  (8)..(8)
<223>  n is a, c, g, or t

<220>
<221>  misc_feature
<222>  (11)..(11)
<223>  n is a, c, g, or t

<400>  36
ntntctgnta n                                                            11


<210>  37
<211>  21
<212>  DNA
<213>  artificial

<220>
<223>  synthetic sequence


<220>
<221>  misc_feature
<222>  (1)..(3)
<223>  n is a, c, g, or t

<220>
<221>  misc_feature
<222>  (5)..(6)
<223>  n is a, c, g, or t

<220>
<221>  misc_feature
<222>  (14)..(15)
<223>  n is a, c, g, or t

<220>
<221>  misc_feature
<222>  (19)..(19)
<223>  n is a, c, g, or t

<220>
<221>  misc_feature
<222>  (21)..(21)
<223>  n is a, c, g, or t

<400>  37

nnnhnnttgt ttcnnhwknm n                                                21

<210>  38
<211>  17
<212>  DNA
<213>  artificial

<220>
<223>  synthetic sequence

<220>
<221>  misc_feature
<222>  (1)..(1)
<223>  n is a, c, g, or t

<220>
<221>  misc_feature
<222>  (5)..(5)
<223>  n is a, c, g, or t

<400>  38
ntyankdttc ytgtgaa                                                     17

<210>  39
<211>  13
<212>  DNA
<213>  artificial

<220>
<223>  synthetic sequence

<220>
<221>  misc_feature
<222>  (4)..(5)
<223>  n is a, c, g, or t

<220>
<221>  misc_feature
<222>  (13)..(13)
<223>  n is a, c, g, or t

<400>  39
yktnnttgct ttn                                                         13

<210>  40
<211>  15
<212>  DNA
<213>  artificial

<220>
<223>  synthetic sequence

<220>
<221>  misc_feature
<222>  (1)..(1)
<223>  n is a, c, g, or t

<220>
<221>  misc_feature
<222>  (10)..(10)
<223>  n is a, c, g, or t

<220>
<221>  misc_feature
<222>  (13)..(13)
<223>  n is a, c, g, or t

<220>
<221>  misc_feature
<222>  (15)..(15)
<223>  n is a, c, g, or t

<400>  40
ngtgatwttn cwnsn                                                        15


<210>  41
<211>  23
<212>  DNA
<213>  artificial

<220>
<223>  synthetic sequence


<220>
<221>  misc_feature
<222>  (3)..(3)
<223>  n is a, c, g, or t

<220>
<221>  misc_feature
<222>  (6)..(8)
<223>  n is a, c, g, or t

<220>
<221>  misc_feature
<222>  (11)..(11)
<223>  n is a, c, g, or t

<220>
<221>  misc_feature
<222>  (23)..(23)
<223>  n is a, c, g, or t

<400>  41
mwnsrnnnbt nbrhggcttg twn                                               23


<210>  42
<211>  19
<212>  DNA
<213>  artificial

<220>
<223>  synthetic sequence


<220>
<221>  misc_feature

<222>  (1)..(1)
<223>  n is a, c, g, or t

<220>
<221>  misc_feature
<222>  (10)..(12)
<223>  n is a, c, g, or t

<220>
<221>  misc_feature
<222>  (14)..(14)
<223>  n is a, c, g, or t

<220>
<221>  misc_feature
<222>  (19)..(19)
<223>  n is a, c, g, or t

<400>  42
nycttttscn nnanywaan                                                    19


<210>  43
<211>  10
<212>  DNA
<213>  artificial

<220>
<223>  synthetic sequence


<220>
<221>  misc_feature
<222>  (1)..(1)
<223>  n is a, c, g, or t

<220>
<221>  misc_feature
<222>  (10)..(10)
<223>  n is a, c, g, or t

<400>  43
nwtgctaccn                                                              10


<210>  44
<211>  18
<212>  DNA
<213>  artificial

<220>
<223>  synthetic sequence


<220>
<221>  misc_feature
<222>  (1)..(1)
<223>  n is a, c, g, or t

<220>
<221>  misc_feature
<222>  (12)..(13)
<223>  n is a, c, g, or t

<220>
<221>  misc_feature
<222>  (16)..(16)
<223>  n is a, c, g, or t

<400>  44
nawtytgatg annawnaw                                                    18


<210>  45
<211>  20
<212>  DNA
<213>  artificial

<220>
<223>  synthetic sequence


<220>
<221>  misc_feature
<222>  (4)..(4)
<223>  n is a, c, g, or t

<220>
<221>  misc_feature
<222>  (7)..(7)
<223>  n is a, c, g, or t

<220>
<221>  misc_feature
<222>  (11)..(11)
<223>  n is a, c, g, or t

<220>
<221>  misc_feature
<222>  (20)..(20)
<223>  n is a, c, g, or t

<400>  45
wgwnabnmkm nagmtccacn                                                  20


<210>  46
<211>  12
<212>  DNA
<213>  artificial

<220>
<223>  synthetic sequence


<220>
<221>  misc_feature
<222>  (1)..(1)
<223>  n is a, c, g, or t

<220>
<221>  misc_feature
<222>  (9)..(9)
<223>  n is a, c, g, or t

<400>  46

ntcataagnr ba                                                          12

<210>  47
<211>  10
<212>  DNA
<213>  artificial

<220>
<223>  synthetic sequence

<400>  47
dtmattttsy                                                             10

<210>  48
<211>  669
<212>  DNA
<213>  Oryza sativa

<400>  48
ggctgatacc aatctgtaat gcctgaaaaa tgataaacca gctacctgtc tgtgttactt      60

cactatgtgc ggatgtaaca aaactacctt taagcatgtt atgcattaag caatgttgca     120

gttggttgct tgatccgaag atgtttcggg ctctcttctg ctagctatga tacatctggt     180

cccgtatgat gataatataa cataactcat ggtgaaaatt ccacttgttt gcgctcaagt     240

cttgcagttt ctttgctata tgattgattg atctgattct gcctgtttcc atgcaagcaa     300

gctgatatgc cgtgcttcca tttcggtcag cagttgctta acatgttaca gaattctgaa     360

ctgatctgat tcagtgttta cgccattcct taacatgtta agagagggtg aggtttttat     420

acagttaccg catcctaaat ttcttacatt atgcaagtct gaacttactg aattttcgat     480

cctgcataca tggtcatgtc tcgaacttaa ccatgtaaag cgatctacta acaagttatg     540

tggaagtgtt tctgtttggt caaataaaaa tgtttcaatc tggtgcattt ctggtaataa     600

tgatatcccc attcccaata tgaaaccaga ctgctcatac agaaacatac tgtgaaccga     660

attgaacga                                                             669

<210>  49
<211>  596
<212>  DNA
<213>  Oryza sativa

<400>  49
acgagccctc ctcatggagg cctgcagata caggggagtt gtgttttgcc ccagagaaga      60

gtagatgaag cctcttccga gaataaattt taaattctgt atggttttat gtccgtcgaa     120

acctaaaact atacttggtt gtatcatggt ggttggttgg gcctggtcat ggctcatatt     180

ttgtgtctaa ttttcttgcg cttaatctaa atcgaagtgt tgcttcgcag atgcatttgc     240

tctgttttct ggttgcttct taaatacgcg cgccctaaac cctatgtgcg cgcgccagag     300

tttcttcctg atttacaacg tttgctgttt agtcattggc caaacccctt agagcccacc     360

ttgattgatc gaaacccatc tcctccatcg ataaaaattg gacctaactt atcgttaatg 420

caagtgcatt gtctcgctgg ggatgagaaa accaccagca ggtcagcaag ccaggagcta 480

cttctgtccg ctttgtctct agaatttgta gaatgctcgt tttcaacgga aatgtggact 540

gccatcaggc ctcaattgtg aagtctccat tgcacacgag gatttatctc tagaat 596

<210> 50
<211> 544
<212> DNA
<213> Oryza sativa

<400> 50
gcggtggggc cctcatggcc aagttatcta tctatctaat cgagctacca tcatcatcat 60

ccgatcgtta tcatcgttag ttttgtgtgg aactactatc tagtttgtgt tactgtgtgg 120

ttgcccatct gtgtttttga tcgcaagaag aaagctcgtc tcgtgtttgc tttgatcaaa 180

tgaaatgaat gaatgaatct tagtgtgctc cgctctcgtc aaatccatcg aattatttaa 240

tttgtcatgg ttgtgaatca tgggggttga tactatttgt tgttgatgct agtgcaaatg 300

atcatcatca tcacgatttg atgatttgct aagcataagc agcatcatta gctaccactc 360

acactgactg tgatgaagct gtgaactcac actgatgcta ctactgaagc gtttgtctga 420

tttccttacg attggatttg ttgctaaaca gcatcgttag ctagcagcgg tgacagtgat 480

gagcagtgat gctgctggac tagagatctg ctgatctgtg tacaaataga ttggagcaaa 540

gctt 544

<210> 51
<211> 488
<212> DNA
<213> Oryza sativa

<400> 51
acgcatcatg taattccgga tggatctaaa attccatgag tactgaaata attgtaacgt 60

cacaacactg ctgcgtgcta ccgctggaat gttctatgtt attgaccaag taacgttaca 120

ccatcgtcta tggacacgat aataagtttc gggccgtaca ttttcgtatt acttttgctg 180

aattcgtcgt ctctttgtta taacaagttt catgccgtta ccttgttgca ttactttgac 240

tgagacggca gtggcaatgt ggcatactgg catgatatgt tcaggtaagc agaaggccgt 300

gtggtggtga actggtgatg ctcaaccggt gacgcctcta attggcagtt cattccaaac 360

ttatccaaaa tagtttattt cgtactgatg cagccaaatt ttgaatattt aaactaactt 420

taaattaaac taggtggttt tgcatcaaag ccttggcttt caaacggcga aaagttacag 480

aaatcaat 488

<210> 52

<211>  622
<212>  DNA
<213>  Oryza sativa

<400>  52
atctagctcc atggagagga tatggaagac ttgagcttct gagagctagc tgtcagtaat      60

ttgtgaagta aagtagctgt tatccttttg tgaagttttc cccactgtta tggaatgatg     120

tctagatcgt aatatgccgt tgagcagaca tgagtttgac atctggagtg tatatttgtt     180

gctgcaaact gcaaagtgaa cactcccatg tatattccat acctttcgtt cccatgcatg     240

tatataaggc attactgcta ccgttgtatg gtatacccgc tgcatgtgtt tgcatttcat     300

cgattcttct cctggtctat tgtcgctgaa aaatgctttg tcgtcctgat tctgccagca     360

gcactttttc atgcgacctg gctactcttt actcaagatt tgccttattt tttttcaggt     420

aagaagacga tgctaatcgt ctgattgcct gatgatacga agaacggttt aaacaacagt     480

tttttttaaa aaaaactttt catgtcataa tttagataac gattatacaa ctgctgaatg     540

ttgtctattt actagttttc aatggatttt acaaaatttg taatttaatt ctgtacactt     600

gacaaagtgg tatggacaat tg                                              622


<210>  53
<211>  653
<212>  DNA
<213>  Oryza sativa

<400>  53
tcggcgacgt atggtaatta attacacggc gtttttaatt ccctttatta tgtgttcata      60

ataagattag aggagatata ttccggtaag ataatctctt ttttttttcg tttttacggt     120

tcatgttcac gttgttgttg ttgtcgtcat cgtcgtcggc aaattaattg ttcttgtcat     180

gtaatgtttg ttgatcgatc ccttttggtg ataggaaaga tgtacatcag actctgtaat     240

aatccatata tatgtctaat caaatttcaa ttacatgtgg caattagctc atatatcttg     300

atcaactctc agaatggtac aatgctcgtg ttgtttttac ccctacatgg ataagtgagg     360

tacttaaact tagagaaaat tatgaaagat ttctcttata taagagagaa aatgcaaaaa     420

tctctgtgtt ttttaagaag tgggaatatt acacctcctc tgcatcttaa cacagcctta     480

ggtttttatt acaagatggg aaaaaaataa atgtgggacc cagtcccact tcaaagcaac     540

aacactaata gagaccaaac aactcatcaa aaaattatgt ttccataaac catcaacaat     600

caactccaag cagataggtc caatgactat accattctga ttcttccatc gct            653


<210>  54
<211>  656
<212>  DNA
<213>  Oryza sativa

<400>  54

aagagggaac ttctctgtaa cccaacattt tacacaaaga cctgctctgt acctacattt 60

caaattcgtg atacgaaaca aaatagtaca tttgcacctg taaatatcgg atggttgata 120

cttcaactat gtgaagatgg atgtgtatca acctgacaag cccgaaaatt cagtgagtaa 180

aaaaaaaacg gcttctctat aaacttgtgg cactgttagt gttagctcag ttctgcatgg 240

agagcttcat ttgtcggctt agagagtagg acatacgtgc ttgtgtggtt gtaattttgt 300

ttttggtgga cgtctgatat atcagctcgt gtttttgatt cagtgcaagc ttctgtacat 360

tggaacaatg cgtcgatgca gacatgattc gcaaattcag caccatttgg gtacattcat 420

cctactcact actcagggac caatctgtga gacttggaaa gctatgaggg cccaactgtc 480

taaactgaaa gaataggagc gtgcgtgaga aacagcacct ttttcgcttt actacctgtg 540

acgtttgacc tgtcctcgtg caaaaacaac aataaacgcg tcagcgtgtg cgtgctcgca 600

tggcgtaagc aatgtttggg tttagaccgt ggttagttca gcgtaaagtt tagatt 656

<210> 55
<211> 828
<212> DNA
<213> Oryza sativa

<400> 55
tcattgattg atggaattgc tgctgtactg ttatcctgtg tctgcattat cctcgtgaaa 60

actttatttg tgctgttagt ggaccatcga gtccgtttaa atgtgctgta ctgctgtccg 120

aacatttgct gggtcatagc agtttaaact aattaataag taaactatta atctgcgttg 180

ctaaatttgc ttatagttct gcacccatta caactcttca ttcaaatttg cctacatttc 240

agattaaagt gcctgcgtgc tgctacatct atttcttgat ttattttatt gactagtaga 300

tcagagtgat ataagtccta ttgggtacac tggagtgaaa caatggcaga ttcttatcat 360

gctaatcacc aaatggatcg tttggacttt gtagccctca aattattagg gcgttttctg 420

gatgatatca tgagggtgtt ttctggggga tggtgctgca aagcaaactt taggaacatg 480

tgactcaatt ttttttagaa tgggcaatat atgtccaatg gttctattac gtcattttcc 540

cgtctattta cacaatcgcg ttctttgaat gctagaactg caacttgatc atgctggagc 600

tctctaagct tatagtttta cagctgaaaa acagaaaata aaatgctaat gtttagtatt 660

cagatcattg cttttgatga gttcagtttt cctcatcaaa gtctatccta caattactaa 720

tgatctcaac aacagttaag actttgttag tgataggaat acacgagtta tcagtgctgt 780

gtttagttcc acgacaaatt cggaagtttg aagccttgaa gccttgaa 828

<210> 56
<211> 687
<212> DNA
<213> Oryza sativa

<400> 56
tgttggtggg gcccatcgtg gccagttatc cttagctatc cgtgtcagaa tcatcttatc 60

atcgagtcga gtcgttatcg tgtccagtgg ctctctcgag tcgagaagcc ctctatccat 120

ccatccagtg ttaggtgttc ttcgtccgtg atgttaccat gaattgagtt cgctttggtt 180

atggtgtttg aactgcttgt tgctatctat cggaatgaaa tgaaatagaa aacaaggaga 240

aaaaaaagag ttcgaaagtt ttgttcgcat accatatatt tccttccggt gcgcgctgtt 300

tattcctcgc tcagcagcaa gattgtttga tcgatattgc agcaagcaat tacacaataa 360

atatattgct acactggtac ttcaaactac actggtggtc ggtgattttc aatagcatga 420

accttaattg aacatctgtg tagcttacat ctccttcgaa agctgcaatg cttgagaact 480

tggaaagaaa ttcttgtgat ggcagaagct attcactgtc cttcgctgca tttacagtcc 540

atacagacac agcatttcca ttttgcacaa gatagagaac aacaatcagc cttttaggtc 600

aatcccaagt gtgcatctta ctgattgtcg aatatgtgct aagaacctgc aagagagtga 660

ggatttttat cattgattga ttgtcga 687

<210> 57
<211> 603
<212> DNA
<213> Sorghum bicolor

<400> 57
tcataagctc caatctcgtg tgtgtggtta tctatccttg aaaaggataa gggctcaggg 60

ctccacaata atgggaaggc accagcaggc tccactgagt gctgccacca catcaatggt 120

agtttttctc cttcctttcc ctgtccaaca atgctttgcc tgcatttgta caagccatct 180

gggtgctttg tgatatttct ccatttcata ttcagcagaa tatatccttt tcatgcaatg 240

taatcctatg gcagaggaaa attcagagct ccatttcata attgcaccaa tgattccttg 300

ttatactctg atttatggta gcattagtct atcaaagtag gagggtcata gtacttggca 360

gaaggtgcag aatcagcata tttgattcca aatgccattc gtgatggctt taactgtcag 420

ccgtcaacta actttgacat gtgacctgta ccttttccat caacaaagct caggattctt 480

gtgaactctg tcgtctgtga gaccctaaag tgctctgcat cagaaaaatg taatgccagg 540

attctgactt tctgctaggg tacaagtgcc acgctaatac gtactagctt attggtttct 600

gta 603

<210> 58
<211> 630
<212> DNA
<213> Brachypodium distachion

<400> 58
gctccatttt tgtgaatccg gccttcaaag ggacatgagc tccaaaaaat tgggaaaaca 60

gagtccatgc gaactgccgc tacacccaat ggtagatctt cccttctagc tattccctgc 120

taaaagatta ataaagtgct ttgcctctgt acaaggcatc tctgcgttcc caacttggtg 180

attttccacc aatgtagcat accataataa aatacttgtc atgtaatgat ctgacgagga 240

cacaaaaaat tcaaagatga aagatcaaag caatatacta ctgtttgact ggtttttgaa 300

tctctttgat gatgctcgta cagtcacatg tgatctatca taattggcag aatcagcatc 360

tttgacacca aaatgccatt cgcgatggct ctaacgtaga cctgtcaagt gtcaactaac 420

tgtcacgtgt gatctattcc ttttccatca actaagttca gggttcttgt gaacctacat 480

atctagagtg ctttgcagct tgtatgcacg gatccttagc tgggagcatc attactgcta 540

tgtagtactt cgtacttggt tgttttgttt aggacaatag ttagcgtgtt tgtcttttgt 600

tgattactgt aattcagtcg ttcaattgca 630

<210> 59
<211> 730
<212> DNA
<213> Zea mays

<400> 59
accccggtct cacgcttaat agtggtaagg cacaggcagg ctccagctcc acatcaatgt 60

tagatttttt ttttctcctt ttttaccctg tccaacgatg ccatgcctgc atttgtacaa 120

gccatctgcg cgtgttcacc atttcacatt cagcaaaata aataattttc gtgcaatgtc 180

attctatgtc gggggagaat ttagagctcc agttcatgac tgcatcaatg gttcctcgtt 240

aaactttgat ttatgggtag cattggtcta tcaaagtagg aaggtcatac ctggcagaag 300

gtgcggaatc agcatatttg attccaaatg ccattcacga tggtcgatgg cctttaaccg 360

tcaaccatcc actacctttg gcatgtgaca tgtacctttt tttccatcaa aaagctcagg 420

attcttgtga actctgtcgt ctgtgaaacc ctaaagtgct ctgcagcagg aaaaatgtga 480

tgccaggtcg gtacaagtgc cacgcaaata ctagcatagt attggttttt gtacttggtt 540

taaggttctg gacctgagtg tttttccttg ttgattccag ccactagagt aactgaactg 600

cttcactagc ttactgcaaa accctgggca atggactgtg tgattaaaca ctgatgaggc 660

ggcattgaac ataaattccg ctgtatttac atctctctgc aagtggccaa aaacaaacag 720

taggcgtgtg 730

<210> 60
<211> 739
<212> DNA
<213> Brachypodium distachion

<400> 60
tgccgattgg tgataatctg tgggcttcag tcggatgcag attcatgcta gccgtgatgc 60

tgttgccacc actttattgt taataagtat atccatcaag gtttcctgta aaacgtatca 120

tttagctgtg ctccgtggtt ctctagcaga tgaagtgtta tctgggattt atctgtgcgg 180

tgttctgagc ctgtaaactc aggaagtatt gtgatattgt tactggattg ctgggtgagt 240

gggtgtggaa tctactaaat tgcccccatt gtgtcggcat aacatgtcgt ggcataactt 300

gaccttccac tgttcgcagt ccacacactg tcttttgctc tgtcatacca actcgagatt 360

tatggttctt gacagtttgc tcgaatgttt tactcctttc cttcatgtct tgttaggaac 420

aaaagccttg ctggaaccac cttgcctcgc aaatttcgca atatcgacca ccttatgata 480

agcaacatcc caattatgta agaaagataa gtacatttct tgacctcctt tgctttttag 540

ggcgtgtaca atgggatttg ctttttaggg cgtgtacaat gagacgacgt taattttctc 600

ttagcgatgc catataggat aaaatctgat gtgaaagaga gagaaatgaa gaaaaaacac 660

aagccttttc ttaattaaga gatgatctct tcacaagaat gataaggcaa agttaagaca 720

aatttaccat tgtactaga 739

<210> 61
<211> 344
<212> DNA
<213> Zea mays

<400> 61
gattcaaaat catttatgca tgcatctcta ttttgtttca ttcatgttta gtcgcattaa 60

gctgtagtgc taatgccacc gaaagtaaac tagatctagc taaagaggta aactgttgac 120

gagttgtcat tttagaagtc tatgtatttg caaagaaaaa tacttggata cttaaaatgc 180

accaaacgca gtaaacttaa catgctaatc atttaggtgc tcccatattt ttttaaagtg 240

ttattaactc agcgttgaac tacaaagttg ggttggttga ttctacttta tactgtttgg 300

cgaaagggcc tctagctgag ttggttaggt ggtctgagta gcac 344

<210> 62
<211> 546
<212> DNA
<213> Sorghum bicolor

<400> 62
gggacctgta aatgcttgtg ccctatattg tgcgcctcca catattggga agcttgaagc 60

agcgacaatt actagtcatt gctttcttta tataagaaca taagaactat tgttctattg 120

tcaattgtgt cttgcttgat gcaagttgtg ttttcgtctc attgttatgt gcggtcagca 180

tatgtgtatg gcttgtacta tgggttattg ccaacttaat aaaagtactt tgtgtttggc 240

tataagagct gatgtttgtc tcgtgcactt gttctgagtt ggtttttatc tgtactaatt 300

acctccttgt tgcgcatgtg gtgttctagc cgtgcgcaac tcaattggat gatctaaagt 360

tgtcaggtgt caattgttct cgtggagcga gctactgtaa attactgttg ccggattaac 420

tcagcatccg tgcgccgcaa ttgcgtgttt ttagtgccaa tgagcttaac tgttgaaatt 480

tacaggagca tagactgcat agttcaaggc cttgtttact ttccgaaatt ttgggccgaa 540

atgcaa 546

<210> 63
<211> 686
<212> DNA
<213> Brachypodium distachion

<400> 63
gcagcctata aatgcttgta tgcattatat tgtgtgctac ctgtgatatt gctggaagcc 60

ttggaaactt gaacctgtga tattgctggc tattactttc atctgtagtg aagacataag 120

gatattgtcg ctgttaagtg cttcttgctt gatgcaagtt gtgttgtcag acgtcacctc 180

attatactgt acccgcatat cagtatggtg tgtttgaaat gttgccgact aaattatatc 240

atctgccatt tgactatagc tcttgagttt ggcccatgtt gttcctattg tcactagtta 300

tttagtttgt ttgctgttca attttctttc tgttaccact gattttcctg tgctcctcgg 360

aagcgtgaaa gcttgtacaa ctgcccgaaa aaccatggat cacgtcgtgt tactgtcttc 420

ctgcgcctcc aaacaggata ggaacgagat caacctagtg ttcagatgag cctgtacaaa 480

cgtgcacact gaagtgcttt tcaggtccgg tgaaagagcg gcatccgaca ttttattgag 540

agcgtaccat tcaataaccc tgatcttgtt ctgtggtgac ttttgtattt gatgatctcc 600

atagtctggc aaaagaagac ccttcccgtc ggtgcttctc tgttgttgaa caaacttcac 660

cagagataaa ccagttcaca attttc 686

<210> 64
<211> 655
<212> DNA
<213> Zea mays

<400> 64
actgcaaatt ttgagtgctt gtgtgtgcct atcatatggt accatatgat accaggtatt 60

gaagcttcgg aagcttgttg gccttcgtaa catcagtagt cgttattcat ctgaatatgt 120

gtcattattt gatttgataa gactggtatt gatgcaagtt gtccttgcag tatgttttgt 180

aagtgttacc tgcatgaaag tatcgtttgt ctggggaact gtctactact gatattgaat 240

aacaagagag attgctgttc gtgttcgtct atgaattatt atgatctttc cccagacttg 300

agctttccaa tccgtgttct tttatgatca ctttgcttct catgtgcttt ctgatatgta 360

tttgaccttc acctcaagtt gtatctttta tgatccaatc cgtgtttgtt tcccatgcca 420

cacaaaatat ggatattaac ctattgtcat gtttttgtgt gggttatcga tctctcatta 480

aataggtttg tttggtttgt tgttgtctca cttgtcgtag tttagcacgt ctacccttag 540

acggatttaa tcaagttagg tatgtgtttt gtttggcaag atttttctcc cgctgcttat 600

gtctcactcg tcaatgactg ttaatagtgt agcaaaattc tcttaggcat tgtca          655

<210>  65
<211>  196
<212>  DNA
<213>  Agrobacterium tumefaciens

<400>  65
ccctgcttta atgagatatg cgagacgcct atgatcgcat gatatttgct ttcaattctg      60

ttgtgcacgt tgtaaaaaac ctgagcatgt gtagctcaga tccttaccgc cggtttcggt     120

tcattctaat gaatatatca cccgttacta tcgtattttt atgaataata ttctccgttc     180

aatttactga ttgtcc                                                     196

<210>  66
<211>  253
<212>  DNA
<213>  Agrobacterium tumefaciens

<400>  66
gatcgttcaa acatttggca ataaagtttc ttaagattga atcctgttgc cggtcttgcg      60

atgattatca tataatttct gttgaattac gttaagcatg taataattaa catgtaatgc     120

atgacgttat ttatgagatg ggtttttatg attagagtcc cgcaattata catttaatac     180

gcgatagaaa acaaaatata gcgcgcaaac taggataaat tatcgcgcgc ggtgtcatct     240

atgttactag atc                                                        253

<210>  67
<211>  40
<212>  DNA
<213>  artificial

<220>
<223>  synthetic sequence

<400>  67
tatactcgag gctgcctata gatgctcgta tgcaatatcg                            40

<210>  68
<211>  40
<212>  DNA
<213>  artificial

<220>
<223>  synthetic sequence

<400>  68
tataaagctt ggggagaaaa caatctttac atcacatgga                            40

<210>  69
<211>  35
<212>  DNA

<213>  artificial

<220>
<223>  synthetic sequence

<400>  69
tatactcgag ggccctggcc ctgatgatcg atcac                                35


<210>  70
<211>  43
<212>  DNA
<213>  artificial

<220>
<223>  synthetic sequence

<400>  70
tataaagctt ttaggatgga gggagtagga cagaatagcc tgc                       43


<210>  71
<211>  40
<212>  DNA
<213>  artificial

<220>
<223>  synthetic sequence

<400>  71
tatactcgag taaggtccac ctttgtggag tcatctatcc                           40


<210>  72
<211>  36
<212>  DNA
<213>  artificial

<220>
<223>  synthetic sequence

<400>  72
tatagaattc tgttgtgcac tttgatgatt taattg                               36


<210>  73
<211>  46
<212>  DNA
<213>  artificial

<220>
<223>  synthetic sequence

<400>  73
tatactcgag aatgtcattt tatctcctgt gatatgtaaa ggttga                   46


<210>  74
<211>  42
<212>  DNA
<213>  artificial

<220>

<223>  synthetic sequence

<400>  74
tataaagctt gcatcgaaac tttttcttta attctttgtc gt                          42


<210>  75
<211>  34
<212>  DNA
<213>  artificial

<220>
<223>  synthetic sequence

<400>  75
tatactcgag ccctgcttta atgagatatg cgag                                   34


<210>  76
<211>  34
<212>  DNA
<213>  artificial

<220>
<223>  synthetic sequence

<400>  76
tataaagctt ggacaatcag taaattgaac ggag                                   34


<210>  77
<211>  36
<212>  DNA
<213>  artificial

<220>
<223>  synthetic sequence

<400>  77
tatactcgag gatcgttcaa acatttggca ataaag                                 36


<210>  78
<211>  34
<212>  DNA
<213>  artificial

<220>
<223>  synthetic sequence

<400>  78
tataaagctt gatctagtaa catagatgac accg                                   34


<210>  79
<211>  40
<212>  DNA
<213>  artificial

<220>
<223>  synthetic sequence

<400>  79

tatactcgag ggctgatacc aatctgtaat gcctgaaaaa                                40

<210>  80
<211>  40
<212>  DNA
<213>  artificial

<220>
<223>  synthetic sequence

<400>  80
tataaagctt tcgttcaatt cggttcacag tatgtttctg                                40

<210>  81
<211>  28
<212>  DNA
<213>  artificial

<220>
<223>  synthetic sequence

<400>  81
tatactcgag acgagccctc ctcatgga                                             28

<210>  82
<211>  35
<212>  DNA
<213>  artificial

<220>
<223>  synthetic sequence

<400>  82
tataaagctt attctagaga taaatcctcg tgtgc                                     35

<210>  83
<211>  28
<212>  DNA
<213>  artificial

<220>
<223>  synthetic sequence

<400>  83
tatactcgag gcggtggggc cctcatgg                                             28

<210>  84
<211>  36
<212>  DNA
<213>  artificial

<220>
<223>  synthetic sequence

<400>  84
tataaagctt tgctccaatc tatttgtaca cagatc                                    36

<210> 85
<211> 38
<212> DNA
<213> artificial

<220>
<223> synthetic sequence

<400> 85
tatactcgag acgcatcatg taattccgga tggatcta 38


<210> 86
<211> 40
<212> DNA
<213> artificial

<220>
<223> synthetic sequence

<400> 86
tataaagctt attgatttct gtaacttttc gccgtttgaa 40


<210> 87
<211> 34
<212> DNA
<213> artificial

<220>
<223> synthetic sequence

<400> 87
tatactcgag atctagctcc atggagagga tatg 34


<210> 88
<211> 34
<212> DNA
<213> artificial

<220>
<223> synthetic sequence

<400> 88
tataaagctt caattgtcca taccactttg tcaa 34


<210> 89
<211> 38
<212> DNA
<213> artificial

<220>
<223> synthetic sequence

<400> 89
tatactcgag tcggcgacgt atggtaatta attacacg 38


<210> 90
<211> 40
<212> DNA

<213>  artificial

<220>
<223>  synthetic sequence

<400>  90
tataaagctt agcgatggaa gaatcagaat ggtatagtca                              40


<210>  91
<211>  40
<212>  DNA
<213>  artificial

<220>
<223>  synthetic sequence

<400>  91
tatactcgag aagagggaac ttctctgtaa cccaacattt                              40


<210>  92
<211>  40
<212>  DNA
<213>  artificial

<220>
<223>  synthetic sequence

<400>  92
tatagaattc aatctaaact ttacgctgaa ctaaccacgg                              40


<210>  93
<211>  40
<212>  DNA
<213>  artificial

<220>
<223>  synthetic sequence

<400>  93
tatactcgag tcattgattg atggaattgc tgctgtactg                              40


<210>  94
<211>  36
<212>  DNA
<213>  artificial

<220>
<223>  synthetic sequence

<400>  94
tatagaattc aaggcttcaa ggcttcaaac ttccga                                  36


<210>  95
<211>  31
<212>  DNA
<213>  artificial

<220>

<223>  synthetic sequence

<400>  95
tatatacata tgttggtggg gcccatcgtg g                                    31


<210>  96
<211>  40
<212>  DNA
<213>  artificial

<220>
<223>  synthetic sequence

<400>  96
tataaagctt tcgacaatca atcaatgata aaaatcctca                          40


<210>  97
<211>  15971
<212>  DNA
<213>  artificial

<220>
<223>  vector sequence

<400>  97
   caggcagcaa cgctctgtca tcgttacaat caacatgcta ccctccgcga gatcatccgt      60
   gtttcaaacc cggcagctta gttgccgttc ttccgaatag catcggtaac atgagcaaag     120
   tctgccgcct tacaacggct ctcccgctga cgccgtcccg gactgatggg ctgcctgtat     180
   cgagtggtga ttttgtgccg agctgccggt cggggagctg ttggctggct ggtggcagga     240
   tatattgtgg tgtaaacaaa ttgacgctta gacaacttaa taacacattg cggacgtttt     300
   taatgtactg aattaacgcc gaattgaatt caagagctca aggatcctaa ctataacggt     360
   cctaaggtag cgaaggcgcg ccgaattcga ggggatcgag cccctgctga gcctcgacat     420
   gttgtcgcaa aattcgccct ggacccgccc aacgatttgt cgtcactgtc aaggtttgac     480
   ctgcacttca tttggggccc acatacacca aaaaaatgct gcataattct cggggcagca     540
   agtcggttac ccggccgccg tgctggaccg ggttgaatgg tgcccgtaac tttcggtaga     600
   gcggacggcc aatactcaac ttcaaggaat ctcacccatg cgcgccggcg gggaaccgga     660
   gttcccttca gtgagcgtta ttagttcgcc gctcggtgtg tcgtagatac tagcccctgg     720
   ggcacttttg aaatttgaat aagatttatg taatcagtct tttaggtttg accggttctg     780
   ccgctttttt taaaattgga tttgtaataa taaaacgcaa ttgtttgtta ttgtggcgct     840
   ctatcataga tgtcgctata aacctattca gcacaatata ttgttttcat tttaatattg     900
   tacatataag tagtagggta caatcagtaa attgaacgga gaatattatt cataaaaata     960
   cgatagtaac gggtgatata ttcattagaa tgaaccgaaa ccggcggtaa ggatctgagc    1020
   tacacatgct caggtttttt acaacgtgca caacagaatt gaaagcaaat atcatgcgat    1080
   cataggcgtc tcgcatatct cattaaagca gggggtgggc gaagaactcc agcatgagat 1140
   ccccgcgctg gaggatcatc cagccggcgt cccggaaaac gattccgaag cccaaccttt 1200
   catagaaggc ggcggtggaa tcgaaatctc gtgatggcag gttgggcgtc gcttggtcgg 1260
   tcatttcgaa ccccagagtc ccgctcagaa gaactcgtca agaaggcgat agaaggcgat 1320
   gcgctgcgaa tcgggagcgg cgataccgta aagcacgagg aagcggtcag cccattcgcc 1380
   gccaagctct tcagcaatat cacgggtagc caacgctatg tcctgatagc ggtccgccac 1440
   acccagccgg ccacagtcga tgaatccaga aaagcggcca ttttccacca tgatattcgg 1500
   caagcaggca tcgccatggg tcacgacgag atcctcgccg tcgggcatgc gcgccttgag 1560
   cctggcgaac agttcggctg gcgcgagccc ctgatgctct tcgtccagat catcctgatc 1620
   gacaagaccg gcttccatcc gagtacgtgc tcgctcgatg cgatgtttcg cttggtggtc 1680
   gaatgggcag gtagccggat caagcgtatg cagccgccgc attgcatcag ccatgatgga 1740
   tactttctcg gcaggagcaa ggtgagatga caggagatcc tgccccggca cttcgcccaa 1800
   tagcagccag tcccttcccg cttcagtgac aacgtcgagc acagctgcgc aaggaacgcc 1860
   cgtcgtggcc agccacgata gccgcgctgc ctcgtcctgc agttcattca gggcaccgga 1920
   caggtcggtc ttgacaaaaa gaaccgggcg cccctgcgct gacagccgga acacggcggc 1980
   atcagagcag ccgattgtct gttgtgccca gtcatagccg aatagcctct ccacccaagc 2040
   ggccggagaa cctgcgtgca atccatcttg ttcaatccac atgatcaaac gttttgagga 2100
   cgcgagagga ttcgattcga cgacgagagc ctcgcgagat tggggagaaa tttttcgggg 2160
   gtggagctga tgcgaggaga ggagatgagg gggctggtat ttatggcggt tgggtggtgg 2220
   gaggagtccc gtgccgtgac gtctccgtct gcttggagaa tccgccacgc tgaaaccacc 2280
   gcggtttccg ggaagacgag gcgggcgagc gagcggttgg gaaatttcga gaagatgccg 2340
   tttgtctccg tttggtacac gtctcgttga ttttttttta gtgaattacg ctttggacca 2400
   cattttatta tctaagggtg tgtttggttg taagccacac tttgccacag tttgccacgc 2460
   ctaaggttag gcaaatttga caggtgtttg gttgtagcca cagttgtggc aagatttccc 2520
   tctaacaaat taagtcccac gtgtcaatgg ctcaaaaaag tgtggcaaga ttcccttagg 2580
   cttagtaagt tgtggctaac aatttgatca cctcacctta gacaaggtgt ggcaactttt 2640
   gttggcaagt aatggtaaag tatggctggg aaccaaacag cccctaagtt ttactttgga 2700
   ctacctttaa acatatcttt tcactttgaa ctagataaat ttgctattgt tgcgatttgg 2760
   attttttttt tctcgtgcaa tcaacgacct taaacacatc agctctagta tacggccgat 2820
   ctcctctata tatggttcat atgtttgccg aaagggaagt tagacatgac gaaaagttgt 2880
   tcatggtagt ccaaaccaca acccggccca atttgaaaag ataggtttaa gggtggtcca 2940
   aattgaaact tgggtaataa aaggtggatc aaagtgcaat ttactttttt ttactgtaat 3000
   ttcttctggc tggtttgttg gtcgccgtta ggaccgggtg acgccgtcaa ccccgcgcct 3060
   ccgtattcgc tgacgtgggg tggcgcgctg gcttccgcct tgacccgaat ttgttttcct 3120
   tccgttaaaa aatggttttt cctttcttta aaaaggaaat agtttgtttt ttaagtctgt 3180
   gtattaggat tattacactt gaattttggt atatgtgtag gataatttac tgcatgttta 3240
   taatagagtt gtactataga tgaaataacc caatttttgg tataattcgt gtttggttgg 3300
   aggtcaaaat aacaggttat tttgtgaaga aaaaactccg tagtatagta ccatatccat 3360
   catgaataca catactgcct agacgagtga ttaggatgaa tccatgttat attcctcaaa 3420
   ataatataaa ccacttgatc ttatgatctt atccaatctg ttcatataaa ctggagatat 3480
   aagatggtgc atttcccttt tgatttcttt tgttgacggc catgagatag gttgcatcca 3540
   ctgcatttat attttggacc aatacaatgc acctattgat acatggggac agctcaacta 3600
   accatgatgc aaaatgctgg ttggtgacca gttcttggca ttatgataat gataggatta 3660
   aaaaaaacag tgcaatgtct cggaaagaaa ccatgacaaa gggtacatgt tgcattccag 3720
   tttctaatga taaaattatg tgccagcaat tcaaaaatca tgcgtgttcc ctacgcacca 3780
   ttctttgcaa taaacaagtg catgcacaat atgattgtgc taaggttcaa gaacttgttg 3840
   cagtggctaa gcttggcgcg cctcgcgacc acctttaatt aagtgaagag caggagcttg 3900
   catgcctgca ggctctagag gatcccccct cagaagacca gagggctatt gagacttttc 3960
   aacaaagggt aatatcggga aacctcctcg gattccattg cccagctatc tgtcacttca 4020
   tcgaaaggac agtagaaaag gaaggtggct cctacaaatg ccatcattgc gataaaggaa 4080
   aggctatcgt tcaagatgcc tctaccgaca gtggtcccaa agatggaccc ccacccacga 4140
   ggaacatcgt ggaaaaagaa gacgttccaa ccacgtcttc aaagcaagtg gattgatgtg 4200
   atatctccac tgacgtaagg gatgacgcac aatcccacta tccttcgcaa gacccttcct 4260
   ctatataagg aagttcattt catttggaga ggacaggctt cttgagatcc ttcaacaatt 4320
   accaacaaca acaaacaaca aacaacatta caattactat ttacaattac agtcgactct 4380
   agaggatcca tggtgagcaa gggcgaggag ctgttcaccg gggtggtgcc catcctggtc 4440
   gagctggacg gcgacgtaaa cggccacaag ttcagcgtgt ccggcgaggg cgagggcgat 4500
   gccacctacg gcaagctgac cctgaagttc atctgcacca ccggcaagct gcccgtgccc 4560
   tggcccaccc tcgtgaccac cttcacctac ggcgtgcagt gcttcagccg ctaccccgac 4620
   cacatgaagc agcacgactt cttcaagtcc gccatgcccg aaggctacgt ccaggagcgc 4680
   accatcttct tcaaggacga cggcaactac aagacccgcg ccgaggtgaa gttcgagggc 4740
   gacaccctgg tgaaccgcat cgagctgaag ggcatcgact tcaaggagga cggcaacatc 4800
   ctggggcaca agctggagta caactacaac agccacaacg tctatatcat ggccgacaag 4860
   cagaagaacg gcatcaaggt gaacttcaag atccgccaca acatcgagga cggcagcgtg 4920
   cagctcgccg accactacca gcagaacacc cccatcggcg acggccccgt gctgctgccc 4980
   gacaaccact acctgagcac ccagtccgcc ctgagcaaag accccaacga gaagcgcgat 5040
   cacatggtcc tgctggagtt cgtgaccgcc gccgggatca ctcacggcat ggacgagctg 5100
   tacaagtaaa gcggccgccc ggctgcagat cgttcaaaca tttggcaata aagtttctta 5160
   agattgaatc ctgttgccgg tcttgcgatg attatcatat aatttctgtt gaattacgtt 5220
   aagcatgtaa taattaacat gtaatgcatg acgttattta tgagatgggt ttttatgatt 5280
   agagtcccgc aattatacat ttaatacgcg atagaaaaca aaatatagcg cgcaaactag 5340
   gataaattat cgcgcgcggt gtcatctatg ttactagatc cgatgataag ctgtcaaaca 5400
   tgagaattcc tttcgtcgac ccacgtgttg ctgaggtatt taaataatcc gaaaagtttc 5460
   tgcaccgttt tcacccccta actaacaata tagggaacgt gtgctaaata taaaatgaga 5520
   ccttatatat gtagcgctga taactagaac tatgcaagaa aaactcatcc acctacttta 5580
   gtggcaatcg ggctaaataa aaaagagtcg ctacactagt ttcgttttcc ttagtaatta 5640
   agtgggaaaa tgaaatcatt attgcttaga atatacgttc acatctctgt catgaagtta 5700
   aattattcga ggtagccata attgtcatca aactcttctt gaataaaaaa atctttctag 5760
   ctgaactcaa tgggtaaaga gagagatttt ttttaaaaaa atagaatgaa gatattctga 5820
   acgtattggc aaagatttaa acatataatt atataatttt atagtttgtg cattcgtcat 5880
   atcgcacatc attaaggaca tgtcttactc catcccaatt tttatttagt aattaaagac 5940
   aattgactta tttttattat ttatcttttt tcgattagat gcaaggtact tacgcacaca 6000
   ctttgtgctc atgtgcatgt gtgagtgcac ctcctcaata cacgttcaac tagcaacaca 6060
   tctctaatat cactcgccta tttaatacat ttaggtagca atatctgaat tcaagcactc 6120
   caccatcacc agaccacttt taataatatc taaaatacaa aaaataattt tacagaatag 6180
   catgaaaagt atgaaacgaa ctatttaggt ttttcacata caaaaaaaaa aagaattttg 6240
   ctcgtgcgcg agcgccaatc tcccatattg ggcacacagg caacaacaga gtggctgccc 6300
   acagaacaac ccacaaaaaa cgatgatcta acggaggaca gcaagtccgc aacaaccttt 6360
   taacagcagg ctttgcggcc aggagagagg aggagaggca aagaaaacca agcatcctcc 6420
   ttctcccatc tataaattcc tccccccttt tcccctctct atataggagg catccaagcc 6480
   aagaagaggg agagcaccaa ggacacgcga ctagcagaag ccgagcgacc gccttctcga 6540
   tccatatctt ccggtcgagt tcttggtcga tctcttccct cctccacctc ctcctcacag 6600
   ggtatgtgcc tcccttcggt tgttcttgga tttattgttc taggttgtgt agtacgggcg 6660
   ttgatgttag gaaaggggat ctgtatctgt gatgattcct gttcttggat ttgggataga 6720
   ggggttcttg atgttgcatg ttatcggttc ggtttgatta gtagtatggt tttcaatcgt 6780
   ctggagagct ctatggaaat gaaatggttt agggatcgga atcttgcgat tttgtgagta 6840
   ccttttgttt gaggtaaaat cagagcaccg gtgattttgc ttggtgtaat aaagtacggt 6900
   tgtttggtcc tcgattctgg tagtgatgct tctcgatttg acgaagctat cctttgttta 6960
   ttccctattg aacaaaaata atccaacttt gaagacggtc ccgttgatga gattgaatga 7020
   ttgattctta agcctgtcca aaatttcgca gctggcttgt ttagatacag tagtccccat 7080
   cacgaaattc atggaaacag ttataatcct caggaacagg ggattccctg ttcttccgat 7140
   ttgctttagt cccagaattt tttttcccaa atatcttaaa aagtcacttt ctggttcagt 7200
   tcaatgaatt gattgctaca aataatgctt ttatagcgtt atcctagctg tagttcagtt 7260
   aataggtaat acccctatag tttagtcagg agaagaactt atccgatttc tgatctccat 7320
   ttttaattat atgaaatgaa ctgtagcata agcagtattc atttggatta ttttttttat 7380
   tagctctcac cccttcatta ttctgagctg aaagtctggc atgaactgtc ctcaattttg 7440
   ttttcaaatt cacatcgatt atctatgcat tatcctcttg tatctacctg tagaagtttc 7500
   tttttggtta ttccttgact gcttgattac agaaagaaat ttatgaagct gtaatcggga 7560
   tagttatact gcttgttctt atgattcatt tcctttgtgc agttcttggt gtagcttgcc 7620
   actttcacca gcaaagttca tttaaatcaa ctagggatat cacaagtttg tacaaaaaag 7680
   caggctggat cctacgtaag atctaccatg gaagacgcca aaaacataaa gaaaggcccg 7740
   gcgccattct atccgctgga agatggaacc gctggagagc aactgcataa ggctatgaag 7800
   agatacgccc tggttcctgg aacaattgct tttacagatg cacatatcga ggtggacatc 7860
   acttacgctg agtacttcga aatgtccgtt cggttggcag aagctatgaa acgatatggg 7920
   ctgaatacaa atcacagaat cgtcgtatgc agtgaaaact ctcttcaatt ctttatgccg 7980
   gtgttgggcg cgttatttat cggagttgca gttgcgcccg cgaacgacat ttataatgaa 8040
   cgtgaattgc tcaacagtat gggcatttcg cagcctaccg tggtgttcgt ttccaaaaag 8100
   gggttgcaaa aaattttgaa cgtgcaaaaa aagctcccaa tcatccaaaa aattattatc 8160
   atggattcta aaacggatta ccagggattt cagtcgatgt acacgttcgt cacatctcat 8220
   ctacctcccg gttttaatga atacgatttt gtgccagagt ccttcgatag ggacaagaca 8280
   attgcactga tcatgaactc ctctggatct actggtctgc ctaaaggtgt cgctctgcct 8340
   catagaactg cctgcgtgag attctcgcat gccagagatc ctatttttgg caatcaaatc 8400
   attccggata ctgcgatttt aagtgttgtt ccattccatc acggttttgg aatgtttact 8460
   acactcggat atttgatatg tggatttcga gtcgtcttaa tgtatagatt tgaagaagag 8520
   ctgtttctga ggagccttca ggattacaag attcaaagtg cgctgctggt gccaacccta 8580
   ttctccttct tcgccaaaag cactctgatt gacaaatacg atttatctaa tttacacgaa 8640
   attgcttctg gtggcgctcc cctctctaag gaagtcgggg aagcggttgc caagaggttc 8700
   catctgccag gtatcaggca aggatatggg ctcactgaga ctacatcagc tattctgatt 8760
   acacccgagg gggatgataa accgggcgcg gtcggtaaag ttgttccatt ttttgaagcg 8820
   aaggttgtgg atctggatac cgggaaaacg ctgggcgtta atcaaagagg cgaactgtgt 8880
   gtgagaggtc ctatgattat gtccggttat gtaaacaatc cggaagcgac caacgccttg 8940
   attgacaagg atggatggct acattctgga gacatagctt actgggacga agacgaacac 9000
   ttcttcatcg ttgaccgcct gaagtctctg attaagtaca aaggctatca ggtggctccc 9060
   gctgaattgg aatccatctt gctccaacac cccaacatct tcgacgcagg tgtcgcaggt 9120
   cttcccgacg atgacgccgg tgaacttccc gccgccgttg ttgttttgga gcacggaaag 9180
   acgatgacgg aaaaagagat cgtggattac gtcgccagtc aagtaacaac cgcgaaaaag 9240
   ttgcgcggag gagttgtgtt tgtggacgaa gtaccgaaag gtcttaccgg aaaactcgac 9300
   gcaagaaaaa tcagagagat cctcataaag gccaagaagg gcggaaagat cgccgtgtaa 9360
   ctcgaggctg cctatagatg ctcgtatgca atatcgtgtg ctgccagata ttgggaagcc 9420
   tctgaagcta ccagttactg ttctctatat ttgaagtcat aagactattt gttgctatta 9480
   aagcgattct tgcttgatgc aagttgtgtc ctcattatgc actaccggca tattatgagt 9540
   atggtttgtc tgggatattg tcaatctaat aaaagtactt gctatttgac tatacatctg 9600
   gttttggttc ctgtgtctgc tatcatcgtg gttacttcca acatgctggc tacctgttga 9660
   tctgtcatag taatatttca acatctggcg ccattttgaa tttccttgta tcggtattaa 9720
   ttttccgtga tgtccttgct tttttttcct atggttcaat tgtatcggag tgtaagctgt 9780
   gccgttgtgc gtcttgtccc gccggtagtg ctaggagaag gcaatttcta ctacctctcc 9840
   gtcccagaat attgagaatt ttttcctaag tacagccatc aaatttttcc tccgtcccac 9900
   ggatccaata tcttggaagc aatgtccatg tgatgtaaag attgttttct ccccaagctt 9960
   ggcgtaatca tggacccagc tttcttgtac aaagtggtga tatcacaagc ccgggcggtc 10020
   ttctagggat aacagggtaa ttatatccct ctagatcaca agcccgggcg gtcttctacg 10080
   atgattgagt aataatgtgt cacgcatcac catgggtggc agtgtcagtg tgagcaatga 10140
   cctgaatgaa caattgaaat gaaaagaaaa aaagtactcc atctgttcca aattaaaatt 10200
   ggttttaacc ttttaatagg tttatacaat aattgatata tgttttctgt atatgtctaa 10260
   tttgttatca tccgggcggt cttctaggga taacagggta attatatccc tctagacaac 10320
   acacaacaaa taagagaaaa aacaaataat attaatttga gaatgaacaa aaggaccata 10380
   tcattcatta actcttctcc atccacttcc atttcacagt tcgatagcga aaaccgaata 10440
   aaaaacacag taaattacaa gcacaacaaa tggtacaaga aaaacagttt tcccaatgcc 10500
   ataatactcg actcgagttc ctgcaggtac caaaagctta gcttgagctt ggatcagatt 10560
   gtcgtttccc gccttcagtt taaactatca gtgtttgaca ggatatattg gcgggtaaac 10620
   ctaagagaaa agagcgttta ttagaataat cggatattta aaagggcgtg aaaaggttta 10680
   tccgttcgtc catttgtatg tgcatgccaa ccacagggtt cccctcggga tcaaagtatg 10740
   aagagatcga ggcggagatg atcgcggccg ggtacgtgtt cgagccgccc gcgcacgtct 10800
   caaccgtgcg gctgcatgaa atcctggccg gtttgtctga tgccaagctg gcggcctggc 10860
   cggccagctt ggccgctgaa gaaaccgagc gccgccgtct aaaaaggtga tgtgtatttg 10920
   agtaaaacag cttgcgtcat gcggtcgctg cgtatatgat gcgatgagta aataaacaaa 10980
   tacgcaaggg gaacgcatga aggttatcgc tgtacttaac cagaaaggcg ggtcaggcaa 11040
   gacgaccatc gcaacccatc tagcccgcgc cctgcaactc gccggggccg atgttctgtt 11100
   agtcgattcc gatccccagg gcagtgcccg cgattgggcg gccgtgcggg aagatcaacc 11160
   gctaaccgtt gtcggcatcg accgcccgac gattgaccgc gacgtgaagg ccatcggccg 11220
   gcgcgacttc gtagtgatcg acggagcgcc ccaggcggcg gacttggctg tgtccgcgat 11280
   caaggcagcc gacttcgtgc tgattccggt gcagccaagc ccttacgaca tatgggccac 11340
   cgccgacctg gtggagctgg ttaagcagcg cattgaggtc acggatggaa ggctacaagc 11400
   ggcctttgtc gtgtcgcggg cgatcaaagg cacgcgcatc ggcggtgagg ttgccgaggc 11460
   gctggccggg tacgagctgc ccattcttga gtcccgtatc acgcagcgcg tgagctaccc 11520
   aggcactgcc gccgccggca caaccgttct tgaatcagaa cccgagggcg acgctgcccg 11580
   cgaggtccag gcgctggccg ctgaaattaa atcaaaactc atttgagtta atgaggtaaa 11640
   gagaaaatga gcaaaagcac aaacacgcta agtgccggcc gtccgagcgc acgcagcagc 11700
   aaggctgcaa cgttggccag cctggcagac acgccagcca tgaagcgggt caactttcag 11760
   ttgccggcgg aggatcacac caagctgaag atgtacgcgg tacgccaagg caagaccatt 11820
   accgagctgc tatctgaata catcgcgcag ctaccagagt aaatgagcaa atgaataaat 11880
   gagtagatga attttagcgg ctaaaggagg cggcatggaa aatcaagaac aaccaggcac 11940
   cgacgccgtg gaatgcccca tgtgtggagg aacgggcggt tggccaggcg taagcggctg 12000
   ggttgtctgc cggccctgca atggcactgg aacccccaag cccgaggaat cggcgtgagc 12060
   ggtcgcaaac catccggccc ggtacaaatc ggcgcggcgc tgggtgatga cctggtggag 12120
   aagttgaagg ccgcgcaggc cgcccagcgg caacgcatcg aggcagaagc acgccccggt 12180
   gaatcgtggc aagcggccgc tgatcgaatc cgcaaagaat cccggcaacc gccggcagcc 12240
   ggtgcgccgt cgattaggaa gccgcccaag ggcgacgagc aaccagattt tttcgttccg 12300
   atgctctatg acgtgggcac ccgcgatagt cgcagcatca tggacgtggc cgttttccgt 12360
   ctgtcgaagc gtgaccgacg agctggcgag gtgatccgct acgagcttcc agacgggcac 12420
   gtagaggttt ccgcagggcc ggccggcatg gccagtgtgt gggattacga cctggtactg 12480
   atggcggttt cccatctaac cgaatccatg aaccgatacc gggaagggaa gggagacaag 12540
   cccggccgcg tgttccgtcc acacgttgcg gacgtactca agttctgccg gcgagccgat 12600
   ggcggaaagc agaaagacga cctggtagaa acctgcattc ggttaaacac cacgcacgtt 12660
   gccatgcagc gtacgaagaa ggccaagaac ggccgcctgg tgacggtatc cgagggtgaa 12720
   gccttgatta gccgctacaa gatcgtaaag agcgaaaccg ggcggccgga gtacatcgag 12780
   atcgagctag ctgattggat gtaccgcgag atcacagaag gcaagaaccc ggacgtgctg 12840
   acggttcacc ccgattactt tttgatcgat cccggcatcg gccgttttct ctaccgcctg 12900
   gcacgccgcg ccgcaggcaa ggcagaagcc agatggttgt tcaagacgat ctacgaacgc 12960
   agtggcagcg ccggagagtt caagaagttc tgtttcaccg tgcgcaagct gatcgggtca 13020
   aatgacctgc cggagtacga tttgaaggag gaggcggggc aggctggccc gatcctagtc 13080
   atgcgctacc gcaacctgat cgagggcgaa gcatccgccg gttcctaatg tacggagcag 13140
   atgctagggc aaattgccct agcaggggaa aaaggtcgaa aaggtctctt tcctgtggat 13200
   agcacgtaca ttgggaaccc aaagccgtac attgggaacc ggaacccgta cattgggaac 13260
   ccaaagccgt acattgggaa ccggtcacac atgtaagtga ctgatataaa agagaaaaaa 13320
   ggcgattttt ccgcctaaaa ctctttaaaa cttattaaaa ctcttaaaac ccgcctggcc 13380
   tgtgcataac tgtctggcca gcgcacagcc gaagagctgc aaaaagcgcc tacccttcgg 13440
   tcgctgcgct ccctacgccc cgccgcttcg cgtcggccta tcgcggccgc tggccgctca 13500
   aaaatggctg gcctacggcc aggcaatcta ccagggcgcg gacaagccgc gccgtcgcca 13560
   ctcgaccgcc ggcgcccaca tcaaggcacc ctgcctcgcg cgtttcggtg atgacggtga 13620
   aaacctctga cacatgcagc tcccggagac ggtcacagct tgtctgtaag cggatgccgg 13680
   gagcagacaa gcccgtcagg gcgcgtcagc gggtgttggc gggtgtcggg gcgcagccat 13740
   gacccagtca cgtagcgata gcggagtgta tactggctta actatgcggc atcagagcag 13800
   attgtactga gagtgcacca tatgcggtgt gaaataccgc acagatgcgt aaggagaaaa 13860
   taccgcatca ggcgctcttc cgcttcctcg ctcactgact cgctgcgctc ggtcgttcgg 13920
   ctgcggcgag cggtatcagc tcactcaaag gcggtaatac ggttatccac agaatcaggg 13980
   gataacgcag gaaagaacat gtgagcaaaa ggccagcaaa aggccaggaa ccgtaaaaag 14040
   gccgcgttgc tggcgttttt ccataggctc cgcccccctg acgagcatca caaaaatcga 14100
   cgctcaagtc agaggtggcg aaacccgaca ggactataaa gataccaggc gtttccccct 14160
   ggaagctccc tcgtgcgctc tcctgttccg accctgccgc ttaccggata cctgtccgcc 14220
   tttctccctt cgggaagcgt ggcgctttct catagctcac gctgtaggta tctcagttcg 14280
   gtgtaggtcg ttcgctccaa gctgggctgt gtgcacgaac cccccgttca gcccgaccgc 14340
   tgcgccttat ccggtaacta tcgtcttgag tccaacccgg taagacacga cttatcgcca 14400
   ctggcagcag ccactggtaa caggattagc agagcgaggt atgtaggcgg tgctacagag 14460
   ttcttgaagt ggtggcctaa ctacggctac actagaagga cagtatttgg tatctgcgct 14520
   ctgctgaagc cagttacctt cggaaaaaga gttggtagct cttgatccgg caaacaaacc 14580
   accgctggta gcggtggttt ttttgtttgc aagcagcaga ttacgcgcag aaaaaaagga 14640
   tctcaagaag atcctttgat cttttctacg gggtctgacg cttagtggaa cgaaaactca 14700
   cgttaaggga ttttggtcat gcatgatata tctcccaatt tgtgtagggc ttattatgca 14760
   cgcttaaaaa taataaaagc agacttgacc tgatagtttg gctgtgagca attatgtgct 14820
   tagtgcatct aacgcttgag ttaagccgcg ccgcgaagcg gcgtcggctt gaacgaattt 14880
   ctagctagac attatttgcc gactaccttg gtgatctcgc ctttcacgta gtggacaaat 14940
   tcttccaact gatctgcgcg cgaggccaag cgatcttctt cttgtccaag ataagcctgt 15000
   ctagcttcaa gtatgacggg ctgatactgg gccggcaggc gctccattgc ccagtcggca 15060
   gcgacatcct tcggcgcgat tttgccggtt actgcgctgt accaaatgcg ggacaacgta 15120
   agcactacat ttcgctcatc gccagcccag tcgggcggcg agttccatag cgttaaggtt 15180
   tcatttagcg cctcaaatag atcctgttca ggaaccggat caaagagttc ctccgccgct 15240
   ggacctacca aggcaacgct atgttctctt gcttttgtca gcaagatagc cagatcaatg 15300
   tcgatcgtgg ctggctcgaa gatacctgca agaatgtcat tgcgctgcca ttctccaaat 15360
   tgcagttcgc gcttagctgg ataacgccac ggaatgatgt cgtcgtgcac aacaatggtg 15420
   acttctacag cgcggagaat ctcgctctct ccaggggaag ccgaagtttc caaaaggtcg 15480
   ttgatcaaag ctcgccgcgt tgtttcatca agccttacgg tcaccgtaac cagcaaatca 15540
   atatcactgt gtggcttcag gccgccatcc actgcggagc cgtacaaatg tacggccagc 15600
   aacgtcggtt cgagatggcg ctcgatgacg ccaactacct ctgatagttg agtcgatact 15660
   tcggcgatca ccgcttcccc catgatgttt aactttgttt tagggcgact gccctgctgc 15720
   gtaacatcgt tgctgctcca taacatcaaa catcgaccca cggcgtaacg cgcttgctgc 15780
   ttggatgccc gaggcataga ctgtacccca aaaaaacagt cataacaagc catgaaaacc 15840
   gccactgcgc cgttaccacc gctgcgttcg gtcaaggttc tggaccagtt gcgtgagcgc 15900
   atacgctact tgcattacag cttacgaacc gaacaggctt atgtccactg ggttcgtgcc 15960
   cgaattgatc a 15971

<210> 98
<211> 15620
<212> DNA
<213> artificial

<220>
<223>  vector sequence

<400>  98
caggcagcaa cgctctgtca tcgttacaat caacatgcta ccctccgcga gatcatccgt      60

gtttcaaacc cggcagctta gttgccgttc ttccgaatag catcggtaac atgagcaaag     120

tctgccgcct tacaacggct ctcccgctga cgccgtcccg gactgatggg ctgcctgtat     180

cgagtggtga ttttgtgccg agctgccggt cggggagctg ttggctggct ggtggcagga     240

tatattgtgg tgtaaacaaa ttgacgctta gacaacttaa taacacattg cggacgtttt     300

taatgtactg aattaacgcc gaattgaatt caagagctca aggatcctaa ctataacggt     360

cctaaggtag cgaaggcgcg ccgaattcga ggggatcgag cccctgctga gcctcgacat     420

gttgtcgcaa aattcgccct ggacccgccc aacgatttgt cgtcactgtc aaggtttgac     480

ctgcacttca tttggggccc acatacacca aaaaaatgct gcataattct cggggcagca     540

agtcggttac ccggccgccg tgctggaccg ggttgaatgg tgcccgtaac tttcggtaga     600

gcggacggcc aatactcaac ttcaaggaat ctcacccatg cgcgccggcg gggaaccgga     660

gttcccttca gtgagcgtta ttagttcgcc gctcggtgtg tcgtagatac tagcccctgg     720

ggcacttttg aaatttgaat aagatttatg taatcagtct tttaggtttg accggttctg     780

ccgctttttt taaaattgga tttgtaataa taaaacgcaa ttgtttgtta ttgtggcgct     840

ctatcataga tgtcgctata aacctattca gcacaatata ttgttttcat tttaatattg     900

tacatataag tagtagggta caatcagtaa attgaacgga gaatattatt cataaaaata     960

cgatagtaac gggtgatata ttcattagaa tgaaccgaaa ccggcggtaa ggatctgagc    1020

tacacatgct caggtttttt acaacgtgca caacagaatt gaaagcaaat atcatgcgat    1080

cataggcgtc tcgcatatct cattaaagca gggggtgggc gaagaactcc agcatgagat    1140

ccccgcgctg gaggatcatc cagccggcgt cccggaaaac gattccgaag cccaaccttt    1200

catagaaggc ggcggtggaa tcgaaatctc gtgatggcag gttgggcgtc gcttggtcgg    1260

tcatttcgaa ccccagagtc ccgctcagaa gaactcgtca agaaggcgat agaaggcgat    1320

gcgctgcgaa tcgggagcgg cgataccgta aagcacgagg aagcggtcag cccattcgcc    1380

gccaagctct tcagcaatat cacgggtagc caacgctatg tcctgatagc ggtccgccac    1440

acccagccgg ccacagtcga tgaatccaga aaagcggcca ttttccacca tgatattcgg    1500

caagcaggca tcgccatggg tcacgacgag atcctcgccg tcgggcatgc gcgccttgag    1560

cctggcgaac agttcggctg gcgcgagccc ctgatgctct tcgtccagat catcctgatc    1620

gacaagaccg gcttccatcc gagtacgtgc tcgctcgatg cgatgtttcg cttggtggtc    1680

gaatgggcag gtagccggat caagcgtatg cagccgccgc attgcatcag ccatgatgga    1740

tactttctcg gcaggagcaa ggtgagatga caggagatcc tgccccggca cttcgcccaa    1800

tagcagccag tcccttcccg cttcagtgac aacgtcgagc acagctgcgc aaggaacgcc 1860

cgtcgtggcc agccacgata gccgcgctgc ctcgtcctgc agttcattca gggcaccgga 1920

caggtcggtc ttgacaaaaa gaaccgggcg cccctgcgct gacagccgga acacggcggc 1980

atcagagcag ccgattgtct gttgtgccca gtcatagccg aatagcctct ccacccaagc 2040

ggccggagaa cctgcgtgca atccatcttg ttcaatccac atgatcaaac gttttgagga 2100

cgcgagagga ttcgattcga cgacgagagc ctcgcgagat tggggagaaa tttttcgggg 2160

gtggagctga tgcgaggaga ggagatgagg gggctggtat ttatggcggt tgggtggtgg 2220

gaggagtccc gtgccgtgac gtctccgtct gcttggagaa tccgccacgc tgaaaccacc 2280

gcggtttccg ggaagacgag gcgggcgagc gagcggttgg gaaatttcga gaagatgccg 2340

tttgtctccg tttggtacac gtctcgttga ttttttttta gtgaattacg ctttggacca 2400

cattttatta tctaagggtg tgtttggttg taagccacac tttgccacag tttgccacgc 2460

ctaaggttag gcaaatttga caggtgtttg gttgtagcca cagttgtggc aagatttccc 2520

tctaacaaat taagtcccac gtgtcaatgg ctcaaaaaag tgtggcaaga ttcccttagg 2580

cttagtaagt tgtggctaac aatttgatca cctcacctta gacaaggtgt ggcaactttt 2640

gttggcaagt aatggtaaag tatggctggg aaccaaacag cccctaagtt ttactttgga 2700

ctacctttaa acatatcttt tcactttgaa ctagataaat ttgctattgt tgcgatttgg 2760

attttttttt tctcgtgcaa tcaacgacct taaacacatc agctctagta tacggccgat 2820

ctcctctata tatggttcat atgtttgccg aaagggaagt tagacatgac gaaaagttgt 2880

tcatggtagt ccaaaccaca acccggccca atttgaaaag ataggtttaa gggtggtcca 2940

aattgaaact tgggtaataa aaggtggatc aaagtgcaat ttactttttt ttactgtaat 3000

ttcttctggc tggtttgttg gtcgccgtta ggaccgggtg acgccgtcaa ccccgcgcct 3060

ccgtattcgc tgacgtgggg tggcgcgctg gcttccgcct tgacccgaat ttgttttcct 3120

tccgttaaaa aaatggtttt ccttttctta aaaaggaaat agtttgtttt ttaagtctgt 3180

gtattaggat tattacactt gaattttggt atatgtgtag gataatttac tgcatgttta 3240

taatagagtt gtactataga tgaaataacc caatttttgg tataattcgt gtttggttgg 3300

aggtcaaaat aacaggttat tttgtgaaga aaaaactccg tagtatagta ccatatccat 3360

catgaataca catactgcct agacgagtga ttaggatgaa tccatgttat attcctcaaa 3420

ataatataaa ccacttgatc ttatgatctt atccaatctg ttcatataaa ctggagatat 3480

aagatggtgc atttcccttt tgatttcttt tgttgacggc catgagatag gttgcatcca 3540

ctgcatttat attttggacc aatacaatgc acctattgat acatggggac agctcaacta 3600

accatgatgc aaaatgctgg ttggtgacca gttcttggca ttatgataat gataggatta 3660

aaaaaaacag tgcaatgtct cggaaagaaa ccatgacaaa gggtacatgt tgcattccag 3720
tttctaatga taaaattatg tgccagcaat tcaaaaatca tgcgtgttcc ctacgcacca 3780
ttctttgcaa taaacaagtg catgcacaat atgattgtgc taaggttcaa gaacttgttg 3840
cagtggctaa gcttggcgcg cctcgcgacc acctttaatt aagtgaagag caggagcttg 3900
catgcctgca ggctctagag gatcccccct cagaagacca gagggctatt gagacttttc 3960
aacaaagggt aatatcggga aacctcctcg gattccattg cccagctatc tgtcacttca 4020
tcgaaaggac agtagaaaag gaaggtggct cctacaaatg ccatcattgc gataaaggaa 4080
aggctatcgt tcaagatgcc tctaccgaca gtggtcccaa agatggaccc ccacccacga 4140
ggaacatcgt ggaaaaagaa gacgttccaa ccacgtcttc aaagcaagtg gattgatgtg 4200
atatctccac tgacgtaagg gatgacgcac aatcccacta tccttcgcaa gacccttcct 4260
ctatataagg aagttcattt catttggaga ggacaggctt cttgagatcc ttcaacaatt 4320
accaacaaca acaaacaaca aacaacatta caattactat ttacaattac agtcgactct 4380
agaggatcca tggtgagcaa gggcgaggag ctgttcaccg gggtggtgcc catcctggtc 4440
gagctggacg gcgacgtaaa cggccacaag ttcagcgtgt ccggcgaggg cgagggcgat 4500
gccacctacg gcaagctgac cctgaagttc atctgcacca ccggcaagct gcccgtgccc 4560
tggcccaccc tcgtgaccac cttcacctac ggcgtgcagt gcttcagccg ctaccccgac 4620
cacatgaagc agcacgactt cttcaagtcc gccatgcccg aaggctacgt ccaggagcgc 4680
accatcttct tcaaggacga cggcaactac aagacccgcg ccgaggtgaa gttcgagggc 4740
gacaccctgg tgaaccgcat cgagctgaag ggcatcgact tcaaggagga cggcaacatc 4800
ctggggcaca agctggagta caactacaac agccacaacg tctatatcat ggccgacaag 4860
cagaagaacg gcatcaaggt gaacttcaag atccgccaca acatcgagga cggcagcgtg 4920
cagctcgccg accactacca gcagaacacc cccatcggcg acggccccgt gctgctgccc 4980
gacaaccact acctgagcac ccagtccgcc ctgagcaaag accccaacga gaagcgcgat 5040
cacatggtcc tgctggagtt cgtgaccgcc gccgggatca ctcacggcat ggacgagctg 5100
tacaagtaaa gcggccgccc ggctgcagat cgttcaaaca tttggcaata aagtttctta 5160
agattgaatc ctgttgccgg tcttgcgatg attatcatat aatttctgtt gaattacgtt 5220
aagcatgtaa taattaacat gtaatgcatg acgttattta tgagatgggt ttttatgatt 5280
agagtcccgc aattatacat ttaatacgcg atagaaaaca aaatatagcg cgcaaactag 5340
gataaattat cgcgcgcggt gtcatctatg ttactagatc cgatgataag ctgtcaaaca 5400
tgagaattcc tttcgtcgac ccacgtgttg ctgaggtatt taaataatcc gaaaagtttc 5460
tgcaccgttt tcacccccta actaacaata tagggaacgt gtgctaaata taaaatgaga 5520
ccttatatat gtagcgctga taactagaac tatgcaagaa aaactcatcc acctacttta 5580

gtggcaatcg ggctaaataa aaaagagtcg ctacactagt ttcgttttcc ttagtaatta 5640

agtgggaaaa tgaaatcatt attgcttaga atatacgttc acatctctgt catgaagtta 5700

aattattcga ggtagccata attgtcatca aactcttctt gaataaaaaa atctttctag 5760

ctgaactcaa tgggtaaaga gagagatttt ttttaaaaaa atagaatgaa gatattctga 5820

acgtattggc aaagatttaa acatataatt atataatttt atagtttgtg cattcgtcat 5880

atcgcacatc attaaggaca tgtcttactc catcccaatt tttatttagt aattaaagac 5940

aattgactta tttttattat ttatcttttt tcgattagat gcaaggtact tacgcacaca 6000

ctttgtgctc atgtgcatgt gtgagtgcac ctcctcaata cacgttcaac tagcaacaca 6060

tctctaatat cactcgccta tttaatacat ttaggtagca atatctgaat tcaagcactc 6120

caccatcacc agaccacttt taataatatc taaaatacaa aaaataattt tacagaatag 6180

catgaaaagt atgaaacgaa ctatttaggt ttttcacata caaaaaaaaa aagaattttg 6240

ctcgtgcgcg agcgccaatc tcccatattg ggcacacagg caacaacaga gtggctgccc 6300

acagaacaac ccacaaaaaa cgatgatcta acggaggaca gcaagtccgc aacaaccttt 6360

taacagcagg ctttgcggcc aggagagagg aggagaggca aagaaaacca agcatcctcc 6420

ttctcccatc tataaattcc tccccccttt tcccctctct atataggagg catccaagcc 6480

aagaagaggg agagcaccaa ggacacgcga ctagcagaag ccgagcgacc gccttctcga 6540

tccatatctt ccggtcgagt tcttggtcga tctcttccct cctccacctc ctcctcacag 6600

ggtatgtgcc tcccttcggt tgttcttgga tttattgttc taggttgtgt agtacgggcg 6660

ttgatgttag gaaaggggat ctgtatctgt gatgattcct gttcttggat ttgggataga 6720

ggggttcttg atgttgcatg ttatcggttc ggtttgatta gtagtatggt tttcaatcgt 6780

ctggagagct ctatggaaat gaaatggttt agggatcgga atcttgcgat tttgtgagta 6840

ccttttgttt gaggtaaaat cagagcaccg gtgattttgc ttggtgtaat aaagtacggt 6900

tgtttggtcc tcgattctgg tagtgatgct tctcgatttg acgaagctat cctttgttta 6960

ttccctattg aacaaaaata atccaacttt gaagacggtc ccgttgatga gattgaatga 7020

ttgattctta agcctgtcca aaatttcgca gctggcttgt ttagatacag tagtccccat 7080

cacgaaattc atggaaacag ttataatcct caggaacagg ggattccctg ttcttccgat 7140

ttgctttagt cccagaattt tttttcccaa atatcttaaa aagtcacttt ctggttcagt 7200

tcaatgaatt gattgctaca aataatgctt ttatagcgtt atcctagctg tagttcagtt 7260

aataggtaat acccctatag tttagtcagg agaagaactt atccgatttc tgatctccat 7320

ttttaattat atgaaatgaa ctgtagcata agcagtattc atttggatta ttttttttat 7380

tagctctcac cccttcatta ttctgagctg aaagtctggc atgaactgtc ctcaattttg 7440

ttttcaaatt cacatcgatt atctatgcat tatcctcttg tatctacctg tagaagtttc 7500

tttttggtta ttccttgact gcttgattac agaaagaaat ttatgaagct gtaatcggga 7560

tagttatact gcttgttctt atgattcatt tcctttgtgc agttcttggt gtagcttgcc 7620

actttcacca gcaaagttca tttaaatcaa ctagggatat cacaagtttg tacaaaaaag 7680

caggctggat cctacgtaag atctaccatg gaagacgcca aaaacataaa gaaaggcccg 7740

gcgccattct atccgctgga agatggaacc gctggagagc aactgcataa ggctatgaag 7800

agatacgccc tggttcctgg aacaattgct tttacagatg cacatatcga ggtggacatc 7860

acttacgctg agtacttcga aatgtccgtt cggttggcag aagctatgaa acgatatggg 7920

ctgaatacaa atcacagaat cgtcgtatgc agtgaaaact ctcttcaatt ctttatgccg 7980

gtgttgggcg cgttatttat cggagttgca gttgcgcccg cgaacgacat ttataatgaa 8040

cgtgaattgc tcaacagtat gggcatttcg cagcctaccg tggtgttcgt ttccaaaaag 8100

gggttgcaaa aaattttgaa cgtgcaaaaa aagctcccaa tcatccaaaa aattattatc 8160

atggattcta aaacggatta ccagggattt cagtcgatgt acacgttcgt cacatctcat 8220

ctacctcccg gttttaatga atacgatttt gtgccagagt ccttcgatag ggacaagaca 8280

attgcactga tcatgaactc ctctggatct actggtctgc ctaaaggtgt cgctctgcct 8340

catagaactg cctgcgtgag attctcgcat gccagagatc ctatttttgg caatcaaatc 8400

attccggata ctgcgatttt aagtgttgtt ccattccatc acggttttgg aatgtttact 8460

acactcggat atttgatatg tggatttcga gtcgtcttaa tgtatagatt tgaagaagag 8520

ctgtttctga ggagccttca ggattacaag attcaaagtg cgctgctggt gccaacccta 8580

ttctccttct tcgccaaaag cactctgatt gacaaatacg atttatctaa tttacacgaa 8640

attgcttctg gtggcgctcc cctctctaag gaagtcgggg aagcggttgc caagaggttc 8700

catctgccag gtatcaggca aggatatggg ctcactgaga ctacatcagc tattctgatt 8760

acacccgagg gggatgataa accgggcgcg gtcggtaaag ttgttccatt ttttgaagcg 8820

aaggttgtgg atctggatac cgggaaaacg ctgggcgtta atcaaagagg cgaactgtgt 8880

gtgagaggtc ctatgattat gtccggttat gtaaacaatc cggaagcgac caacgccttg 8940

attgacaagg atggatggct acattctgga gacatagctt actgggacga agacgaacac 9000

ttcttcatcg ttgaccgcct gaagtctctg attaagtaca aaggctatca ggtggctccc 9060

gctgaattgg aatccatctt gctccaacac cccaacatct tcgacgcagg tgtcgcaggt 9120

cttcccgacg atgacgccgg tgaacttccc gccgccgttg ttgttttgga gcacggaaag 9180

acgatgacgg aaaaagagat cgtggattac gtcgccagtc aagtaacaac cgcgaaaaag 9240

ttgcgcggag gagttgtgtt tgtggacgaa gtaccgaaag gtcttaccgg aaaactcgac 9300

gcaagaaaaa tcagagagat cctcataaag gccaagaagg gcggaaagat cgccgtgtaa 9360

ctcgagcatg catctagagg gcccgctagc gttaaccctg ctttaatgag atatgcgaga 9420

cgcctatgat cgcatgatat ttgctttcaa ttctgttgtg cacgttgtaa aaaacctgag 9480

catgtgtagc tcagatcctt accgccggtt tcggttcatt ctaatgaata tatcacccgt 9540

tactatcgta tttttatgaa taatattctc cgttcaattt actgattgtc cgtcgacgaa 9600

ttcaagcttg gcgtaatcat ggacccagct ttcttgtaca aagtggtgat atcacaagcc 9660

cgggcggtct tctagggata acagggtaat tatatccctc tagatcacaa gcccgggcgg 9720

tcttctacga tgattgagta ataatgtgtc acgcatcacc atgggtggca gtgtcagtgt 9780

gagcaatgac ctgaatgaac aattgaaatg aaaagaaaaa aagtactcca tctgttccaa 9840

attaaaattg gttttaacct tttaataggt ttatacaata attgatatat gttttctgta 9900

tatgtctaat ttgttatcat ccgggcggtc ttctagggat aacagggtaa ttatatccct 9960

ctagacaaca cacaacaaat aagagaaaaa acaaataata ttaatttgag aatgaacaaa 10020

aggaccatat cattcattaa ctcttctcca tccacttcca tttcacagtt cgatagcgaa 10080

aaccgaataa aaaacacagt aaattacaag cacaacaaat ggtacaagaa aaacagtttt 10140

cccaatgcca taatactcga ctcgagttcc tgcaggtacc aaaagcttag cttgagcttg 10200

gatcagattg tcgtttcccg ccttcagttt aaactatcag tgtttgacag gatatattgg 10260

cgggtaaacc taagagaaaa gagcgtttat tagaataatc ggatatttaa aagggcgtga 10320

aaaggtttat ccgttcgtcc atttgtatgt gcatgccaac cacagggttc ccctcgggat 10380

caaagtatga agagatcgag gcggagatga tcgcggccgg gtacgtgttc gagccgcccg 10440

cgcacgtctc aaccgtgcgg ctgcatgaaa tcctggccgg tttgtctgat gccaagctgg 10500

cggcctggcc ggccagcttg gccgctgaag aaaccgagcg ccgccgtcta aaaaggtgat 10560

gtgtatttga gtaaaacagc ttgcgtcatg cggtcgctgc gtatatgatg cgatgagtaa 10620

ataaacaaat acgcaagggg aacgcatgaa ggttatcgct gtacttaacc agaaaggcgg 10680

gtcaggcaag acgaccatcg caacccatct agcccgcgcc ctgcaactcg ccggggccga 10740

tgttctgtta gtcgattccg atccccaggg cagtgcccgc gattgggcgg ccgtgcggga 10800

agatcaaccg ctaaccgttg tcggcatcga ccgcccgacg attgaccgcg acgtgaaggc 10860

catcggccgg cgcgacttcg tagtgatcga cggagcgccc caggcggcgg acttggctgt 10920

gtccgcgatc aaggcagccg acttcgtgct gattccggtg cagccaagcc cttacgacat 10980

atgggccacc gccgacctgg tggagctggt taagcagcgc attgaggtca cggatggaag 11040

gctacaagcg gcctttgtcg tgtcgcgggc gatcaaaggc acgcgcatcg gcggtgaggt 11100

tgccgaggcg ctggccgggt acgagctgcc cattcttgag tcccgtatca cgcagcgcgt 11160

gagctaccca ggcactgccg ccgccggcac aaccgttctt gaatcagaac ccgagggcga 11220

cgctgcccgc gaggtccagg cgctggccgc tgaaattaaa tcaaaactca tttgagttaa 11280

tgaggtaaag agaaaatgag caaaagcaca aacacgctaa gtgccggccg tccgagcgca 11340

cgcagcagca aggctgcaac gttggccagc ctggcagaca cgccagccat gaagcgggtc 11400

aactttcagt tgccggcgga ggatcacacc aagctgaaga tgtacgcggt acgccaaggc 11460

aagaccatta ccgagctgct atctgaatac atcgcgcagc taccagagta aatgagcaaa 11520

tgaataaatg agtagatgaa ttttagcggc taaaggaggc ggcatggaaa atcaagaaca 11580

accaggcacc gacgccgtgg aatgccccat gtgtggagga acgggcggtt ggccaggcgt 11640

aagcggctgg gttgtctgcc ggccctgcaa tggcactgga acccccaagc ccgaggaatc 11700

ggcgtgagcg gtcgcaaacc atccggcccg gtacaaatcg gcgcggcgct gggtgatgac 11760

ctggtggaga agttgaaggc cgcgcaggcc gcccagcggc aacgcatcga ggcagaagca 11820

cgccccggtg aatcgtggca agcggccgct gatcgaatcc gcaaagaatc ccggcaaccg 11880

ccggcagccg gtgcgccgtc gattaggaag ccgcccaagg gcgacgagca accagatttt 11940

ttcgttccga tgctctatga cgtgggcacc cgcgatagtc gcagcatcat ggacgtggcc 12000

gttttccgtc tgtcgaagcg tgaccgacga gctggcgagg tgatccgcta cgagcttcca 12060

gacgggcacg tagaggtttc cgcagggccg gccggcatgg ccagtgtgtg ggattacgac 12120

ctggtactga tggcggtttc ccatctaacc gaatccatga accgataccg ggaagggaag 12180

ggagacaagc ccggccgcgt gttccgtcca cacgttgcgg acgtactcaa gttctgccgg 12240

cgagccgatg gcggaaagca gaaagacgac ctggtagaaa cctgcattcg gttaaacacc 12300

acgcacgttg ccatgcagcg tacgaagaag gccaagaacg gccgcctggt gacggtatcc 12360

gagggtgaag ccttgattag ccgctacaag atcgtaaaga gcgaaaccgg gcggccggag 12420

tacatcgaga tcgagctagc tgattggatg taccgcgaga tcacagaagg caagaacccg 12480

gacgtgctga cggttcaccc cgattacttt ttgatcgatc ccggcatcgg ccgttttctc 12540

taccgcctgg cacgccgcgc cgcaggcaag gcagaagcca gatggttgtt caagacgatc 12600

tacgaacgca gtggcagcgc cggagagttc aagaagttct gtttcaccgt gcgcaagctg 12660

atcgggtcaa atgacctgcc ggagtacgat ttgaaggagg aggcggggca ggctggcccg 12720

atcctagtca tgcgctaccg caacctgatc gagggcgaag catccgccgg ttcctaatgt 12780

acggagcaga tgctagggca aattgcccta gcaggggaaa aaggtcgaaa aggtctcttt 12840

cctgtggata gcacgtacat tgggaaccca aagccgtaca ttgggaaccg gaacccgtac 12900

attgggaacc caaagccgta cattgggaac cggtcacaca tgtaagtgac tgatataaaa 12960

gagaaaaaag gcgatttttc cgcctaaaac tctttaaaac ttattaaaac tcttaaaacc 13020

cgcctggcct gtgcataact gtctggccag cgcacagccg aagagctgca aaaagcgcct 13080

acccttcggt cgctgcgctc cctacgcccc gccgcttcgc gtcggcctat cgcggccgct 13140

ggccgctcaa aaatggctgg cctacggcca ggcaatctac cagggcgcgg acaagccgcg 13200

ccgtcgccac tcgaccgccg gcgcccacat caaggcaccc tgcctcgcgc gtttcggtga 13260

tgacggtgaa aacctctgac acatgcagct cccggagacg gtcacagctt gtctgtaagc 13320

ggatgccggg agcagacaag cccgtcaggg cgcgtcagcg ggtgttggcg ggtgtcgggg 13380

cgcagccatg acccagtcac gtagcgatag cggagtgtat actggcttaa ctatgcggca 13440

tcagagcaga ttgtactgag agtgcaccat atgcggtgtg aaataccgca cagatgcgta 13500

aggagaaaat accgcatcag gcgctcttcc gcttcctcgc tcactgactc gctgcgctcg 13560

gtcgttcggc tgcggcgagc ggtatcagct cactcaaagg cggtaatacg gttatccaca 13620

gaatcagggg ataacgcagg aaagaacatg tgagcaaaag gccagcaaaa ggccaggaac 13680

cgtaaaaagg ccgcgttgct ggcgtttttc cataggctcc gcccccctga cgagcatcac 13740

aaaaatcgac gctcaagtca gaggtggcga aacccgacag gactataaag ataccaggcg 13800

tttccccctg gaagctccct cgtgcgctct cctgttccga ccctgccgct taccggatac 13860

ctgtccgcct ttctcccttc gggaagcgtg gcgctttctc atagctcacg ctgtaggtat 13920

ctcagttcgg tgtaggtcgt tcgctccaag ctgggctgtg tgcacgaacc ccccgttcag 13980

cccgaccgct gcgccttatc cggtaactat cgtcttgagt ccaacccggt aagacacgac 14040

ttatcgccac tggcagcagc cactggtaac aggattagca gagcgaggta tgtaggcggt 14100

gctacagagt tcttgaagtg gtggcctaac tacggctaca ctagaaggac agtatttggt 14160

atctgcgctc tgctgaagcc agttaccttc ggaaaaagag ttggtagctc ttgatccggc 14220

aaacaaacca ccgctggtag cggtggtttt tttgtttgca agcagcagat tacgcgcaga 14280

aaaaaaggat ctcaagaaga tcctttgatc ttttctacgg ggtctgacgc ttagtggaac 14340

gaaaactcac gttaagggat tttggtcatg catgatatat ctcccaattt gtgtagggct 14400

tattatgcac gcttaaaaat aataaaagca gacttgacct gatagtttgg ctgtgagcaa 14460

ttatgtgctt agtgcatcta acgcttgagt taagccgcgc cgcgaagcgg cgtcggcttg 14520

aacgaatttc tagctagaca ttatttgccg actaccttgg tgatctcgcc tttcacgtag 14580

tggacaaatt cttccaactg atctgcgcgc gaggccaagc gatcttcttc ttgtccaaga 14640

taagcctgtc tagcttcaag tatgacgggc tgatactggg ccggcaggcg ctccattgcc 14700

cagtcggcag cgacatcctt cggcgcgatt ttgccggtta ctgcgctgta ccaaatgcgg 14760

gacaacgtaa gcactacatt tcgctcatcg ccagcccagt cgggcggcga gttccatagc 14820

gttaaggttt catttagcgc ctcaaataga tcctgttcag gaaccggatc aaagagttcc 14880

tccgccgctg gacctaccaa ggcaacgcta tgttctcttg cttttgtcag caagatagcc 14940

agatcaatgt cgatcgtggc tggctcgaag atacctgcaa gaatgtcatt gcgctgccat 15000

tctccaaatt gcagttcgcg cttagctgga taacgccacg gaatgatgtc gtcgtgcaca 15060

acaatggtga cttctacagc gcggagaatc tcgctctctc caggggaagc cgaagtttcc 15120

aaaaggtcgt tgatcaaagc tcgccgcgtt gtttcatcaa gccttacggt caccgtaacc 15180

agcaaatcaa tatcactgtg tggcttcagg ccgccatcca ctgcggagcc gtacaaatgt 15240

acggccagca acgtcggttc gagatggcgc tcgatgacgc caactacctc tgatagttga 15300

gtcgatactt cggcgatcac cgcttccccc atgatgttta actttgtttt agggcgactg 15360

ccctgctgcg taacatcgtt gctgctccat aacatcaaac atcgacccac ggcgtaacgc 15420

gcttgctgct tggatgcccg aggcatagac tgtaccccaa aaaaacagtc ataacaagcc 15480

atgaaaaccg ccactgcgcc gttaccaccg ctgcgttcgg tcaaggttct ggaccagttg 15540

cgtgagcgca tacgctactt gcattacagc ttacgaaccg aacaggctta tgtccactgg 15600

gttcgtgccc gaattgatca 15620

<210> 99
<211> 15665
<212> DNA
<213> artificial

<220>
<223> vector sequence

<400> 99
caggcagcaa cgctctgtca tcgttacaat caacatgcta ccctccgcga gatcatccgt 60

gtttcaaacc cggcagctta gttgccgttc ttccgaatag catcggtaac atgagcaaag 120

tctgccgcct tacaacggct ctcccgctga cgccgtcccg gactgatggg ctgcctgtat 180

cgagtggtga ttttgtgccg agctgccggt cggggagctg ttggctggct ggtggcagga 240

tatattgtgg tgtaaacaaa ttgacgctta gacaacttaa taacacattg cggacgtttt 300

taatgtactg aattaacgcc gaattgaatt caagagctca aggatcctaa ctataacggt 360

cctaaggtag cgaaggcgcg ccgaattcga ggggatcgag cccctgctga gcctcgacat 420

gttgtcgcaa aattcgccct ggacccgccc aacgatttgt cgtcactgtc aaggtttgac 480

ctgcacttca tttggggccc acatacacca aaaaaatgct gcataattct cggggcagca 540

agtcggttac ccggccgccg tgctggaccg ggttgaatgg tgcccgtaac tttcggtaga 600

gcggacggcc aatactcaac ttcaaggaat ctcacccatg cgcgccggcg gggaaccgga 660

gttcccttca gtgagcgtta ttagttcgcc gctcggtgtg tcgtagatac tagcccctgg 720

ggcacttttg aaatttgaat aagatttatg taatcagtct tttaggtttg accggttctg 780

ccgctttttt taaaattgga tttgtaataa taaaacgcaa ttgtttgtta ttgtggcgct 840

ctatcataga tgtcgctata aacctattca gcacaatata ttgttttcat tttaatattg 900

tacatataag tagtagggta caatcagtaa attgaacgga gaatattatt cataaaaata 960

cgatagtaac gggtgatata ttcattagaa tgaaccgaaa ccggcggtaa ggatctgagc 1020

tacacatgct caggtttttt acaacgtgca caacagaatt gaaagcaaat atcatgcgat 1080

cataggcgtc tcgcatatct cattaaagca gggggtgggc gaagaactcc agcatgagat 1140

ccccgcgctg gaggatcatc cagccggcgt cccggaaaac gattccgaag cccaaccttt 1200

catagaaggc ggcggtggaa tcgaaatctc gtgatggcag gttgggcgtc gcttggtcgg 1260

tcatttcgaa ccccagagtc ccgctcagaa gaactcgtca agaaggcgat agaaggcgat 1320

gcgctgcgaa tcgggagcgg cgataccgta aagcacgagg aagcggtcag cccattcgcc 1380

gccaagctct tcagcaatat cacgggtagc caacgctatg tcctgatagc ggtccgccac 1440

acccagccgg ccacagtcga tgaatccaga aaagcggcca ttttccacca tgatattcgg 1500

caagcaggca tcgccatggg tcacgacgag atcctcgccg tcgggcatgc gcgccttgag 1560

cctggcgaac agttcggctg gcgcgagccc ctgatgctct tcgtccagat catcctgatc 1620

gacaagaccg gcttccatcc gagtacgtgc tcgctcgatg cgatgtttcg cttggtggtc 1680

gaatgggcag gtagccggat caagcgtatg cagccgccgc attgcatcag ccatgatgga 1740

tactttctcg gcaggagcaa ggtgagatga caggagatcc tgccccggca cttcgcccaa 1800

tagcagccag tcccttcccg cttcagtgac aacgtcgagc acagctgcgc aaggaacgcc 1860

cgtcgtggcc agccacgata gccgcgctgc ctcgtcctgc agttcattca gggcaccgga 1920

caggtcggtc ttgacaaaaa gaaccgggcg cccctgcgct gacagccgga acacggcggc 1980

atcagagcag ccgattgtct gttgtgccca gtcatagccg aatagcctct ccacccaagc 2040

ggccggagaa cctgcgtgca atccatcttg ttcaatccac atgatcaaac gttttgagga 2100

cgcgagagga ttcgattcga cgacgagagc ctcgcgagat tggggagaaa tttttcgggg 2160

gtggagctga tgcgaggaga ggagatgagg gggctggtat ttatggcggt tgggtggtgg 2220

gaggagtccc gtgccgtgac gtctccgtct gcttggagaa tccgccacgc tgaaaccacc 2280

gcggtttccg ggaagacgag gcgggcgagc gagcggttgg gaaatttcga gaagatgccg 2340

tttgtctccg tttggtacac gtctcgttga ttttttttta gtgaattacg ctttggacca 2400

cattttatta tctaagggtg tgtttggttg taagccacac tttgccacag tttgccacgc 2460

ctaaggttag gcaaatttga caggtgtttg gttgtagcca cagttgtggc aagatttccc 2520

tctaacaaat taagtcccac gtgtcaatgg ctcaaaaaag tgtggcaaga ttcccttagg 2580

cttagtaagt tgtggctaac aatttgatca cctcacctta gacaaggtgt ggcaactttt 2640

gttggcaagt aatggtaaag tatggctggg aaccaaacag cccctaagtt ttactttgga 2700

ctacctttaa acatatcttt tcactttgaa ctagataaat ttgctattgt tgcgatttgg 2760

attttttttt tctcgtgcaa tcaacgacct taaacacatc agctctagta tacggccgat 2820

ctcctctata tatggttcat atgtttgccg aaagggaagt tagacatgac gaaaagttgt 2880

tcatggtagt ccaaaccaca acccggccca atttgaaaag ataggtttaa gggtggtcca 2940

aattgaaact tgggtaataa aaggtggatc aaagtgcaat ttactttttt ttactgtaat 3000

ttcttctggc tggtttgttg gtcgccgtta ggaccgggtg acgccgtcaa ccccgcgcct 3060

ccgtattcgc tgacgtgggg tggcgcgctg gcttccgcct tgacccgaat ttgttttcct 3120

tccgttaaaa aaatggtttt ccttttctta aaaaggaaat agtttgtttt ttaagtctgt 3180

gtattaggat tattacactt gaattttggt atatgtgtag gataatttac tgcatgttta 3240

taatagagtt gtactataga tgaaataacc caatttttgg tataattcgt gtttggttgg 3300

aggtcaaaat aacaggttat tttgtgaaga aaaaactccg tagtatagta ccatatccat 3360

catgaataca catactgcct agacgagtga ttaggatgaa tccatgttat attcctcaaa 3420

ataatataaa ccacttgatc ttatgatctt atccaatctg ttcatataaa ctggagatat 3480

aagatggtgc atttcccttt tgatttcttt tgttgacggc catgagatag gttgcatcca 3540

ctgcatttat attttggacc aatacaatgc acctattgat acatggggac agctcaacta 3600

accatgatgc aaaatgctgg ttggtgacca gttcttggca ttatgataat gataggatta 3660

aaaaaaacag tgcaatgtct cggaaagaaa ccatgacaaa gggtacatgt tgcattccag 3720

tttctaatga taaaattatg tgccagcaat tcaaaaatca tgcgtgttcc ctacgcacca 3780

ttctttgcaa taaacaagtg catgcacaat atgattgtgc taaggttcaa gaacttgttg 3840

cagtggctaa gcttggcgcg cctcgcgacc acctttaatt aagtgaagag caggagcttg 3900

catgcctgca ggctctagag gatcccccct cagaagacca gagggctatt gagacttttc 3960

aacaaagggt aatatcggga aacctcctcg gattccattg cccagctatc tgtcacttca 4020

tcgaaaggac agtagaaaag gaaggtggct cctacaaatg ccatcattgc gataaaggaa 4080

aggctatcgt tcaagatgcc tctaccgaca gtggtcccaa agatggaccc ccacccacga 4140

ggaacatcgt ggaaaaagaa gacgttccaa ccacgtcttc aaagcaagtg gattgatgtg 4200

atatctccac tgacgtaagg gatgacgcac aatcccacta tccttcgcaa gacccttcct 4260

ctatataagg aagttcattt catttggaga ggacaggctt cttgagatcc ttcaacaatt 4320

accaacaaca acaaacaaca aacaacatta caattactat ttacaattac agtcgactct 4380

agaggatcca tggtgagcaa gggcgaggag ctgttcaccg gggtggtgcc catcctggtc 4440

gagctggacg gcgacgtaaa cggccacaag ttcagcgtgt ccggcgaggg cgagggcgat 4500

gccacctacg gcaagctgac cctgaagttc atctgcacca ccggcaagct gcccgtgccc 4560

tggcccaccc tcgtgaccac cttcacctac ggcgtgcagt gcttcagccg ctaccccgac 4620

cacatgaagc agcacgactt cttcaagtcc gccatgcccg aaggctacgt ccaggagcgc 4680

accatcttct tcaaggacga cggcaactac aagacccgcg ccgaggtgaa gttcgagggc 4740

gacaccctgg tgaaccgcat cgagctgaag ggcatcgact tcaaggagga cggcaacatc 4800

ctggggcaca agctggagta caactacaac agccacaacg tctatatcat ggccgacaag 4860

cagaagaacg gcatcaaggt gaacttcaag atccgccaca acatcgagga cggcagcgtg 4920

cagctcgccg accactacca gcagaacacc cccatcggcg acggccccgt gctgctgccc 4980

gacaaccact acctgagcac ccagtccgcc ctgagcaaag accccaacga gaagcgcgat 5040

cacatggtcc tgctggagtt cgtgaccgcc gccgggatca ctcacggcat ggacgagctg 5100

tacaagtaaa gcggccgccc ggctgcagat cgttcaaaca tttggcaata aagtttctta 5160

agattgaatc ctgttgccgg tcttgcgatg attatcatat aatttctgtt gaattacgtt 5220

aagcatgtaa taattaacat gtaatgcatg acgttattta tgagatgggt ttttatgatt 5280

agagtcccgc aattatacat ttaatacgcg atagaaaaca aaatatagcg cgcaaactag 5340

gataaattat cgcgcgcggt gtcatctatg ttactagatc cgatgataag ctgtcaaaca 5400

tgagaattcc tttcgtcgac ccacgtgttg ctgaggtatt taaataatcc gaaaagtttc 5460

tgcaccgttt tcacccccta actaacaata tagggaacgt gtgctaaata taaaatgaga 5520

ccttatatat gtagcgctga taactagaac tatgcaagaa aaactcatcc acctacttta 5580

gtggcaatcg ggctaaataa aaaagagtcg ctacactagt ttcgttttcc ttagtaatta 5640

agtgggaaaa tgaaatcatt attgcttaga atatacgttc acatctctgt catgaagtta 5700

aattattcga ggtagccata attgtcatca aactcttctt gaataaaaaa atctttctag 5760

ctgaactcaa tgggtaaaga gagagatttt ttttaaaaaa atagaatgaa gatattctga 5820

acgtattggc aaagatttaa acatataatt atataatttt atagtttgtg cattcgtcat 5880

atcgcacatc attaaggaca tgtcttactc catcccaatt tttatttagt aattaaagac 5940

aattgactta tttttattat ttatcttttt tcgattagat gcaaggtact tacgcacaca 6000

ctttgtgctc atgtgcatgt gtgagtgcac ctcctcaata cacgttcaac tagcaacaca 6060

tctctaatat cactcgccta tttaatacat ttaggtagca atatctgaat tcaagcactc 6120

caccatcacc agaccacttt taataatatc taaaatacaa aaaataattt tacagaatag 6180

catgaaaagt atgaaacgaa ctatttaggt ttttcacata caaaaaaaaa aagaattttg 6240

ctcgtgcgcg agcgccaatc tcccatattg ggcacacagg caacaacaga gtggctgccc 6300

acagaacaac ccacaaaaaa cgatgatcta acggaggaca gcaagtccgc aacaaccttt 6360

taacagcagg ctttgcggcc aggagagagg aggagaggca aagaaaacca agcatcctcc 6420

ttctcccatc tataaattcc tccccccttt tcccctctct atataggagg catccaagcc 6480

aagaagaggg agagcaccaa ggacacgcga ctagcagaag ccgagcgacc gccttctcga 6540

tccatatctt ccggtcgagt tcttggtcga tctcttccct cctccacctc ctcctcacag 6600

ggtatgtgcc tcccttcggt tgttcttgga tttattgttc taggttgtgt agtacgggcg 6660

ttgatgttag gaaaggggat ctgtatctgt gatgattcct gttcttggat ttgggataga 6720

ggggttcttg atgttgcatg ttatcggttc ggtttgatta gtagtatggt tttcaatcgt 6780

ctggagagct ctatggaaat gaaatggttt agggatcgga atcttgcgat tttgtgagta 6840

ccttttgttt gaggtaaaat cagagcaccg gtgattttgc ttggtgtaat aaagtacggt 6900

tgtttggtcc tcgattctgg tagtgatgct tctcgatttg acgaagctat cctttgttta 6960

ttccctattg aacaaaaata atccaacttt gaagacggtc ccgttgatga gattgaatga 7020

ttgattctta agcctgtcca aaatttcgca gctggcttgt ttagatacag tagtccccat 7080

cacgaaattc atggaaacag ttataatcct caggaacagg ggattccctg ttcttccgat 7140

ttgctttagt cccagaattt tttttcccaa atatcttaaa aagtcacttt ctggttcagt 7200

tcaatgaatt gattgctaca aataatgctt ttatagcgtt atcctagctg tagttcagtt 7260

aataggtaat acccctatag tttagtcagg agaagaactt atccgatttc tgatctccat 7320

ttttaattat atgaaatgaa ctgtagcata agcagtattc atttggatta ttttttttat 7380

tagctctcac cccttcatta ttctgagctg aaagtctggc atgaactgtc ctcaattttg 7440

ttttcaaatt cacatcgatt atctatgcat tatcctcttg tatctacctg tagaagtttc 7500

tttttggtta ttccttgact gcttgattac agaaagaaat ttatgaagct gtaatcggga 7560

tagttatact gcttgttctt atgattcatt tcctttgtgc agttcttggt gtagcttgcc 7620

actttcacca gcaaagttca tttaaatcaa ctagggatat cacaagtttg tacaaaaaag 7680

caggctggat cctacgtaag atctaccatg gaagacgcca aaaacataaa gaaaggcccg 7740

gcgccattct atccgctgga agatggaacc gctggagagc aactgcataa ggctatgaag 7800

agatacgccc tggttcctgg aacaattgct tttacagatg cacatatcga ggtggacatc 7860

acttacgctg agtacttcga aatgtccgtt cggttggcag aagctatgaa acgatatggg 7920

ctgaatacaa atcacagaat cgtcgtatgc agtgaaaact ctcttcaatt ctttatgccg 7980

gtgttgggcg cgttatttat cggagttgca gttgcgcccg cgaacgacat ttataatgaa 8040

cgtgaattgc tcaacagtat gggcatttcg cagcctaccg tggtgttcgt ttccaaaaag 8100

gggttgcaaa aaattttgaa cgtgcaaaaa aagctcccaa tcatccaaaa aattattatc 8160

atggattcta aaacggatta ccagggattt cagtcgatgt acacgttcgt cacatctcat 8220

ctacctcccg gttttaatga atacgatttt gtgccagagt ccttcgatag ggacaagaca 8280

attgcactga tcatgaactc ctctggatct actggtctgc ctaaaggtgt cgctctgcct 8340

catagaactg cctgcgtgag attctcgcat gccagagatc ctatttttgg caatcaaatc 8400

attccggata ctgcgatttt aagtgttgtt ccattccatc acggttttgg aatgtttact 8460

acactcggat atttgatatg tggatttcga gtcgtcttaa tgtatagatt tgaagaagag 8520

ctgtttctga ggagccttca ggattacaag attcaaagtg cgctgctggt gccaacccta 8580

ttctccttct tcgccaaaag cactctgatt gacaaatacg atttatctaa tttacacgaa 8640

attgcttctg gtggcgctcc cctctctaag gaagtcgggg aagcggttgc caagaggttc 8700

catctgccag gtatcaggca aggatatggg ctcactgaga ctacatcagc tattctgatt 8760

acacccgagg gggatgataa accgggcgcg gtcggtaaag ttgttccatt ttttgaagcg 8820

aaggttgtgg atctggatac cgggaaaacg ctgggcgtta atcaaagagg cgaactgtgt 8880

gtgagaggtc ctatgattat gtccggttat gtaaacaatc cggaagcgac caacgccttg 8940

attgacaagg atggatggct acattctgga gacatagctt actgggacga agacgaacac 9000

ttcttcatcg ttgaccgcct gaagtctctg attaagtaca aaggctatca ggtggctccc 9060

gctgaattgg aatccatctt gctccaacac cccaacatct tcgacgcagg tgtcgcaggt 9120

cttcccgacg atgacgccgg tgaacttccc gccgccgttg ttgttttgga gcacggaaag 9180

acgatgacgg aaaaagagat cgtggattac gtcgccagtc aagtaacaac cgcgaaaaag 9240

ttgcgcggag gagttgtgtt tgtggacgaa gtaccgaaag gtcttaccgg aaaactcgac 9300

gcaagaaaaa tcagagagat cctcataaag gccaagaagg gcggaaagat cgccgtgtaa 9360

ctcgagcata tgggctcgaa tttccccgat cgttcaaaca tttggcaata aagtttctta 9420

agattgaatc ctgttgccgg tcttgcgatg attatcatat aatttctgtt gaattacgtt 9480

aagcatgtaa taattaacat gtaatgcatg acgttattta tgagatgggt ttttatgatt 9540

agagtcccgc aattatacat ttaatacgcg atagaaaaca aaatatagcg cgcaaactag 9600

gataaattat cgcgcgcggt gtcatctatg ttactagatc gggaattcaa gcttggcgta 9660

atcatggacc cagctttctt gtacaaagtg gtgatatcac aagcccgggc ggtcttctag 9720

ggataacagg gtaattatat ccctctagat cacaagcccg ggcggtcttc tacgatgatt 9780

gagtaataat gtgtcacgca tcaccatggg tggcagtgtc agtgtgagca atgacctgaa 9840

tgaacaattg aaatgaaaag aaaaaaagta ctccatctgt tccaaattaa aattggtttt 9900

aaccttttaa taggtttata caataattga tatatgtttt ctgtatatgt ctaatttgtt 9960

atcatccggg cggtcttcta gggataacag ggtaattata tccctctaga caacacacaa 10020

caaataagag aaaaaacaaa taatattaat ttgagaatga acaaaaggac catatcattc 10080

attaactctt ctccatccac ttccatttca cagttcgata gcgaaaaccg aataaaaaac 10140

acagtaaatt acaagcacaa caaatggtac aagaaaaaca gttttcccaa tgccataata 10200

ctcgactcga gttcctgcag gtaccaaaag cttagcttga gcttggatca gattgtcgtt 10260

tcccgccttc agtttaaact atcagtgttt gacaggatat attggcgggt aaacctaaga 10320

gaaaagagcg tttattagaa taatcggata tttaaaaggg cgtgaaaagg tttatccgtt 10380

cgtccatttg tatgtgcatg ccaaccacag ggttcccctc gggatcaaag tatgaagaga 10440

tcgaggcgga gatgatcgcg gccgggtacg tgttcgagcc gcccgcgcac gtctcaaccg 10500

tgcggctgca tgaaatcctg gccggtttgt ctgatgccaa gctggcggcc tggccggcca 10560

gcttggccgc tgaagaaacc gagcgccgcc gtctaaaaag gtgatgtgta tttgagtaaa 10620

acagcttgcg tcatgcggtc gctgcgtata tgatgcgatg agtaaataaa caaatacgca 10680

aggggaacgc atgaaggtta tcgctgtact taaccagaaa ggcgggtcag gcaagacgac 10740

catcgcaacc catctagccc gcgccctgca actcgccggg gccgatgttc tgttagtcga 10800

ttccgatccc cagggcagtg cccgcgattg ggcggccgtg cgggaagatc aaccgctaac 10860

cgttgtcggc atcgaccgcc cgacgattga ccgcgacgtg aaggccatcg gccggcgcga 10920

cttcgtagtg atcgacggag cgccccaggc ggcggacttg gctgtgtccg cgatcaaggc 10980

agccgacttc gtgctgattc cggtgcagcc aagcccttac gacatatggg ccaccgccga 11040

cctggtggag ctggttaagc agcgcattga ggtcacggat ggaaggctac aagcggcctt 11100

tgtcgtgtcg cgggcgatca aaggcacgcg catcggcggt gaggttgccg aggcgctggc 11160

cgggtacgag ctgcccattc ttgagtcccg tatcacgcag cgcgtgagct acccaggcac 11220

tgccgccgcc ggcacaaccg ttcttgaatc agaacccgag ggcgacgctg cccgcgaggt 11280

ccaggcgctg gccgctgaaa ttaaatcaaa actcatttga gttaatgagg taaagagaaa 11340

atgagcaaaa gcacaaacac gctaagtgcc ggccgtccga gcgcacgcag cagcaaggct 11400

gcaacgttgg ccagcctggc agacacgcca gccatgaagc gggtcaactt tcagttgccg 11460

gcggaggatc acaccaagct gaagatgtac gcggtacgcc aaggcaagac cattaccgag 11520

ctgctatctg aatacatcgc gcagctacca gagtaaatga gcaaatgaat aaatgagtag 11580

atgaatttta gcggctaaag gaggcggcat ggaaaatcaa gaacaaccag gcaccgacgc 11640

cgtggaatgc cccatgtgtg gaggaacggg cggttggcca ggcgtaagcg gctgggttgt 11700

ctgccggccc tgcaatggca ctggaacccc caagcccgag gaatcggcgt gagcggtcgc 11760

aaaccatccg gcccggtaca aatcggcgcg gcgctgggtg atgacctggt ggagaagttg 11820

aaggccgcgc aggccgccca gcggcaacgc atcgaggcag aagcacgccc cggtgaatcg 11880

tggcaagcgg ccgctgatcg aatccgcaaa gaatcccggc aaccgccggc agccggtgcg 11940

ccgtcgatta ggaagccgcc caagggcgac gagcaaccag attttttcgt tccgatgctc 12000

tatgacgtgg gcacccgcga tagtcgcagc atcatggacg tggccgtttt ccgtctgtcg 12060

aagcgtgacc gacgagctgg cgaggtgatc cgctacgagc ttccagacgg gcacgtagag 12120

gtttccgcag ggccggccgg catggccagt gtgtgggatt acgacctggt actgatggcg 12180

gtttcccatc taaccgaatc catgaaccga taccgggaag ggaagggaga caagcccggc 12240

cgcgtgttcc gtccacacgt tgcggacgta ctcaagttct gccggcgagc cgatggcgga 12300

aagcagaaag acgacctggt agaaacctgc attcggttaa acaccacgca cgttgccatg 12360

cagcgtacga agaaggccaa gaacggccgc ctggtgacgg tatccgaggg tgaagccttg 12420

attagccgct acaagatcgt aaagagcgaa accgggcggc cggagtacat cgagatcgag 12480

ctagctgatt ggatgtaccg cgagatcaca gaaggcaaga acccggacgt gctgacggtt 12540

caccccgatt actttttgat cgatcccggc atcggccgtt ttctctaccg cctggcacgc 12600

cgcgccgcag gcaaggcaga agccagatgg ttgttcaaga cgatctacga acgcagtggc 12660

agcgccggag agttcaagaa gttctgtttc accgtgcgca agctgatcgg gtcaaatgac 12720

ctgccggagt acgatttgaa ggaggaggcg gggcaggctg gcccgatcct agtcatgcgc 12780

taccgcaacc tgatcgaggg cgaagcatcc gccggttcct aatgtacgga gcagatgcta 12840

gggcaaattg ccctagcagg ggaaaaaggt cgaaaaggtc tctttcctgt ggatagcacg 12900

tacattggga acccaaagcc gtacattggg aaccggaacc cgtacattgg gaacccaaag 12960

ccgtacattg ggaaccggtc acacatgtaa gtgactgata taaaagagaa aaaaggcgat 13020

ttttccgcct aaaactcttt aaaacttatt aaaactctta aaacccgcct ggcctgtgca 13080

taactgtctg gccagcgcac agccgaagag ctgcaaaaag cgcctaccct tcggtcgctg 13140

cgctccctac gccccgccgc ttcgcgtcgg cctatcgcgg ccgctggccg ctcaaaaatg 13200

gctggcctac ggccaggcaa tctaccaggg cgcggacaag ccgcgccgtc gccactcgac 13260

cgccggcgcc cacatcaagg caccctgcct cgcgcgtttc ggtgatgacg gtgaaaacct 13320

ctgacacatg cagctcccgg agacggtcac agcttgtctg taagcggatg ccgggagcag 13380

acaagcccgt cagggcgcgt cagcgggtgt tggcgggtgt cggggcgcag ccatgaccca 13440

gtcacgtagc gatagcggag tgtatactgg cttaactatg cggcatcaga gcagattgta 13500

ctgagagtgc accatatgcg gtgtgaaata ccgcacagat gcgtaaggag aaaataccgc 13560

atcaggcgct cttccgcttc ctcgctcact gactcgctgc gctcggtcgt tcggctgcgg 13620

cgagcggtat cagctcactc aaaggcggta atacggttat ccacagaatc aggggataac 13680

gcaggaaaga acatgtgagc aaaaggccag caaaaggcca ggaaccgtaa aaaggccgcg 13740

ttgctggcgt ttttccatag gctccgcccc cctgacgagc atcacaaaaa tcgacgctca 13800

agtcagaggt ggcgaaaccc gacaggacta taaagatacc aggcgtttcc ccctggaagc 13860

tccctcgtgc gctctcctgt tccgaccctg ccgcttaccg gatacctgtc cgcctttctc 13920

ccttcgggaa gcgtggcgct ttctcatagc tcacgctgta ggtatctcag ttcggtgtag 13980

gtcgttcgct ccaagctggg ctgtgtgcac gaaccccccg ttcagcccga ccgctgcgcc 14040

ttatccggta actatcgtct tgagtccaac ccggtaagac acgacttatc gccactggca 14100

gcagccactg gtaacaggat tagcagagcg aggtatgtag gcggtgctac agagttcttg 14160

aagtggtggc ctaactacgg ctacactaga aggacagtat ttggtatctg cgctctgctg 14220

aagccagtta ccttcggaaa aagagttggt agctcttgat ccggcaaaca aaccaccgct 14280

ggtagcggtg gtttttttgt ttgcaagcag cagattacgc gcagaaaaaa aggatctcaa 14340

gaagatcctt tgatcttttc tacggggtct gacgcttagt ggaacgaaaa ctcacgttaa 14400

gggattttgg tcatgcatga tatatctccc aatttgtgta gggcttatta tgcacgctta 14460

aaaataataa aagcagactt gacctgatag tttggctgtg agcaattatg tgcttagtgc 14520

atctaacgct tgagttaagc cgcgccgcga agcggcgtcg gcttgaacga atttctagct 14580

agacattatt tgccgactac cttggtgatc tcgcctttca cgtagtggac aaattcttcc 14640

aactgatctg cgcgcgaggc caagcgatct tcttcttgtc caagataagc ctgtctagct 14700

tcaagtatga cgggctgata ctgggccggc aggcgctcca ttgcccagtc ggcagcgaca 14760

tccttcggcg cgattttgcc ggttactgcg ctgtaccaaa tgcgggacaa cgtaagcact 14820

acatttcgct catcgccagc ccagtcgggc ggcgagttcc atagcgttaa ggtttcattt 14880

agcgcctcaa atagatcctg ttcaggaacc ggatcaaaga gttcctccgc cgctggacct 14940

accaaggcaa cgctatgttc tcttgctttt gtcagcaaga tagccagatc aatgtcgatc 15000

gtggctggct cgaagatacc tgcaagaatg tcattgcgct gccattctcc aaattgcagt 15060

tcgcgcttag ctggataacg ccacggaatg atgtcgtcgt gcacaacaat ggtgacttct 15120

acagcgcgga gaatctcgct ctctccaggg gaagccgaag tttccaaaag gtcgttgatc 15180

aaagctcgcc gcgttgtttc atcaagcctt acggtcaccg taaccagcaa atcaatatca 15240

ctgtgtggct tcaggccgcc atccactgcg gagccgtaca aatgtacggc cagcaacgtc 15300

ggttcgagat ggcgctcgat gacgccaact acctctgata gttgagtcga tacttcggcg 15360

atcaccgctt cccccatgat gtttaacttt gttttagggc gactgccctg ctgcgtaaca 15420

tcgttgctgc tccataacat caaacatcga cccacggcgt aacgcgcttg ctgcttggat 15480

gcccgaggca tagactgtac cccaaaaaaa cagtcataac aagccatgaa aaccgccact 15540

gcgccgttac caccgctgcg ttcggtcaag gttctggacc agttgcgtga gcgcatacgc 15600

tacttgcatt acagcttacg aaccgaacag gcttatgtcc actgggttcg tgcccgaatt 15660

gatca 15665

<210> 100
<211> 15339
<212> DNA
<213> artificial

<220>
<223> vector sequence

<400> 100
tcagaagaac tcgtcaagaa ggcgatagaa ggcgatgcgc tgcgaatcgg gagcggcgat 60

accgtaaagc acgaggaagc ggtcagccca ttcgccgcca agctcttcag caatatcacg 120

ggtagccaac gctatgtcct gatagcggtc cgccacaccc agccggccac agtcgatgaa 180

tccagaaaag cggccatttt ccaccatgat attcggcaag caggcatcgc catgggtcac 240

gacgagatcc tcgccgtcgg gcatgcgcgc cttgagcctg gcgaacagtt cggctggcgc 300

gagcccctga tgctcttcgt ccagatcatc ctgatcgaca agaccggctt ccatccgagt 360

acgtgctcgc tcgatgcgat gtttcgcttg gtggtcgaat gggcaggtag ccggatcaag 420

cgtatgcagc cgccgcattg catcagccat gatggatact ttctcggcag gagcaaggtg 480

agatgacagg agatcctgcc ccggcacttc gcccaatagc agccagtccc ttcccgcttc 540

agtgacaacg tcgagcacag ctgcgcaagg aacgcccgtc gtggccagcc acgatagccg 600

cgctgcctcg tcctgcagtt cattcagggc accggacagg tcggtcttga caaaaagaac 660

cgggcgcccc tgcgctgaca gccggaacac ggcggcatca gagcagccga ttgtctgttg 720

tgcccagtca tagccgaata gcctctccac ccaagcggcc ggagaacctg cgtgcaatcc 780

atcttgttca atccacatga tcaaacgttt tgaggacgcg agaggattcg attcgacgac 840

gagagcctcg cgagattggg gagaaatttt tcgggggtgg agctgatgcg aggagaggag 900

atgagggggc tggtatttat ggcggttggg tggtgggagg agtcccgtgc cgtgacgtct 960

ccgtctgctt ggagaatccg ccacgctgaa accaccgcgg tttccgggaa gacgaggcgg 1020

gcgagcgagc ggttgggaaa tttcgagaag atgccgtttg tctccgtttg gtacacgtct 1080

cgttgatttt tttttagtga attacgcttt ggaccacatt ttattatcta agggtgtgtt 1140

tggttgtaag ccacactttg ccacagtttg ccacgcctaa ggttaggcaa atttgacagg 1200

tgtttggttg tagccacagt tgtggcaaga tttccctcta acaaattaag tcccacgtgt 1260

caatggctca aaaaagtgtg gcaagattcc cttaggctta gtaagttgtg gctaacaatt 1320

tgatcacctc accttagaca aggtgtggca acttttgttg gcaagtaatg gtaaagtatg 1380

gctgggaacc aaacagcccc taagttttac tttggactac ctttaaacat atcttttcac 1440

tttgaactag ataaatttgc tattgttgcg atttggattt tttttttctc gtgcaatcaa 1500

cgaccttaaa cacatcagct ctagtatacg gccgatctcc tctatatatg gttcatatgt 1560

ttgccgaaag ggaagttaga catgacgaaa agttgttcat ggtagtccaa accacaaccc 1620

ggcccaattt gaaaagatag gtttaagggt ggtccaaatt gaaacttggg taataaaagg 1680

tggatcaaag tgcaatttac ttttttttac tgtaatttct tctggctggt ttgttggtcg 1740

ccgttaggac cgggtgacgc cgtcaacccc gcgcctccgt attcgctgac gtggggtggc 1800

gcgctggctt ccgccttgac ccgaatttgt tttccttccg ttaaaaaaat ggttttcctt 1860

ttcttaaaaa ggaaatagtt tgttttttaa gtctgtgtat taggattatt acacttgaat 1920

tttggtatat gtgtaggata atttactgca tgtttataat agagttgtac tatagatgaa 1980

ataacccaat ttttggtata attcgtgttt ggttggaggt caaaataaca ggttattttg 2040

tgaagaaaaa actccgtagt atagtaccat atccatcatg aatacacata ctgcctagac 2100

gagtgattag gatgaatcca tgttatattc ctcaaaataa tataaaccac ttgatcttat 2160

gatcttatcc aatctgttca tataaactgg agatataaga tggtgcattt cccttttgat 2220

ttcttttgtt gacggccatg agataggttg catccactgc atttatattt tggaccaata 2280

caatgcacct attgatacat ggggacagct caactaacca tgatgcaaaa tgctggttgg 2340

tgaccagttc ttggcattat gataatgata ggattaaaaa aaacagtgca atgtctcgga 2400

aagaaaccat gacaaagggt acatgttgca ttccagtttc taatgataaa attatgtgcc 2460

agcaattcaa aaatcatgcg tgttccctac gcaccattct ttgcaataaa caagtgcatg 2520

cacaatatga ttgtgctaag gttcaagaac ttgttgcagt ggctaagctt ggcgcgcctc 2580

gcgaccacct ttaattaagt gaagagcagg agcttgcatg cctgcaggct ctagaggatc 2640

ccccctcaga agaccagagg gctattgaga cttttcaaca aagggtaata tcgggaaacc 2700

tcctcggatt ccattgccca gctatctgtc acttcatcga aaggacagta gaaaaggaag 2760

gtggctccta caaatgccat cattgcgata aaggaaaggc tatcgttcaa gatgcctcta 2820

ccgacagtgg tcccaaagat ggacccccac ccacgaggaa catcgtggaa aaagaagacg 2880

ttccaaccac gtcttcaaag caagtggatt gatgtgatat ctccactgac gtaagggatg 2940

acgcacaatc ccactatcct tcgcaagacc cttcctctat ataaggaagt tcatttcatt 3000

tggagaggac aggcttcttg agatccttca acaattacca acaacaacaa acaacaaaca 3060

acattacaat tactatttac aattacagtc gactctagag gatccatggt gagcaagggc 3120

gaggagctgt tcaccggggt ggtgcccatc ctggtcgagc tggacggcga cgtaaacggc 3180

cacaagttca gcgtgtccgg cgagggcgag ggcgatgcca cctacggcaa gctgaccctg 3240

aagttcatct gcaccaccgg caagctgccc gtgccctggc ccaccctcgt gaccaccttc 3300

acctacggcg tgcagtgctt cagccgctac cccgaccaca tgaagcagca cgacttcttc 3360

aagtccgcca tgcccgaagg ctacgtccag gagcgcacca tcttcttcaa ggacgacggc 3420

aactacaaga cccgcgccga ggtgaagttc gagggcgaca ccctggtgaa ccgcatcgag 3480

ctgaagggca tcgacttcaa ggaggacggc aacatcctgg ggcacaagct ggagtacaac 3540

tacaacagcc acaacgtcta tatcatggcc gacaagcaga agaacggcat caaggtgaac 3600

ttcaagatcc gccacaacat cgaggacggc agcgtgcagc tcgccgacca ctaccagcag 3660

aacaccccca tcggcgacgg ccccgtgctg ctgcccgaca accactacct gagcacccag 3720

tccgccctga gcaaagaccc caacgagaag cgcgatcaca tggtcctgct ggagttcgtg 3780

accgccgccg ggatcactca cggcatggac gagctgtaca agtaaagcgg ccgcccggct 3840

gcagatcgtt caaacatttg gcaataaagt ttcttaagat tgaatcctgt tgccggtctt 3900

gcgatgatta tcatataatt tctgttgaat tacgttaagc atgtaataat taacatgtaa 3960

tgcatgacgt tatttatgag atgggttttt atgattagag tcccgcaatt atacatttaa 4020

tacgcgatag aaaacaaaat atagcgcgca aactaggata aattatcgcg cgcggtgtca 4080

tctatgttac tagatccgat gataagctgt caaacatgag aattcctttc gtcgacccac 4140

gtgttgctga ggtatttaaa taatccgaaa agtttctgca ccgttttcac cccctaacta 4200

acaatatagg gaacgtgtgc taaatataaa atgagacctt atatatgtag cgctgataac 4260

tagaactatg caagaaaaac tcatccacct actttagtgg caatcgggct aaataaaaaa 4320

gagtcgctac actagtttcg ttttccttag taattaagtg ggaaaatgaa atcattattg 4380

cttagaatat acgttcacat ctctgtcatg aagttaaatt attcgaggta gccataattg 4440

tcatcaaact cttcttgaat aaaaaaatct ttctagctga actcaatggg taaagagaga 4500

gatttttttt aaaaaaatag aatgaagata ttctgaacgt attggcaaag atttaaacat 4560

ataattatat aattttatag tttgtgcatt cgtcatatcg cacatcatta aggacatgtc 4620

ttactccatc ccaattttta tttagtaatt aaagacaatt gacttatttt tattatttat 4680

cttttttcga ttagatgcaa ggtacttacg cacacacttt gtgctcatgt gcatgtgtga 4740

gtgcacctcc tcaatacacg ttcaactagc aacacatctc taatatcact cgcctattta 4800

atacatttag gtagcaatat ctgaattcaa gcactccacc atcaccagac cacttttaat 4860

aatatctaaa atacaaaaaa taattttaca gaatagcatg aaaagtatga aacgaactat 4920

ttaggttttt cacatacaaa aaaaaaaaga attttgctcg tgcgcgagcg ccaatctccc 4980

atattgggca cacaggcaac aacagagtgg ctgcccacag aacaacccac aaaaaacgat 5040

gatctaacgg aggacagcaa gtccgcaaca accttttaac agcaggcttt gcggccagga 5100

gagaggagga gaggcaaaga aaaccaagca tcctccttct cccatctata aattcctccc 5160

cccttttccc ctctctatat aggaggcatc caagccaaga agagggagag caccaaggac 5220

acgcgactag cagaagccga gcgaccgcct tctcgatcca tatcttccgg tcgagttctt 5280

ggtcgatctc ttccctcctc cacctcctcc tcacagggta tgtgcctccc ttcggttgtt 5340

cttggattta ttgttctagg ttgtgtagta cgggcgttga tgttaggaaa ggggatctgt 5400

atctgtgatg attcctgttc ttggatttgg gatagagggg ttcttgatgt tgcatgttat 5460

cggttcggtt tgattagtag tatggttttc aatcgtctgg agagctctat ggaaatgaaa 5520

tggtttaggg atcggaatct tgcgattttg tgagtacctt ttgtttgagg taaaatcaga 5580

gcaccggtga ttttgcttgg tgtaataaag tacggttgtt tggtcctcga ttctggtagt 5640

gatgcttctc gatttgacga agctatcctt tgtttattcc ctattgaaca aaaataatcc 5700

aactttgaag acggtcccgt tgatgagatt gaatgattga ttcttaagcc tgtccaaaat 5760
ttcgcagctg gcttgtttag atacagtagt ccccatcacg aaattcatgg aaacagttat 5820
aatcctcagg aacaggggat tccctgttct tccgatttgc tttagtccca gaattttttt 5880
tcccaaatat cttaaaaagt cactttctgg ttcagttcaa tgaattgatt gctacaaata 5940
atgcttttat agcgttatcc tagctgtagt tcagttaata ggtaataccc ctatagttta 6000
gtcaggagaa gaacttatcc gatttctgat ctccattttt aattatatga aatgaactgt 6060
agcataagca gtattcattt ggattatttt ttttattagc tctcacccct tcattattct 6120
gagctgaaag tctggcatga actgtcctca attttgtttt caaattcaca tcgattatct 6180
atgcattatc ctcttgtatc tacctgtaga agtttctttt tggttattcc ttgactgctt 6240
gattacagaa agaaatttat gaagctgtaa tcgggatagt tatactgctt gttcttatga 6300
ttcatttcct ttgtgcagtt cttggtgtag cttgccactt tcaccagcaa agttcattta 6360
aatcaactag ggatatcaca agtttgtaca aaaaagctga acgagaaacg taaaatgata 6420
taaatatcaa tatattaaat tagattttgc ataaaaaaca gactacataa tactgtaaaa 6480
cacaacatat ccagtcacta tggcggccgc attaggcacc ccaggcttta cactttatgc 6540
ttccggctcg tataatgtgt ggattttgag ttaggatccg tcgagatttt caggagctaa 6600
ggaagctaaa atggagaaaa aaatcactgg atataccacc gttgatatat cccaatggca 6660
tcgtaaagaa cattttgagg catttcagtc agttgctcaa tgtacctata accagaccgt 6720
tcagctggat attacggcct ttttaaagac cgtaaagaaa aataagcaca agttttatcc 6780
ggcctttatt cacattcttg cccgcctgat gaatgctcat ccggaattcc gtatggcaat 6840
gaaagacggt gagctggtga tatgggatag tgttcaccct tgttacaccg ttttccatga 6900
gcaaactgaa acgttttcat cgctctggag tgaataccac gacgatttcc ggcagtttct 6960
acacatatat tcgcaagatg tggcgtgtta cggtgaaaac ctggcctatt tccctaaagg 7020
gtttattgag aatatgtttt tcgtctcagc caatccctgg gtgagtttca ccagttttga 7080
tttaaacgtg gccaatatgg acaacttctt cgcccccgtt ttcaccatgg gcaaatatta 7140
tacgcaaggc gacaaggtgc tgatgccgct ggcgattcag gttcatcatg ccgtttgtga 7200
tggcttccat gtcggcagaa tgcttaatga attacaacag tactgcgatg agtggcaggg 7260
cggggcgtaa acgcgtggat ccggcttact aaaagccaga taacagtatg cgtatttgcg 7320
cgctgatttt tgcggtataa gaatatatac tgatatgtat acccgaagta tgtcaaaaag 7380
aggtatgcta tgaagcagcg tattacagtg acagttgaca gcgacagcta tcagttgctc 7440
aaggcatata tgatgtcaat atctccggtc tggtaagcac aaccatgcag aatgaagccc 7500
gtcgtctgcg tgccgaacgc tggaaagcgg aaaatcagga agggatggct gaggtcgccc 7560
ggtttattga aatgaacggc tcttttgctg acgagaacag gggctggtga aatgcagttt 7620

aaggtttaca cctataaaag agagagccgt tatcgtctgt ttgtggatgt acagagtgat 7680

attattgaca cgcccgggcg acggatggtg atccccctgg ccagtgcacg tctgctgtca 7740

gataaagtct cccgtgaact ttacccggtg gtgcatatcg gggatgaaag ctggcgcatg 7800

atgaccaccg atatggccag tgtgccggtc tccgttatcg gggaagaagt ggctgatctc 7860

agccaccgcg aaaatgacat caaaaacgcc attaacctga tgttctgggg aatataaatg 7920

tcaggctccc ttatacacag ccagtctgca ggtcgaccat agtgactgga tatgttgtgt 7980

tttacagtat tatgtagtct gttttttatg caaaatctaa tttaatatat tgatatttat 8040

atcattttac gtttctcgtt cagctttctt gtacaaagtg gtgatatcac aagcccgggc 8100

ggtcttctag ggataacagg gtaattatat ccctctagat cacaagcccg ggcggtcttc 8160

tacgatgatt gagtaataat gtgtcacgca tcaccatggg tggcagtgtc agtgtgagca 8220

atgacctgaa tgaacaattg aaatgaaaag aaaaaaagta ctccatctgt tccaaattaa 8280

aattggtttt aaccttttaa taggtttata caataattga tatatgtttt ctgtatatgt 8340

ctaatttgtt atcatccggg cggtcttcta gggataacag ggtaattata tccctctaga 8400

caacacacaa caaataagag aaaaaacaaa taatattaat ttgagaatga acaaaaggac 8460

catatcattc attaactctt ctccatccac ttccatttca cagttcgata gcgaaaaccg 8520

aataaaaaac acagtaaatt acaagcacaa caaatggtac aagaaaaaca gttttcccaa 8580

tgccataata ctcgactcga gttcctgcag gtaccaaaag cttagcttga gcttggatca 8640

gattgtcgtt tcccgccttc agtttaaact atcagtgttt gacaggatat attggcgggt 8700

aaacctaaga gaaaagagcg tttattagaa taatcggata tttaaaaggg cgtgaaaagg 8760

tttatccgtt cgtccatttg tatgtgcatg ccaaccacag ggttcccctc gggatcaaag 8820

tatgaagaga tcgaggcgga gatgatcgcg gccgggtacg tgttcgagcc gcccgcgcac 8880

gtctcaaccg tgcggctgca tgaaatcctg gccggtttgt ctgatgccaa gctggcggcc 8940

tggccggcca gcttggccgc tgaagaaacc gagcgccgcc gtctaaaaag gtgatgtgta 9000

tttgagtaaa acagcttgcg tcatgcggtc gctgcgtata tgatgcgatg agtaaataaa 9060

caaatacgca aggggaacgc atgaaggtta tcgctgtact taaccagaaa ggcgggtcag 9120

gcaagacgac catcgcaacc catctagccc gcgccctgca actcgccggg gccgatgttc 9180

tgttagtcga ttccgatccc cagggcagtg cccgcgattg ggcggccgtg cgggaagatc 9240

aaccgctaac cgttgtcggc atcgaccgcc cgacgattga ccgcgacgtg aaggccatcg 9300

gccggcgcga cttcgtagtg atcgacggag cgccccaggc ggcggacttg gctgtgtccg 9360

cgatcaaggc agccgacttc gtgctgattc cggtgcagcc aagcccttac gacatatggg 9420

ccaccgccga cctggtggag ctggttaagc agcgcattga ggtcacggat ggaaggctac 9480

aagcggcctt tgtcgtgtcg cgggcgatca aaggcacgcg catcggcggt gaggttgccg 9540

aggcgctggc cgggtacgag ctgcccattc ttgagtcccg tatcacgcag cgcgtgagct 9600

acccaggcac tgccgccgcc ggcacaaccg ttcttgaatc agaacccgag ggcgacgctg 9660

cccgcgaggt ccaggcgctg gccgctgaaa ttaaatcaaa actcatttga gttaatgagg 9720

taaagagaaa atgagcaaaa gcacaaacac gctaagtgcc ggccgtccga gcgcacgcag 9780

cagcaaggct gcaacgttgg ccagcctggc agacacgcca gccatgaagc gggtcaactt 9840

tcagttgccg gcggaggatc acaccaagct gaagatgtac gcggtacgcc aaggcaagac 9900

cattaccgag ctgctatctg aatacatcgc gcagctacca gagtaaatga gcaaatgaat 9960

aaatgagtag atgaatttta gcggctaaag gaggcggcat ggaaaatcaa gaacaaccag 10020

gcaccgacgc cgtggaatgc cccatgtgtg gaggaacggg cggttggcca ggcgtaagcg 10080

gctgggttgt ctgccggccc tgcaatggca ctggaacccc caagcccgag gaatcggcgt 10140

gagcggtcgc aaaccatccg gcccggtaca aatcggcgcg gcgctgggtg atgacctggt 10200

ggagaagttg aaggccgcgc aggccgccca gcggcaacgc atcgaggcag aagcacgccc 10260

cggtgaatcg tggcaagcgg ccgctgatcg aatccgcaaa gaatcccggc aaccgccggc 10320

agccggtgcg ccgtcgatta ggaagccgcc caagggcgac gagcaaccag attttttcgt 10380

tccgatgctc tatgacgtgg gcacccgcga tagtcgcagc atcatggacg tggccgtttt 10440

ccgtctgtcg aagcgtgacc gacgagctgg cgaggtgatc cgctacgagc ttccagacgg 10500

gcacgtagag gtttccgcag ggccggccgg catggccagt gtgtgggatt acgacctggt 10560

actgatggcg gtttcccatc taaccgaatc catgaaccga taccgggaag ggaagggaga 10620

caagcccggc cgcgtgttcc gtccacacgt tgcggacgta ctcaagttct gccggcgagc 10680

cgatggcgga aagcagaaag acgacctggt agaaacctgc attcggttaa acaccacgca 10740

cgttgccatg cagcgtacga agaaggccaa gaacggccgc ctggtgacgg tatccgaggg 10800

tgaagccttg attagccgct acaagatcgt aaagagcgaa accgggcggc cggagtacat 10860

cgagatcgag ctagctgatt ggatgtaccg cgagatcaca gaaggcaaga acccggacgt 10920

gctgacggtt caccccgatt actttttgat cgatcccggc atcggccgtt ttctctaccg 10980

cctggcacgc cgcgccgcag gcaaggcaga agccagatgg ttgttcaaga cgatctacga 11040

acgcagtggc agcgccggag agttcaagaa gttctgtttc accgtgcgca agctgatcgg 11100

gtcaaatgac ctgccggagt acgatttgaa ggaggaggcg gggcaggctg gcccgatcct 11160

agtcatgcgc taccgcaacc tgatcgaggg cgaagcatcc gccggttcct aatgtacgga 11220

gcagatgcta gggcaaattg ccctagcagg ggaaaaaggt cgaaaaggtc tctttcctgt 11280

ggatagcacg tacattggga acccaaagcc gtacattggg aaccggaacc cgtacattgg 11340

gaacccaaag ccgtacattg ggaaccggtc acacatgtaa gtgactgata taaaagagaa 11400

aaaaggcgat ttttccgcct aaaactcttt aaaacttatt aaaactctta aaacccgcct 11460

ggcctgtgca taactgtctg gccagcgcac agccgaagag ctgcaaaaag cgcctaccct 11520

tcggtcgctg cgctccctac gccccgccgc ttcgcgtcgg cctatcgcgg ccgctggccg 11580

ctcaaaaatg gctggcctac ggccaggcaa tctaccaggg cgcggacaag ccgcgccgtc 11640

gccactcgac cgccggcgcc cacatcaagg caccctgcct cgcgcgtttc ggtgatgacg 11700

gtgaaaacct ctgacacatg cagctcccgg agacggtcac agcttgtctg taagcggatg 11760

ccgggagcag acaagcccgt cagggcgcgt cagcgggtgt tggcgggtgt cggggcgcag 11820

ccatgaccca gtcacgtagc gatagcggag tgtatactgg cttaactatg cggcatcaga 11880

gcagattgta ctgagagtgc accatatgcg gtgtgaaata ccgcacagat gcgtaaggag 11940

aaaataccgc atcaggcgct cttccgcttc ctcgctcact gactcgctgc gctcggtcgt 12000

tcggctgcgg cgagcggtat cagctcactc aaaggcggta atacggttat ccacagaatc 12060

aggggataac gcaggaaaga acatgtgagc aaaaggccag caaaaggcca ggaaccgtaa 12120

aaaggccgcg ttgctggcgt ttttccatag gctccgcccc cctgacgagc atcacaaaaa 12180

tcgacgctca agtcagaggt ggcgaaaccc gacaggacta taaagatacc aggcgtttcc 12240

ccctggaagc tccctcgtgc gctctcctgt tccgaccctg ccgcttaccg gatacctgtc 12300

cgcctttctc ccttcgggaa gcgtggcgct ttctcatagc tcacgctgta ggtatctcag 12360

ttcggtgtag gtcgttcgct ccaagctggg ctgtgtgcac gaaccccccg ttcagcccga 12420

ccgctgcgcc ttatccggta actatcgtct tgagtccaac ccggtaagac acgacttatc 12480

gccactggca gcagccactg gtaacaggat tagcagagcg aggtatgtag gcggtgctac 12540

agagttcttg aagtggtggc ctaactacgg ctacactaga aggacagtat ttggtatctg 12600

cgctctgctg aagccagtta ccttcggaaa aagagttggt agctcttgat ccggcaaaca 12660

aaccaccgct ggtagcggtg gtttttttgt ttgcaagcag cagattacgc gcagaaaaaa 12720

aggatctcaa gaagatcctt tgatcttttc tacggggtct gacgcttagt ggaacgaaaa 12780

ctcacgttaa gggattttgg tcatgcatga tatatctccc aatttgtgta gggcttatta 12840

tgcacgctta aaaataataa aagcagactt gacctgatag tttggctgtg agcaattatg 12900

tgcttagtgc atctaacgct tgagttaagc cgcgccgcga agcggcgtcg gcttgaacga 12960

atttctagct agacattatt tgccgactac cttggtgatc tcgcctttca cgtagtggac 13020

aaattcttcc aactgatctg cgcgcgaggc caagcgatct tcttcttgtc caagataagc 13080

ctgtctagct tcaagtatga cgggctgata ctgggccggc aggcgctcca ttgcccagtc 13140

ggcagcgaca tccttcggcg cgattttgcc ggttactgcg ctgtaccaaa tgcgggacaa 13200

cgtaagcact acatttcgct catcgccagc ccagtcgggc ggcgagttcc atagcgttaa 13260

ggtttcattt agcgcctcaa atagatcctg ttcaggaacc ggatcaaaga gttcctccgc 13320

cgctggacct accaaggcaa cgctatgttc tcttgctttt gtcagcaaga tagccagatc 13380

aatgtcgatc gtggctggct cgaagatacc tgcaagaatg tcattgcgct gccattctcc 13440

aaattgcagt tcgcgcttag ctggataacg ccacggaatg atgtcgtcgt gcacaacaat 13500

ggtgacttct acagcgcgga gaatctcgct ctctccaggg gaagccgaag tttccaaaag 13560

gtcgttgatc aaagctcgcc gcgttgtttc atcaagcctt acggtcaccg taaccagcaa 13620

atcaatatca ctgtgtggct tcaggccgcc atccactgcg gagccgtaca aatgtacggc 13680

cagcaacgtc ggttcgagat ggcgctcgat gacgccaact acctctgata gttgagtcga 13740

tacttcggcg atcaccgctt cccccatgat gtttaacttt gttttagggc gactgccctg 13800

ctgcgtaaca tcgttgctgc tccataacat caaacatcga cccacggcgt aacgcgcttg 13860

ctgcttggat gcccgaggca tagactgtac cccaaaaaaa cagtcataac aagccatgaa 13920

aaccgccact gcgccgttac caccgctgcg ttcggtcaag gttctggacc agttgcgtga 13980

gcgcatacgc tacttgcatt acagcttacg aaccgaacag gcttatgtcc actgggttcg 14040

tgcccgaatt gatcacaggc agcaacgctc tgtcatcgtt acaatcaaca tgctaccctc 14100

cgcgagatca tccgtgtttc aaacccggca gcttagttgc cgttcttccg aatagcatcg 14160

gtaacatgag caaagtctgc cgccttacaa cggctctccc gctgacgccg tcccggactg 14220

atgggctgcc tgtatcgagt ggtgattttg tgccgagctg ccggtcgggg agctgttggc 14280

tggctggtgg caggatatat tgtggtgtaa acaaattgac gcttagacaa cttaataaca 14340

cattgcggac gtttttaatg tactgaatta acgccgaatt gaattcaaga gctcaaggat 14400

cctaactata acggtcctaa ggtagcgaag gcgcgccgaa ttcgagggga tcgagcccct 14460

gctgagcctc gacatgttgt cgcaaaattc gccctggacc cgcccaacga tttgtcgtca 14520

ctgtcaaggt ttgacctgca cttcatttgg ggcccacata caccaaaaaa atgctgcata 14580

attctcgggg cagcaagtcg gttacccggc cgccgtgctg gaccgggttg aatggtgccc 14640

gtaactttcg gtagagcgga cggccaatac tcaacttcaa ggaatctcac ccatgcgcgc 14700

cggcggggaa ccggagttcc cttcagtgag cgttattagt tcgccgctcg gtgtgtcgta 14760

gatactagcc cctggggcac ttttgaaatt tgaataagat ttatgtaatc agtcttttag 14820

gtttgaccgg ttctgccgct ttttttaaaa ttggatttgt aataataaaa cgcaattgtt 14880

tgttattgtg gcgctctatc atagatgtcg ctataaacct attcagcaca atatattgtt 14940

ttcattttaa tattgtacat ataagtagta gggtacaatc agtaaattga acggagaata 15000

ttattcataa aaatacgata gtaacgggtg atatattcat tagaatgaac cgaaaccggc 15060

ggtaaggatc tgagctacac atgctcaggt tttttacaac gtgcacaaca gaattgaaag 15120

caaatatcat gcgatcatag gcgtctcgca tatctcatta aagcaggggg tgggcgaaga 15180

actccagcat gagatccccg cgctggagga tcatccagcc ggcgtcccgg aaaacgattc 15240

cgaagcccaa cctttcatag aaggcggcgg tggaatcgaa atctcgtgat ggcaggttgg 15300

gcgtcgcttg gtcggtcatt tcgaacccca gagtcccgc 15339